(19)

Europäisches
Patentamt
European
Patent Office
Office européen
des brevets

(11) **EP 2 299 267 A2**

(12) **EUROPEAN PATENT APPLICATION**

(43) Date of publication:
**23.03.2011 Bulletin 2011/12**

(51) Int Cl.:
***G01N 33/50*** *(2006.01)*

(21) Application number: **10176130.2**

(22) Date of filing: **14.11.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK RS**

(30) Priority: **16.11.2005 US 737410 P**

(62) Document number(s) of the earlier application(s) in
accordance with Art. 76 EPC:
**06829992.4 / 1 952 147**

(27) Previously filed application:
**14.11.2006 PCT/EP2006/068457**

(71) Applicants:
• **Novartis AG**
**4056 Basel (CH)**
• **The University of Zurich**
**8006 Zürich (CH)**

(72) Inventors:
• **Kinnunen, Anu**
**79117 Freiburg (DE)**
• **Schwab, Martin E.**
**8057 Zürich (CH)**
• **Montani, M. Laura**
**8046 Zürich (CH)**
• **Dimou, Leda**
**80336 München (DE)**
• **Mir, Anis Khusro**
**68870 Bartenheim (FR)**
• **Schnell, Lisa**
**8001 Zürich (CH)**

(74) Representative: **Rouquayrol, Céline Hélène**
**Novartis Pharma AG**
**Patent Department**
**4002 Basel (CH)**

Remarks:
This application was filed on 10-09-2010 as a
divisional application to the application mentioned
under INID code 62.

(54) **Biomarkers for anti-NogoA antibody treatment in spinal cord injury**

(57)     This disclosure of this invention confirms, at the level of gene expression, the injured spinal cord and motor cortex as the primary sites of action of the anti-Nogo-A antibody treatment applied intrathecally. The disclosure further provides methods for predicting the response of a subject to a medicament comprising an anti-Nogo-A antibody.

EP 2 299 267 A2

**Description**

FIELD OF THE INVENTION

This invention relates generally to the analytical testing of tissue samples *in vitro,* and more particularly to aspects of gene expression induced by administration of anti-Nogo-A antibody.

BACKGROUND OF THE INVENTION

Nogo-A plays an important role in inhibition of neurite outgrowth. Antibodies against Nogo-A have been shown to result in axonal regeneration and functional recovery after spinal cord injury.
A number of microarray gene expression profiling studies have addressed molecular changes after spinal cord injury. For a review, see Bareyre FM & Schwab ME, Trends Neurosci. 26:555-563 (2003). However, there continues to be a need in the art for early peripheral biomarkers for efficacy of the anti-Nogo-A antibody treatment. Such biomarkers would be useful in differentiating the responders from non-responders as well as guiding the dosing in a clinical setting.

SUMMARY OF THE INVENTION

The invention provides a description of the molecular changes resulting from inhibition of Nogo-A function using anti-Nogo-A antibodies. Genes and functional pathways affected by inhibition or reduction of Nogo-A have been identified in an *in vivo* system using a genomics approach.
The invention also relates to novel molecular targets to enhance central nervous system recovery, to enhance regeneration of neuronal connections and to enhance neuronal and synaptic plasticity in clinical conditions such as but not exclusively injury such as trauma or stroke, neurodegenerative disorders such as but not exclusively Alzheimer's disease, Parkinson's disease, Huntington's disease, amyotrophic lateral sclerosis, depression and any other disorder where axonal or dendritic pathology is part of the disease process or result of the disease, such as but not exclusively any demyelinating disorders, such as multiple sclerosis. It also relates to novel indications for targeting Nogo-A and/or genes and pathways affected as a result of inhibition of Nogo-A such as but not exclusively neurodegenerative disorders (Alzheimer's disease, Parkinson's disease, Huntington's disease, ALS) depression and any other disorder where axonal or dendritic pathology is part of the disease process or result of the disease, such as but not exclusively any demyelinating disorders, such as multiple sclerosis.
In particular, the present invention relates to a method for predicting the response of a subject to a medicament comprising an anti-Nogo-A antibody, wherein the expression of at least one gene of Table 25 is assessed before and after administration of said medicament comprising an anti-Nogo-A antibody and wherein said expression of said at least one gene of Table 25 after administration of said medicament comprising an anti-Nogo-A antibody is compared to the expression of said gene prior to said administration of the medicament comprising an anti-Nogo-A antibody. In a particular embodiment, a dysregulation of said expression of at least one gene of Table 25 after administration of the medicament comprising an anti-Nogo-A antibody as compared to the expression of said gene prior said administration of the medicament comprising an anti-Nogo-A antibody is indicative of a positive response (responder) to said administration of the medicament comprising an anti-Nogo-A antibody. In another embodiment, the lack of a dysregulation of said expression of at least one gene of Table 25 after administration of the medicament comprising an anti-Nogo-A antibody as compared to the expression of said gene prior said administration of the medicament comprising an anti-Nogo-A antibody is indicative of a lack of response (non-responder) to said administration of the medicament comprising an anti-Nogo-A antibody. In a preferred embodiment, said dysregulation of said expression of at least one gene of Table 25 after administration of the medicament comprising an anti-Nogo-A antibody is a change in expression that is larger or equal to 1.2 fold and statistically significant ($p < 0.05$, Student's t-test) as compared to the expression of said gene prior said administration of the medicament comprising an anti-Nogo-A antibody. In a most preferred embodiment, the expression of at least one gene of each of the groups of adhesion genes, cytoskeleton genes and signalling genes is assessed, wherein said group of adhesion genes consists of cadherin 11, cadherin 2, cadherin 8, cadherin 22, Eph receptor A3, Eph receptor A4, Ephrin A3, Ephrin B2, Eph receptor B2, semaphorin 4A, semaphorin 4D, semaphorin 4F, semaphorin 6A, semaphorin 6B, semaF cytoplasmic domain associated protein 3 and Plexin B2, wherein said group of cytoskeleton genes consist of capping protein (actin filament) gelsolin-like, casein kinase 1 delta, centractin, gelsolin, microtubule-associated protein tau and neurofilament 68, and wherein said group of signalling genes consists of Rho-GDP-dissociation inhibitor 1, dihydropyrimidinase related protein 2, dihydropyrimidinase related protein 1, dihydropyrimidinase related protein 5. In another embodiment, the expression of all the genes of Table 25 is assessed.
In one embodiment of the present invention, a dysregulation of the expression of at least one gene of Table 25 after administration of the medicament comprising an anti-Nogo-A antibody as compared to the expression of said gene prior said administration of the medicament comprising an anti-Nogo-A antibody is indicative of indicates central nervous system regeneration.

The methods of the invention can be performed *in vitro*.

Also encompassed with the present invention is the use of an anti-Nogo-A antibody in the manufacture of a medicament for the treatment of central nervous system injury in a patient population, wherein the patient population is selected as described herein.

Preferably, the anti-Nogo-A antibody is a fully human monoclonal antibody (IgG4/□) that binds to the epitope of human Nogo-A fragment from amino acid 342-357.

The present invention also relates to methods for treating a central nervous system injury in a subject with an anti-Nogo-A antibody, as well as methods for diagnosing central nervous system regeneration in a subject after administering of an anti-Nogo-A.

Moreover, the present invention also encompasses a kit for performing the methods described herein, said kit comprising at least two probes, each probe being capable of specifically detecting the expression of one gene of Table 25, wherein said at least two probes do not detect the expression of the same gene.

Genes and molecular pathways affected by inhibition of Nogo-A can by themselves be therapeutically targeted for similar disorders as those treatable by Nogo-A antibody therapy. Alternatively, novel therapeutics designed for the genes and pathways affected by inhibition of Nogo-A can be used as add-on therapies to enhance the therapeutic effect of Nogo-A inhibition. In addition, the genes and pathways affected by inhibition of Nogo-A provide therapeutic indications for inhibition of Nogo-A such as but not exclusively conditions where neuronal or synaptic plasticity has been challenged such as cognitive impairments related neurodegenerative disorders (Alzheimer's disease, Parkinson's disease, Huntington's disease) and psychiatric disorders.

BRIEF DESCRIPTION OF THE DRAWINGS

The drawing figures depict preferred embodiments by way of example, not by way of limitations. In the figures, like reference numerals refer to the same or similar elements.

FIG. 1. Enrichment of immunity and defence-related transcripts in the direction of 11C7 after one week of treatment identified by GSEA in T8

FIG. 2. Enrichment of cytokine and chemokine mediated signalling pathway in the direction of 11 C7 after one week of treatment identified by GSEA in T8

FIG. 3. Enrichment of Jak-stat cascade-related transcripts in the direction of 11C7 after one week of treatment identified by GSEA in T8

FIG. 4. Enrichment of oxidative phosphorylation-related transcripts in the direction of 11C7 after two weeks of treatment identified by GSEA in T8

FIG. 5. Enrichment of synaptic transmission-related transcripts in the direction of 11C7 after two weeks of treatment identified by GSEA in T8

FIG. 6. Enrichment of ECM-mediated signalling—related transcripts in the direction of IgG after one week of treatment identified by GSEA in T1-7

FIG. 7. Enrichment of lipid metabolism-related transcripts in the direction of 11C7 after one week of treatment identified by GSEA in T1-7

FIG. 8. Enrichment of growth factor homeostasis-related transcripts in the direction of IgG after one week of treatment identified by GSEA in T 1-7

FIG. 9. Enrichment of immunity and defence-related transcripts in the direction of 11C7 after one week of treatment identified by GSEA in L1-5

FIG. 10. Enrichment of signal transduction-related transcripts in the direction of 11C7 after one week of treatment identified by GSEA in L1-5

FIG. 11. Enrichment of cell communication-related transcripts in the direction of 11C7 after one week of treatment identified by GSEA in L1-5

FIG. 12. Enrichment of immunity and defence-related transcripts in the direction of IgG after two weeks of treatment identified by GSEA in L1-5

FIG. 13. Enrichment of cell communication-related transcripts in the direction of IgG after two weeks of treatment identified by GSEA in L1-5

FIG. 14. Enrichment of synaptic transmission-related transcripts in the direction of 11C7 after two weeks of treatment identified by GSEA in L1-5

FIG. 15. Enrichment of Huntington's disease—related transcripts in the direction of IgG after two weeks of treatment identified by GSEA in motor-somatosensory cortex

FIG. 16. Enrichment of EGF receptor mediated signalling—related transcripts in the direction of IgG after two weeks of treatment identified by GSEA in motor-somatosensory cortex

FIG. 17. Enrichment of FGF receptor mediated signalling-related transcripts in the direction of IgG after two weeks of treatment identified by GSEA in motor-somatosensory cortex

FIG. 18. Enrichment of NGF receptor mediated signalling—related transcripts in the direction of IgG after two weeks of treatment identified by GSEA in motor-somatosensory cortex

FIG. 19. Enrichment of receptor mediated endocytosis—related transcripts in the direction of 11C7 after one week of treatment identified by GSEA in blood

FIG. 20. Enrichment of interferon mediated immunity—related transcripts in the direction of 11C7 after one week of treatment identified by GSEA in blood

FIG. 21. Enrichment of neuroactive ligand-receptor interaction—related transcripts in the direction of IgG after one week of treatment identified by GSEA in blood

FIG. 22. Enrichment of macrophage mediated immunity—related transcripts in the direction of 11C7 after one week of treatment identified by GSEA in blood

FIG. 23. Enrichment of I11b signalling—related transcripts in the direction of IgG after one week of treatment identified by GSEA in blood

FIG. 24. Enrichment of B cell activation—related transcripts in the direction of 11C7 after one week of treatment identified by GSEA in blood

FIG. 25. Enrichment of immunity and defence—related transcripts in the direction of IgG after two weeks of treatment identified by GSEA in blood

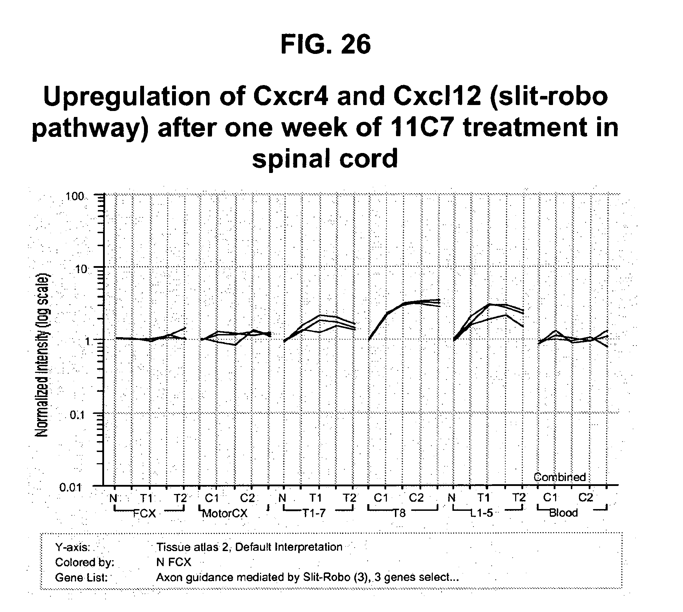

FIG. 26. Upregulation of Cxcr4 and Cxc112 (slit-robo pathway) after one week of 11C7 treatment in spinal cord

## DETAILED DESCRIPTION OF THE INVENTION

It is to be appreciated that certain aspects, modes, embodiments, variation and features of the invention are described below in various levels of detail in order to provide a substantial understanding of the present invention. In general, such disclosure provides useful biomarkers for the diagnosis and treatment of subjects in need thereof. Accordingly, the various aspects of the present invention relate to diagnostic/theranostic methods and kits to identify individuals predisposed to disease or to classify individuals with regard to drug responsiveness, side effects, or optimal drug dose. The methods and kits are useful for studying the aetiology of diseases, studying the efficacy of drug targeting, predicting individual susceptibility to diseases, and predicting individual responsiveness to drugs targeting the gene product. Accordingly, various particular embodiments that illustrate these aspects follow.

*Polynucleotides and Polypeptides of the Invention.* Gene expression profiling in a rat spinal cord injury model was undertaken after mouse monoclonal anti-Nogo-A antibody 11C7—treatment and compared to control mouse anti-plant lectin IgG after seven and 14 days of treatment in different tissues, resulting in 12 different comparisons. The datasets were subjected to the following analyses: (1) statistical restriction (Welch t-test p<0.05) and ranking by fold change; and (2) gene set enrichment analysis (GSEA), which is a pathway centric view of the data first introduced by Mootha VK et al., Nat. Genet. 34:267-273 (2003) and recently by Subramanian et al. Proc. Natl. Acad. Sci. U.SA 102 (43): 15545-50 (2005). The analysis resulted in identification of 24 pathways significantly affected by the treatment in three or more of the tissues at either timepoint.

Ranked by the treatment effect size based on the number of significantly differentially expressed genes and the fold change of the top 100 significantly changed transcripts in each treatment group, spinal cord distal to the site of lesion (L1-5), the site of the lesion (T8) and blood were the most affected tissues after one week of treatment. L1-5, motor-somatosensory cortex and spinal cord proximal to the site of lesion (T1-7) were the most affected regions after two weeks of treatment. At either timepoint, only minimal effect in the frontal cortex was observed.

GSEA identified immunity and defence, protein metabolism and phosphorylation, nucleoside, nucleotide and nucleic acid metabolism, neuronal activities and Jak-stat cascade as the most widely affected pathways overall. All of these pathways were affected in three to four tissues concomitantly.

Anti-Nogo-A treatment applied intrathecally after spinal cord injury in rat has the largest effect in spinal cord. Genes promoting axon guidance and neurite outgrowth were upregulated, inhibitory cues downregulated in spinal cord after anti-Nogo-A treatment. Of the neurite outgrowth/axon guidance related pathways, GSEA pointed the slit-robo mediated axon guidance pathway as most frequently affected by 11C7 treatment. Cxc112 and Cxc4r, two members of this pathway were upregulated by 11 C7 in a concerted fashion after one week of treatment in all segments of the spinal cord studied. Cxc112 and Cxc4r were recently identified as key players in defining the initial trajectory of mammalian motor axons during development by Lieberam I et al., Neuron 47:667-679 (2005). This finding suggests that this pathway is affected by 11C7 treatment and may thus contribute to the mechanism of action of anti-Nogo A during regeneration.

At the site of the lesion, the EGF-receptor mediated signalling pathway was upregulated by 11C7 after one week of treatment but downregulated after two weeks of treatment. In the motor cortex, the EGF-receptor mediated signalling pathway was downregulated by 11C7 after one week and after two weeks of treatment. Altogether 24 pathways with significant enrichment (q<0.001) were identified to be affected by anti-Nogo-A treatment in three or more tissues at either timepoint. The most widely affected pathways overall were related to immunity and defence, protein metabolism and phosphorylation and neuronal activities. Upregulation of synaptic transmission related probesets in lumbal spinal cord after two weeks of anti-Nogo-A treatment.

The results confirm at the level of gene expression the injured spinal cord and motor cortex as the primary sites of action of the anti-Nogo-A antibody treatment applied intrathecally. The analysis identified novel molecular and pathways candidates as possible targets of anti-Nogo-A treatment, such as myocilin and the split-robo pathway. The results also pointed to strong involvement of immune defence related pathways in the treatment effect.

TAQMAN analysis confirmed selected findings concerning the secreted proteins Sfrp4, Mmp9 and myocilin.

*Anti-Nogo antibodies.* Published PCT patent application WO 00/31235 discloses several antibodies raised against Nogo proteins and derivatives thereof. For examples of anti-Nogo antibodies, including monoclonal antibodies and fragments thereof, and of methods of their use, see Bregman BS et al., Nature 378:498-501 (1995); Brosamle C et al., J Neurosci. 20:8061-8068 (2000); Bareyre FM et al., J. Neurosci. 22:7097-7110 (2002); Chen et al., Nature 403: 434-439 (2000); Fiedler M et al., Protein Eng. 15:931-941 (2002); Merkler D et al., J. Neurosci. 21:3665-3673 (2001); Oertle T et al., J Neurosci. 23:5393-5406 (2003); Papadopoulos CM et al., .Ann. Neurol. (2002); and Von Meyenburg J et al., Exp.Neurol. 154:583-594 (1998). See also, Wiessner C et al., In Pharmacology of Cerebral Ischemia, Krieglstein J & Klumpp S, eds. (2003) pp. 343-353; and Wiessner C et al., J. Cereb. Blood Flow & Metab. 23: 154-165 (2003) for the use of anti-Nogo antibodies in a stroke model. Doses of anti-Nogo A antibody used in the EXAMPLES have been shown to result in functional recovery in the same model. Liebscher et al., Ann. Neurol. 58:706-719 (2005). Published PCT patent application WO 00/31235 also discloses two antisera raised against Nogo A sequences, AS Bruna and AS 472. See also published PCT patent application WO 2000/05364A1, which discloses antibodies to Nogo protein fragments. In the EXAMPLES, anti Nogo-A antibody 11C7: Mouse monoclonal antibody (mAb) 11 C7, raised against a 18aa peptide Nogo-A corresponding to rat sequence amino acids 623—640; used at a concentration of 3mg/ml in PBS. The control antibody was a mouse monoclonal IgG directed against plant lectin used at a concentration of 3mg/ml in PBS. The biochemical and neutralizing properties of both antibodies are described in Oertle T et al., J Neurosci. 23:5393-5406 (2003). In one embodiment of the invention, the anti-Nogo antibody is a fully human monoclonal antibody (IgG4/κ) generated from mice which are genetically reconstituted with human immunoglobulin genes and which binds to the epitope of human Nogo-A fragment from aa342-357. See Published PCT patent applications WO 90/05191 and WO 00/31235.

Accordingly, the invention is relevant to ischemic brain injury (stroke), traumatic brain injury (head injury), multiple sclerosis (MS), amyotrophic lateral sclerosis (ALS), Alzheimer's disease. The invention is also relevant to axonal regeneration and improved sprouting after nerve fibre damage; various diseases of the peripheral and central nervous system, neurodegenerative diseases such as Alzheimer disease, Parkinson disease, ALS, Lewy like pathologies or other dementia in general, diseases following cranial, cerebral or spinal trauma, stroke or a demyeliating disease including multiple sclerosis, monophasic demyelination, encephalomyelitis, multifocal leukoencephalopathy, panencephalitis, Marchiafava-Bignami disease, pontine myelmolysis, adrenoleukodystrophy, Pelizaeus-Merzbacher disease, Spongy degeneration, Alexander's disease, Canavan's disease, metachromatic leukodystrophy and Krabbe's disease; degenerative ocular disorders involving the degeneration of retinal or corneal cells including ischemic retinopathies, anterior ischemic optic neuropathy, optic neuritis, age-related macular degeneration, diabetic retinopathy, cystoid macular oedema, retinitis pigmentosa, Stargardt's disease, Best's vitelliform retinal degeneration, Leber's congenital amaurosis and other hereditary retinal degenerations, pathologic myopia, retinopathy of prematurity, Leber's hereditary optic neuropathy, the after effects of corneal transplantation or of refractive corneal surgery, herpes keratitis.

*Definitions.* The definitions of certain terms as used in this specification are provided below. Definitions of other terms may be found in the glossary provided by the U.S. Department of Energy, Office of Science, Human Genome Project (http://www.ornl.gov/sci/techresources/Human Genome/glossary/). In practicing the present invention, many conventional techniques in molecular biology, microbiology and recombinant DNA are used. These techniques are well-known and are explained in, e.g., Current Protocols in Molecular Biology, Vols. I-III, Ausubel, ed. (1997); Sambrook et al., Molecular Cloning: A Laboratory Manual, Second Edition (Cold Spring Harbor Laboratory Press, Cold Spring Harbor, New York, 1989); DNA Cloning: A Practical Approach, Vols. I and II, Glover D, ed. (1985); Oligonucleotide Synthesis, Gait, ed. (1984); Nucleic Acid Hybridization, Hames & Higgins, eds. (1985); Transcription and Translation, Hames & Higgins, eds. (1984); Animal Cell Culture, Freshney, ed. (1986); Immobilized Cells and Enzymes (IRL Press, 1986); Perbal, A Practical Guide to Molecular Cloning; the series, Methods in Enzymol. (Academic Press, Inc., 1984); Gene Transfer Vectors for Mammalian Cells, Miller & Calos, eds. (Cold Spring Harbor Laboratory, New York, 1987); and Methods in Enzymology, Vols. 154 and 155, Wu & Grossman, and Wu, Eds., respectively.

As used herein, the term "antibody" includes, but is not limited to, e.g., polyclonal antibodies, monoclonal antibodies, humanized or chimaeric antibodies and biologically functional antibody fragments sufficient for binding of the antibody fragment to the protein. In an embodiment of the invention, the antibody is an anti-Nogo antibody.

The term "biological sample" is intended to include, but is not limited to, e.g., tissues, cells and biological fluids isolated from a subject, as well as tissues, cells and fluids present within a subject. In the EXAMPLES, the biological samples are central nervous system samples. However, the use of other biological samples is envisioned. Suitable "biological samples" are for instance blood, serum, lymph, endothelial cells, sputum, urine, faeces or semen. Particularly suited for the methods of the invention are central nervous system (CNS) interstitial fluid and/or cerebrospinal fluid (CSF).

As used herein, the term "clinical response" means any or all of the following: a quantitative measure of the response, no response, and adverse response (i.e., side effects).

As used herein, the term "clinical trial" means any research study designed to collect clinical data on responses to a particular treatment, and includes, but is not limited to phase I, phase II and phase III clinical trials. Standard methods are used to define the patient population and to enrol subjects.

As used herein, the term "effective amount" of a compound is a quantity sufficient to achieve a desired therapeutic and/or prophylactic effect, for example, an amount which results in the prevention of, or a decrease in the symptoms associated with, a disease that is being treated. The amount of compound administered to the subject will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. Typically, an effective amount of the compounds of the present invention, sufficient for achieving a therapeutic or prophylactic effect range from about 0.000001 mg per kilogram body weight per day to about 10,000 mg per kilogram body weight per day. A preferred dosage ranges from about 0.0001 mg per kilogram body weight per day to about 1,000 mg per kilogram body weight per day. Another preferred dosage ranges from about 0.01 mg per kilogram body weight per day to about 100 mg per kilogram body weight per day. The compounds of the present invention can also be administered in combination with each other, or with one or more additional therapeutic compounds. In the EXAMPLES, doses of anti-Nogo A antibody used in the EXAMPLES have been shown to result in functional recovery in the same model. Liebscher et al., Ann. Neurol. 58: 706-719 (2005). See also published PCT patent application WO 2000/05364A1, which discloses antibodies to Nogo protein fragments.

As used herein, "expression" includes but is not limited to one or more of the following: transcription of the gene into precursor mRNA; splicing and other processing of the precursor mRNA to produce mature mRNA; mRNA stability; translation of the mature mRNA into protein (including codon usage and mRNA availability); and glycosylation and/or other modifications of the translation product, if required for proper expression and function.

As used herein, the term "gene" means a segment of DNA that contains all the information for the regulated biosynthesis of an RNA product, including promoters, exons, introns, and other untranslated regions that control expression.

As used herein, the term "genotype" means an unphased 5' to 3' sequence of nucleotide pair(s) found at one or more polymorphic sites in a locus on a pair of homologous chromosomes in an individual. As used herein, genotype includes a full-genotype and/or a sub-genotype.

As used herein, the term "locus" means a location on a chromosome or DNA molecule corresponding to a gene or a physical or phenotypic feature.

As used herein, the term "isogene" means the different forms of a given gene that exist in the population.

As used herein, the term "mutant" means any heritable variation from the wild-type that is the result of a mutation, e.g., single nucleotide polymorphism. The term "mutant" is used interchangeably with the terms "marker", "biomarker", and "target" throughout the specification.

As used herein, the term "medical condition" includes, but is not limited to, any condition or disease manifested as one or more physical and/or psychological symptoms for which treatment is desirable, and includes previously and newly identified diseases and other disorders.

As used herein, the term "nucleotide pair" means the nucleotides found at a polymorphic site on the two copies of a chromosome from an individual.

As used herein, the term "polymorphic site" means a position within a locus at which at least two alternative sequences are found in a population, the most frequent of which has a frequency of no more than 99%.

As used herein, the term "population" may be any group of at least two individuals. A population may include, e.g., but is not limited to, a reference population, a population group, a family population, a clinical population, and a same sex population.

As used herein, the term "phased" means, when applied to a sequence of nucleotide pairs for two or more polymorphic sites in a locus, the combination of nucleotides present at those polymorphic sites on a single copy of the locus is known.

As used herein, the term "polymorphism" means any sequence variant present at a frequency of>1% in a population. The sequence variant may be present at a frequency significantly greater than 1% such as 5% or 10% or more. Also, the term may be used to refer to the sequence variation observed in an individual at a polymorphic site. Polymorphisms include nucleotide substitutions, insertions, deletions and microsatellites and may, but need not, result in detectable differences in gene expression or protein function.

As used herein, the term "polynucleotide" means any RNA or DNA, which may be unmodified or modified RNA or DNA. Polynucleotides include, without limitation, single- and double-stranded DNA, DNA that is a mixture of single- and double-stranded regions, single- and double-stranded RNA, RNA that is mixture of single- and double-stranded regions, and hybrid molecules comprising DNA and RNA that may be single-stranded or, more typically, double-stranded or a mixture of single- and double-stranded regions. In addition, polynucleotide refers to triple-stranded regions comprising RNA or DNA or both RNA and DNA. The term polynucleotide also includes DNAs or RNAs containing one or more modified bases and DNAs or RNAs with backbones modified for stability or for other reasons.

As used herein, the term "polypeptide" means any polypeptide comprising two or more amino acids joined to each other by peptide bonds or modified peptide bonds, *i.e.,* peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well-known in the art. Such modifications are well described in basic texts and in more detailed monographs, as well as in a voluminous research literature.

As used herein, the term "reference standard population" means a population characterized by one or more biological characteristics, e.g., drug responsiveness, genotype, haplotype, phenotype, *etc.*

As used herein, the term "reference standard gene expression profile" is the pattern of expression of one or more gene observed in either a reference standard population or a single subject prior to administration of a compound.

As used herein, the term "subject" means that preferably the subject is a mammal, such as a human, but can also be an animal, including but not limited to, domestic animals *(e.g.,* dogs, cats and the like), farm animals (e.g., cows, sheep, pigs, horses and the like) and laboratory animals (e.g., monkeys such as cynmologous monkeys, rats, mice, guinea pigs and the like).

As used herein, a "test sample" means a biological sample obtained from a subject of interest. For example, a test sample can be a biological fluid (e.g., serum), cell sample, or tissue, or isolated nucleic acid or polypeptide derived therefrom.

As used herein, the term "dysregulation" means a change that is larger or equal to 1.2 fold and statistically significant ($p < 0.05$, Student's t-test) from the control.. For example, a 1.5, 2, 2.5, 3, 3.5, 4, 4.5, or 5 fold change.

As used herein, the administration of an agent or drug to a subject or patient includes self-administration and the administration by another. It is also to be appreciated that the various modes of treatment or prevention of medical conditions as described are intended to mean "substantial", which includes total but also less than total treatment or prevention, and wherein some biologically or medically relevant result is achieved.

The details of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description and the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs. All references cited herein are incorporated herein by reference in their entirety and for all purposes to the same extent as if each individual publication, patent, or patent application was specifically and individually indicated to be incorporated by reference in its entirety for all purposes.

*Amplifying a Target Gene Region.* The target region(s) may be amplified using any oligonucleotide-directed amplification method, including but not limited to polymerase chain reaction (PCR). (U.S. Pat. No. 4,965,188), ligase chain reaction (LCR) (Barany et al., Proc. Natl. Acad. Sci. USA, 88:189-193 (1991); published PCT patent application WO 90/01069), and oligonucleotide ligation assay (OLA) (Landegren et al., Science, 241:1077-1080 (1988)). Qligonucleotides useful as primers or probes in such methods should specifically hybridize to a region of the nucleic acid that contains or is adjacent to the polymorphic site. Other known nucleic acid amplification procedures may be used to amplify the target region including transcription-based amplification systems. (U,S, Pat. No. 5,130,238; EP 0 329 822; U.S. Pat. No. 5,169,766, published PCT patent application WO 89/06700) and isothermal methods *(*Walker et al., Proc. Natl. Acad. Sci., USA, 89:392-396 (1992).

*Hybridizing Allele-Specific Oligonucleotide to a Target Gene.* Hybridization of an allele-specific oligonucleotide to a target polynucleotide may be performed with both entities in solution, or such hybridization may be performed when either the oligonucleotide or the target polynucleotide is covalently or noncovalently affixed to a solid support. Attachment may be mediated, for example, by antibody-antigen interactions, poly-L-Lysine, streptavidin or avidin-biotin, salt bridges, hydrophobic interactions, chemical linkages, UV cross-linking, baking, *etc.* Allele-specific oligonucleotide may be synthesized directly on the solid support or attached to the solid support subsequent to synthesis. Solid-supports suitable for use in detection methods of the invention include substrates made of silicon, glass, plastic, paper and the like, which may be formed, for example, into wells (as in 96-well plates), slides, sheets, membranes, fibres, chips, dishes, and beads. The solid support may be treated, coated or derivatised to facilitate the immobilization of the allele-specific oligonucleotide or target nucleic acid.

The genotype or haplotype for the gene of an individual may also be determined by hybridization of a nucleic sample containing one or both copies of the gene to nucleic acid arrays and subarrays such as described in WO 95/11995. The arrays would contain a battery of allele-specific oligonucleotides representing each of the polymorphic sites to be included in the genotype or haplotype.

*See,* also, Molecular Cloning A Laboratory Manual, Second Ed., Sambrook, Fritsch & Maniatis, ed. (Cold Spring Harbor Laboratory Press, 1989); DNA Cloning, Volumes I and II, Glover DN ed. (1985); Oligonucleotide Synthesis, Gait MJ ed. (1984); Nucleic Acid Hybridization, Hames BD & Higgins SJ, eds., 1984).

*Computer System for Storing or Displaying Gene Expression or Polymorphism Data.* The invention also provides a computer system for storing and displaying data determined for the gene. Polymorphism data is information that includes, but is not limited to, e.g., the location of polymorphic sites; sequence variation at those sites; frequency of polymorphisms in one or more populations; the different genotypes and/or haplotypes determined for the gene; frequency of one or more of these genotypes and/or haplotypes in one or more populations; any known association(s) between a trait and a genotype or a haplotype for the gene. The computer system comprises a computer processing unit, a display, and a database containing the polymorphism data. The polymorphism data includes the polymorphisms, the genotypes and the haplotypes identified for a given gene in a reference population. In a preferred embodiment, the computer system is capable of producing a display showing gene expression pattern organized according to their evolutionary relationships.

In addition, the computer may execute a program that generates views (or screens) displayed on a display device and with which the user can interact to view and analyze large amounts of information, relating to the gene and its genomic variation, including chromosome location, gene structure, and gene family, gene expression data, polymorphism data, genetic sequence data, and clinical data population data (e.g., data on ethnogeographic origin, clinical responses, and gene expression pattern for one or more populations). The polymorphism data described herein maybe stored as part of a relational database (e.g., an instance of an Oracle database or a set of ASCII flat files). These polymorphism data may be stored on the computer's hard drive or may, for example, be stored on a CD-ROM or on one or more other storage devices accessible by the computer. For example, the data may be stored on one or more databases in communication with the computer *via* a network.

In the EXAMPLE below, the data normalization was performed as follows: Values below 0 were set to 0.1. Each measurement was divided by the 50.0th percentile of all measurements in that sample. Finally, per gene normalization was performed by normalizing to the expression value of the median of naïve samples.

In EXAMPLE 1, differentially expressed genes between the vehicle and the treatments were identified within each experiment based on the following restrictions: (1) Prefiltering restrictions: Probe sets included in further analysis had to flagged present in 4/6 of replicates in any condition. Raw data signal intensity had to be minimum 50 in at least one of the treatment groups. (2) Statistical restriction: $p<0.05$ (Welch t-test (parametric)). Similar statistical restriction was always applied to different groups to be compared and is mentioned in each comparison.

In EXAMPLE 1, the Gene Set Enrichment Analysis (GSEA) method was used to analyze microarray data. Genes with expression levels below 100 on more than 75% of the chips are discarded as low- or non-expressed. Microarray results are then analyzed in a series of pairwise comparisons between sets of condition (e.g. treated vs. control). Each gene's relative expression level under *condition* and *condition$_2$* is computed as an expression ratio $r_i$

$$ r_i = \frac{\mu_{i,1}}{\mu_{i,2}} $$

where $\mu_{ij}$ is the average expression value for gene *i* under *condition$_j$*. The genes are then sorted according to their expression ratios such that those genes with higher expression under *condition$_1$* than *condition$_2$* are at the top of the list. Next, the collection of available gene sets is projected onto the sorted list. This step in essence applies *a priori* biological knowledge to the experimental data to identify functionally related genes that are expressed in a coordinated fashion. Gene sets are processed one at a time. For gene set G each expression ratio $r_i$ is labelled 'in' the gene set if $gene_i \in$ G and 'out' of the gene set *if gene$_j \notin$* G. A two-tailed Wilcoxon rank-sum test is calculated to determine if the genes labelled 'in' gene set G are enriched at either the top or bottom of the sorted list. The false discovery rate method of Storey JD & Tibshirani R, Proc Natl Acad Sci USA 100:9440-9445 (2003) is applied to transform p-values to multiple testing corrected q-values. The output from GSEA is a list of q-values *($q_1$, $q_2$, ..., $q_N$)* and labels *($l_1$, $l_2$, ..., $l_N$), $l_i \in$ (top, bottom)* that correspond to the *N* available gene sets. A small q-value $q_i$ indicates that the genes in gene set $G_i$ are significantly enriched at either the top or bottom of the list of expression ratios.

EXAMPLE 2 also provides a description of a GSEA analysis method.

*Kits of the Invention.* It is to be understood that the methods of the invention described herein generally may further comprise the use of a kit according to the invention. The invention provides nucleic acid and polypeptide detection kits useful for haplotyping and/or genotyping the gene in an individual. Such kits are useful to classify subjects. Generally, the methods of the invention may be performed *ex-vivo,* and such *ex-vivo* methods are specifically contemplated by the present invention. Also, where a method of the invention may include steps that may be practised on the human or animal body, methods that only comprise those steps which are not practised on the human or animal body are specifically contemplated by the present invention.

The kits of the invention are useful for detecting the presence of a polypeptide or nucleic acid corresponding to a marker of the invention in a biological sample, e.g., any body fluid including, but not limited to, e.g., serum, plasma, lymph, cystic fluid, urine, stool, cerebrospinal fluid, acitic fluid or blood and including biopsy samples of body tissue. For example, the kit can comprise a labelled compound or agent capable of detecting a polypeptide or an mRNA encoding a polypeptide corresponding to a marker of the invention in a biological sample and means for determining the amount of the polypeptide or mRNA in the sample e.g., an antibody which binds the polypeptide or an oligonucleotide probe which binds to DNA or mRNA encoding the polypeptide.

For antibody-based kits, the kit can comprise, e.g., (1) a first antibody, e.g., attached to a solid support, which binds to a polypeptide corresponding to a marker or the invention; and, optionally; (2) a second, different antibody which binds to either the polypeptide or the first antibody and is conjugated to a detectable label.

For oligonucleotide-based kits, the kit can comprise, e.g., (1) an oligonucleotide, e.g., a detectably-labelled oligonucleotide, which hybridizes to a nucleic acid sequence encoding a polypeptide corresponding to a marker of the invention; or (2) a pair of primers useful for amplifying a nucleic acid molecule corresponding to a marker of the invention.

The kit can also comprise, e.g., a buffering agent, a preservative or a protein-stabilizing agent. The kit can further comprise components necessary for detecting the detectable-label, e.g., an enzyme or a substrate. The kit can also contain a control sample or a series of control samples, which can be assayed and compared to the test sample. Each component of the kit can be enclosed within an individual container and all of the various containers can be within a single package, along with instructions for interpreting the results of the assays performed using the kit. In a preferred embodiment, such kit may further comprise a DNA sample collecting means. The kits of the invention may contain a written product on or in the kit container. The written product describes how to use the reagents contained in the kit, e.g., to use the biomarkers of the present invention in determining a strategy for preventing or treating a medical condition in a subject. In several embodiments, the use of the reagents can be according to the methods of the invention. In one embodiment, the reagent is a gene chip for determining the gene expression of relevant genes.

*Correlating a Subject to a Standard Reference Population.* To deduce a correlation between clinical response to a treatment and a gene expression pattern, it is necessary to obtain data on the clinical responses exhibited by a population of individuals who received the treatment, i.e., a clinical population. This clinical data maybe obtained by retrospective analysis of the results of a clinical trial(s). Alternatively, the clinical data may be obtained by designing and carrying out one or more new clinical trials. The analysis of clinical population data is useful to define a standard reference populations which, in turn, is useful to classify subjects for clinical trial enrolment or for selection of therapeutic treatment. In a preferred embodiment, the subjects included in the clinical population have been graded for the existence of the medical condition of interest. Grading of potential subjects can include, e.g., a standard physical exam or one or more lab tests. Alternatively, grading of subjects can include use of a gene expression pattern. For example, gene expression pattern is useful as grading criteria where there is a strong correlation between gene expression pattern and disease susceptibility or severity. Such standard reference population comprising subjects sharing gene expression pattern profile characteristic(s). For example, biomarker gene expression characteristic(s), are useful in the methods of the present invention to compare with the measured level of one or more gene expression product in a given subject. This gene expression product(s) useful in the methods of the present invention include, but are not limited to, e.g., characteristic mRNA associated with that particular genotype group or the polypeptide gene expression product of that genotype group. In one embodiment, a subject is classified or assigned to a particular genotype group or class based on similarity between the measured levels of a one or more biomarkers in the subject and the level of the one or more biomarkers observed in a standard reference population.

In one embodiment of the invention, a therapeutic treatment of interest is administered to each subject in a trial population, and each subject's response to the treatment is measured using one or more predetermined criteria. It is contemplated that in many cases, the trial population will exhibit a range of responses, and that the investigator will choose the number of responder groups (e.g., low, medium, high) made up by the various responses. In addition, the gene for each individual in the trial population is genotyped and/or haplotyped, which may be done before or after administering the treatment.

Statistical analysis methods, which may be used, are described in Fisher LD & vanBelle G, Biostatistics: A Methodology for the Health Sciences (Wiley-Interscience, New York, 1993). This analysis may also include a regression calculation of which polymorphic sites in the gene contribute most significantly to the differences in phenotype.

An alternative method for finding correlations between haplotype content and clinical responses uses predictive models based on error-minimizing optimization algorithms, one of which is a genetic algorithm (Judson R, "Genetic Algorithms and Their Uses in Chemistry" in Reviews in Computational Chemistry, Vol. 10, pp 1-73, Lipkowitz KB and Boyd DB, eds, (VCH Publishers, New York, 1997). Simulated annealing *(*Press et al., Numerical Recipes in C: The Art of Scientific Computing, Ch. 10 (Cambridge University Press, Cambridge, 1992), neural networks (Rich E & Knight K, Artificial Intelligence, 2nd Edition, Ch.10 (McGraw-Hill, New York, 1991), standard gradient descent methods (Press *et al., supra* Ch. 10), or other global or local optimization approaches can also be used.

Correlations may also be analyzed using analysis of variation (ANOVA) techniques to determine how much of the variation in the clinical data is explained by different subsets of the polymorphic sites in the gene. ANOVA is used to test hypotheses about whether a response variable is caused by, or correlates with, one or more traits or variables that can be measured. See, Fisher LD & vanBelle G, Biostatistics: A Methodology for the Health Sciences (Wiley-Interscience, New York, 1993), Ch. 10.

After both the clinical and polymorphism data have been obtained, correlations between individual response and genotype or haplotype content are created. Correlations may be produced in several ways. In one method, individuals are grouped by their genotype or haplotype (or haplotype pair) (also referred to as a polymorphism group), and then the averages and standard deviations of clinical responses exhibited by the members of each polymorphism group are calculated.

The skilled artisan can construct a mathematical model that predicts clinical response as a function of genotype or haplotype from the analyses described above. The identification of an association between a clinical response and a genotype or haplotype (or haplotype pair) for the gene may be the basis for designing a diagnostic method to determine those individuals who will or will not respond to the treatment, or alternatively, will respond at a lower level and thus may require more treatment, i.e., a greater dose of a drug. The diagnostic method may take one of several forms: for example, a direct DNA test (i.e., genotyping or haplotyping one or more of the polymorphic sites in the gene), a serological test, or a physical exam measurement. The only requirement is that there be a good correlation between the diagnostic test results and the underlying genotype or haplotype. In a preferred embodiment, this diagnostic method uses the predictive haplotyping method described above.

*Predictive Medicine.* The invention also pertains to the field of predictive medicine in which diagnostic assays, prognostic assays, and monitoring clinical trials are used for prognostic (predictive) purposes to treat prophylactically a subject. Accordingly, one aspect of the invention relates to diagnostic assays for determining biomarker molecule expression as well as biomarker molecule activity, in the context of a biological sample (e.g., blood, serum, cells, tissue) to thereby determine whether an individual is afflicted with a disease or disorder, or is at risk of developing a disorder, associated with aberrant biomarker molecule expression or activity.

The invention also provides for prognostic (or predictive) assays for determining whether an individual is at risk of developing a disorder associated with biomarker molecule expression or activity. Such assays can be used for prognostic or predictive purpose to thereby prophylactically treat an individual prior to the onset of a disorder characterized by or associated with a biomarker polypeptide.

The levels of certain polypeptides in a particular tissue (or in the blood) of a subject may be indicative of the toxicity, efficacy, rate of clearance or rate of metabolism of a given drug when administered to the subject. The methods described herein can also be used to determine the levels of such polypeptides in subjects to aid in predicting the response of such subjects to these drugs. Another aspect of the invention provides methods for determining mutant polypeptide activity in an individual to thereby select appropriate therapeutic or prophylactic compounds for that individual. Methods of the present invention allow for the selection of compounds (e.g., drugs) for therapeutic or prophylactic treatment of an individual based on the genotype of the individual (e.g., the genotype of the individual examined to determine the ability of the individual to respond to a particular compound.)

*Prognostic Assays.* The binding of a prognostic compound to a biomarker molecule, e.g., biomarker polypeptide or nucleic acid encoding a biomarker polypeptide, can be utilized to identify a subject having or at risk of developing a disorder associated with biomarker polypeptide expression or activity (which are described above). A prognostic compound is any compound which binds to or associates with a biomarker molecule, including, but not limited to, e.g., anti-biomarker polypeptide antibody, small molecule, nucleic acid, polypeptide, oligosaccharide, lipid, or combination thereof. Alternatively, the prognostic assays can be utilized to identify a subject having or at risk for developing the disease or disorder. Thus, the invention provides a method for identifying a disease or disorder associated with biomarker expression or activity in which a test sample is obtained from a subject and prognostic compound binding or activity is detected, wherein the presence of an alteration of prognostic compound binding or activity is diagnostic for a subject having , or at risk of developing, a disease or disorder associated with biomarker expression or activity. As used herein, a "test sample" refers to a biological sample obtained from a subject of interest. For example, a test sample can be a biological fluid (e.g., serum), cell sample, or tissue, or isolated nucleic acid or polypeptide derived therefrom.

Furthermore, the prognostic assays described herein can be used to determine whether a subject can be administered a compound (e.g., an agonist, antagonist, peptidomimetic, polypeptide, peptide, nucleic acid, small molecule, or other drug candidate) to treat a biomarker-associated disease or disorder. As used herein, the administration of a compound to a subject or patient includes self-administration and the administration by another. In one embodiment, the prognostic assays described herein are used to determine if a subject will be responsive to a compound. For example, such methods can be used to determine whether a subject can be effectively treated with a therapeutic compound for a biomarker-associated disorder (i.e., biomarker-associated medical condition). Thus, the invention provides methods for determining whether a subject can be effectively treated with a compound for a disorder associated with biomarker expression or activity in which a test sample is obtained and biomarker molecule is detected using prognostic compound (e.g., wherein the presence, or altered level of expression of, the biomarker molecule compared with the level of expression of the biomarker in a reference is diagnostic for a subject that can be administered the compound to treat a biomarker-associated disorder.

There are a number of diseases in which the degree of overexpression (or underexpression) of certain biomarker molecules, i.e., biomarker-associated disease or medical condition, is known to be indicative of whether a subject will develop a disease. Thus, the method of detecting a biomarker in a sample can be used as a method of predicting whether a subject will develop a disease. The level of a one or more biomoarkers in a suitable tissue or blood sample from a subject at risk of developing the disease is determined and compared with a suitable control, e.g., the level in subjects who are not at risk of developing the disease. The degree to which the one or more biomarkers is overexpressed (or underexpressed) in the sample compared with the control may be predictive of likelihood that the subject will develop the disease. The greater the overexpression (or underexpression) relative to the control, the more likely the subject will development the disease.

The methods described herein can be performed, for example, by utilizing prepackaged diagnostic kits comprising at least one probe reagent, e.g., anti-biomarker polypeptide antibody described herein, which can be conveniently used, e.g., in clinical setting to diagnose patients exhibiting symptoms or family history of a disease or illness involving a biomarker of the invention. Furthermore, any cell type or tissue in which a biomarker of the invention is expressed can be utilized in the prognostic assays described herein.

*Monitoring Clinical Efficacy.* Monitoring the influence of agents (e.g., drugs, compounds) on the expression or activity of a biomarker (e.g., the ability to modulate aberrant cell proliferation and/or differentiation) can be applied in basic drug screening and in clinical trials. For example, the effectiveness of an agent determined by a screening assay as described herein to increase biomarker gene expression, protein levels, or upregulate biomarker activity, can be monitored in clinical trials of subjects exhibiting decreased biomarker gene expression, protein levels, or downregulated biomarker activity. Alternatively, the effectiveness of an agent determined by a screening assay to decrease biomarker gene expression, protein levels, or downregulate biomarker activity, can be monitored in clinical trials of subjects exhibiting increased biomarker gene expression, protein levels, or upregulated biomarker activity. In such clinical trials, the expression or activity of a biomarker and, preferably, other genes that have been implicated in, for example, a proliferative disorder and cancers, can be used as a "read out" or marker of the responsiveness of a particular cell.

For example, genes, including genes encoding a biomarker of the invention, that are modulated in cells by treatment with an agent (e.g., compound, drug or small molecule) that modulates a biomarker activity (e.g., identified in a screening assay as described herein) can be identified. Thus, to study the effect of agents on cellular proliferation disorders, for example, in a clinical trial, cells can be isolated and RNA prepared and analyzed for the levels of expression of a biomarker and other genes implicated in the disorder. The levels of gene expression (i.e., a gene expression pattern) can be quantified by Northern blot analysis or RT-PCR, as described herein, or alternatively by measuring the amount of protein produced, by one of the methods as described herein, or by measuring the levels of activity of a gene or other genes. In this way, the gene expression pattern can serve as a marker, indicative of the physiological response of the cells to the agent. Accordingly, this response state may be determined before, and at various points during, treatment of the individual with the agent.

*Gene Expression and Subject Classification.* Standard control levels of a gene expression product are determined by measuring gene expression in different control groups. The control group gene expression levels are then compared with the measured level of a gene expression product in a given subject. This gene expression product could be the characteristic mRNA associated with that particular genotype group or the polypeptide gene expression product of that genotype group. The subject can be classified or assigned to a particular genotype group based on how similar the measured levels were compared to the control levels for a given group.

As one of skill in the art will understand, there will be a certain degree of uncertainty involved in making this determination. Therefore, the standard deviations of the control group levels can be used to make a probabilistic determination and the method of this invention are applicable over a wide range of probability-based genotype group determinations. Thus, for example, and not by way of limitation, in one embodiment, if the measured level of the gene expression product falls within 2.5 standard deviations of the mean of any of the control groups, then that individual may be assigned to that genotype group. In another embodiment if the measured level of the gene expression product falls within 2.0 standard deviations of the mean of any of the control groups then that individual may be assigned to that genotype group. In still another embodiment, if the measured level of the gene expression product falls within 1.5 standard deviations of the mean of any of the control groups then that individual may be assigned to that genotype group. In yet another embodiment, if the measured level of the gene expression product is 1.0 or less standard deviations of the mean of any of the control groups levels then that individual may be assigned to that genotype group.

Thus, this process allows determination, with various degrees of probability, which group a specific subject should be placed in, and such assignment to a genotype group would then determine the risk category into which the individual should be placed.

*Detection of Biomarker Gene Expression.* An exemplary method for detecting the presence or absence of mutant polypeptide or nucleic acid of the invention in a biological sample involves obtaining a biological sample from a test subject and contacting the biological sample with a compound, or a compound capable of detecting mutant polypeptide or nucleic acid (e.g., mRNA, genomic DNA) that encodes mutant polypeptide of the invention, such that the presence of mutant gene is detected in the biological sample. A compound for detecting mutant mRNA or mutant genomic DNA is a labelled nucleic acid probe capable of hybridizing to mutant mRNA or mutant genomic DNA. The nucleic acid probe can be, for example, a full-length mutant nucleic acid or a portion thereof, such as an oligonucleotide of at least 5,15, 30, 50,100, 250 or 500 nucleotides in length and sufficient to specifically hybridize under stringent conditions to mutant mRNA or mutant genomic DNA. Other suitable probes for use in the diagnostic assays of the invention are described herein. An example of a compound for detecting a mutant polypeptide of the invention is an antibody raised against mutant polypeptide of the invention, capable of binding to the mutant polypeptide, preferably an antibody with a detectable label. Antibodies can be polyclonal, or more preferably, monoclonal. An intact antibody, or a fragment thereof (e.g., Fab or F(ab')2) can be used. The term "labelled", with regard to the probe or antibody, is intended to encompass direct labelling of the probe or antibody by coupling (i.e., physically linking) a detectable substance to the probe or antibody, as well as indirect labelling of the probe or antibody by reactivity with another compound that is directly labelled. Examples of indirect labelling include detection of a primary antibody using a fluorescently-labelled secondary antibody and end-labelling of a DNA probe with biotin such that it can be detected with fluorescently-labelled streptavidin. That is, the detection method of the invention can be used to detect mutant mRNA, polypeptide, or genomic DNA of the invention in a biological sample *in vitro* as well as *in vivo.* For example, *in vitro* techniques for detection of mutant mRNA include Northern hybridizations and *in situ* hybridizations. *In vitro* techniques for detection of mutant polypeptide of the invention include enzyme linked immunosorbent assays (ELISAs), Western blots, immunoprecipitations, and immunofluorescence. *In vitro* techniques for detection of mutant genomic DNA include Southern hybridizations. Furthermore, *in vivo* techniques for detection of mutant polypeptide include introducing into a subject a labelled anti-mutant polypeptide antibody. For example, the antibody can be labelled with a radioactive marker whose presence and location in a subject can be detected by standard imaging techniques. In one embodiment, the biological sample contains polypeptide molecules from the test subject. Alternatively, the biological sample can contain mRNA molecules from the test subject or genomic DNA molecules from the test subject. A preferred biological sample is a peripheral blood leukocyte sample isolated by conventional means from a subject.

In practicing the present invention, many conventional techniques in molecular biology, protein biochemistry, cell biology, immunology, microbiology and recombinant DNA are used. These techniques are well-known and are explained in, *e.g.,* Current Protocols in Molecular Biology, Vols. I-III, Ausubel, Ed. (1997); Sambrook et al., Molecular Cloning: A Laboratory Manual, Second Edition (Cold Spring Harbor Laboratory Press, Cold Spring Harbor, NY, 1989); DNA Cloning: A Practical Approach, Vols. I and II, Glover, Ed. (1985); Oligonucleotide Synthesis, Gait, Ed. (1984); Nucleic Acid Hybridization, Hames & Higgins, Eds. (1985); Transcription and Translation, Hames & Higgins, Eds. (1984); Animal Cell Culture, Freshney, ed. (1986); Immobilized Cells and Enzymes (IRL Press, 1986); Perbal, A Practical Guide to Molecular Cloning; the series, Meth. Enzymol., (Academic Press, Inc., 1984); Gene Transfer Vectors for Mammalian Cells, Miller & Calos, Eds. (Cold Spring Harbor Laboratory, New York, 1987); and Meth. Enzymol., Vols. 154 and 155, Wu & Grossman, and Wu, eds., respectively. Methods to detect and measure mRNA levels *(i.e.,* gene transcription level) and levels of polypeptide gene expression products (i.e., gene translation level) are well-known in the art and include the use of nucleotide microarrays and polypeptide detection methods involving mass spectrometers and/or antibody detection and quantification techniques. See also, Strachan & Read, Human Molecular Genetics, Second Edition. (John Wiley and Sons, Inc., New York, 1999).

Techniques for the detection of gene expression of the genes described by this invention include, but are not limited to Northern blots, RT-PCT, real time PCR, primer extension, RNase protection, RNA expression profiling and related techniques. Techniques for the detection of gene expression by detection of the protein products encoded by the genes described by this invention include, but are not limited to, e.g., antibodies recognizing the protein products, western blots, immunofluorescence, immunoprecipitation, ELISAs and related techniques. These techniques are well known to those of skill in the art. Sambrook J. et al., Molecular Cloning: A Laboratoty Manual, Third Edition (Cold Spring Harbor Press, Cold Spring Harbor, New York, 2000). In one embodiment, the technique for detecting gene expression includes the use of a gene chip. The construction and use of gene chips are well known in the art. See, U.S. Pat Nos. 5,202,231; 5,445,934; 5,525,464; 5,695,940; 5,744,305; 5,795,716 and 5,800,992. See also, Johnston M, Curr. Biol., 8:R171-174 (1998); Iyer VR et al., Science, 283:83-87 (1999) and Elias P, "New human genome 'chip' is a revolution in the offing" Los Angeles Daily News (October 3, 2003).

In EXAMPLE 1 below, microarray hybridizations were conducted as recommended by the manufacturer of the microarray system (Affymetrix, Santa Clara, California; Expression analysis technical manual). Six samples from each treatment group were individually hybridized (no pooling) on the rat genome RAE230 2.0 gene expression probe array set containing >31 000 probe sets (Affymetrix, Inc., Santa Clara, California, USA).

Double stranded cDNA was synthesized with a starting amount of approximately 5 μg full-length total RNA using the Superscript Choice System (Invitrogen Life Technologies) in the presence of a T7-(dT)24 DNA oligonucleotide primer. Following synthesis, the cDNA was purified by phenol/chloroform/isoamylalcohol extraction and ethanol precipitation. The purified cDNA was then transcribed in vitro using the BioArray® High Yield RNA Transcript Labelling Kit (ENZO) in the presence of biotinylated ribonucleotides form biotin labelled cRNA. The labelled cRNA was then purified on an affinity resin (RNeasy, Qiagen), quantified and fragmented. An amount of approximately 10 μg labelled cRNA was hybridized for approximately 16 hours at 45°C to an expression probe array. The array was then washed and stained twice with streptavidin-phycoerythrin (Molecular Probes) using the GeneChip Fluidics Workstation 400 (Affymetrix). The array was then scanned twice using a confocal laser scanner (GeneArray Scanner, Agilent) resulting in one scanned image. This resulting ".dat-file" was processed using the MASS program (Affymetrix) into a ".cel-file". Raw data was converted to expression levels using a "target intensity" of 150.

*Determination of Marker Gene Transcription.* The determination of the level of the expression product of a marker gene in a biological sample, e.g.., the tissue or body fluids of an individual, may be performed in a variety of ways. Many expression detection methods use isolated RNA. For *in vitro* methods, any RNA isolation technique that does not select against the isolation of mRNA can be utilized for the purification of RNA from cells. See, e.g., Ausubel et al., ed., Curr. Prot. Mol. Biol. (John Wiley & Sons, NY, 1987-1999).

In one embodiment, the level of the mRNA expression product of a marker gene is determined. Methods to measure the level of a specific mRNA are well-known in the art and include Northern blot analysis, reverse transcription PCR and real time quantitative PCR or by hybridization to a oligonucleotide array or microarray. In other more preferred embodiments, the determination of the level of expression may be performed by determination of the level of the protein or polypeptide expression product of the gene in body fluids or tissue samples including but not limited to blood or serum.

In a particular embodiment, the level of mRNA corresponding to a marker can be determined both by in situ and by in vitro formats in a biological sample using methods known in the art. Additionally, large numbers of tissue samples can readily be processed using techniques well-known to those of skill in the art, such as, e.g., the single-step RNA isolation process of U.S. Pat. No. 4,843,155.

The isolated mRNA can be used in hybridization or amplification assays that include, but are not limited to, Southern or Northern analyses, PCR analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, e.g., a full-length cDNA, or a portion hereof, such as an oligonucleotide of at least 7, 15, 30, 50, 100, 250 or 500 nucleotides in length and sufficient to specifically hybridize under stringent conditions to an mRNA or genomic DNA encoding a marker of the present invention. Other suitable probes for use in the diagnostic assays of the invention are described herein. Hybridization of an mRNA with the probe indicates that the marker in question is being expressed.

In one format, the mRNA is immobilized on a solid surface and contacted with a probe, for example, by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the markers of the present invention.

An alternative method for determining the level of mRNA corresponding to a marker of the present invention in a sample involves the process of nucleic acid amplification, e.g., by RT-PCR (the experimental embodiment set forth by Mullis, U.S. Pat. No. 4,683,232); ligase chain reaction, Barany (1991), *supra;* self-sustained sequence replication, Guatelli et al., Proc, Natl. Acad. Sci, USA, 87:1874-1878 (1990); transcriptional amplification system, Kwoh et al., Proc. Natl. Acad. Sci. USA, 86:1173-1177 (1989); Q-Beta Replicase, Lizardi et al., Biol. Technolog, 6: 1197 (1988); rolling circle replication, U.S. Pat. No. 5,854,033; or any other nucleic acid amplification method, followed by the detection of the amplified molecules using techniques well-known to those of skill in the art. These detection schemes are especially useful for the detection of the nucleic acid molecules if such molecules are present in very low numbers. As used herein, amplification primers are defined as being a pair of nucleic acid molecules that can anneal to 5' or 3' regions of a gene (plus and minus strands, respectively, or *vice-versa)* and contain a short region in between. In general, amplification primers are from about 10-30 nucleotides in length and flank a region from about 50-200 nucleotides in length. Under appropriate conditions and with appropriate reagents, such primers permit the amplification of a nucleic acid molecule comprising the nucleotide sequence flanked by the primers.

As noted above, RT-PCR (real-time quantitative PCR) is one way to assess gene expression levels, *e.g.,* of genes of the invention (e.g., those containing SNPs and polymorphisms of interest). The RT-PCR assay utilizes an RNA reverse transcriptase to catalyze the synthesis of a DNA strand from an RNA strand, including an mRNA strand. The resultant DNA may be specifically detected and quantified and this process may be used to determine the levels of specific species of mRNA. One method for doing this is known under the Trademark TAQMAN (PE Applied Biosystems, Foster City, CA) and exploits the 5' nuclease activity of AMPLITAQ GOLD™ DNA polymerase to cleave a specific form of probe during a PCR reaction. This is referred to as a TAQMAN™ probe. See Luthra et al., Am. J. Pathol., 153: 63-68 (1998)). The probe consists of an oligonucleotide (usually ≈20 mer) with a 5'-reporter dye and a 3'-quencher dye. The fluorescent reporter dye, such as FAM (6-carboxyfluorescein), is covalently linked to the 5' end of the oligonucleotide. The reporter is quenched by TAMRA (6-carboxy-N,N,N',N'-tetramethylrhodamine) attached *via* a linker arm that is located at the 3' end. See Kuimelis et al., Nucl. Acids Symp. Ser., 37: 255-256 (1997) and Mullah et al., Nucl. Acids Res., 26 (4):1026-1031 (1998)). During the reaction, cleavage of the probe separates the reporter dye and the quencher dye, resulting in increased fluorescence of the reporter.

The accumulation of PCR products is detected directly by monitoring the increase in fluorescence of the reporter dye. See Heid et al., Genome Res., 6(6): 986-994 (1996). Reactions are characterized by the point in time during cycling when amplification of a PCR product is first detected rather than the amount of PCR product accumulated after a fixed number of cycles. The higher the starting copy number of nucleic acid target, the sooner a. significant increase in fluorescence is observed, (Gibson et al., Genome Res., 6: 995-1001 (1996)).

When the probe is intact, the proximity of the reporter dye to the quencher dye results in suppression of the reporter fluorescence primarily by Förster-type energy transfer. See Lakowicz et al., J. Biol. Chem., 258:4794-4801 (1983)). During PCR, if the target of interest is present, the probe specifically anneals between the forward and reverse primer sites. The 5'-3' nucleolytic activity of the AMPLITAQ GOLD™ DNA polymerase cleaves the probe between the reporter and the quencher only if the probe hybridizes to the target. The probe fragments are then displaced from the target, and polymerization of the strand continues. This process occurs in every cycle and does not interfere with the exponential accumulation of product. The 3' end of the probe is blocked to prevent extension of the probe during PCR.

The passive reference is a dye included in the TAQMAN™ buffer and does not participate in the 5' nuclease assay. The passive reference provides an internal reference to which the reporter dye signal can be normalized during data analysis. Normalization is necessary to correct for fluorescent fluctuations due to changes in concentration or volume.

Normalization is accomplished by dividing the emission intensity of the reporter dye by the emission intensity of the passive reference to obtain a ratio defined as the $R_n$ (normalized reporter) for a given reaction tube.

The threshold cycle or $C_t$ value is the cycle at which a statistically significant increase in $\Delta R_n$ is first detected. On a graph of $R_n$ vs. cycle number, the threshold cycle occurs when the sequence detection application begins to detect the increase in signal associated with an exponential growth of PCR product.

To perform quantitative measurements, serial dilutions of a cRNA (standard) are included in each experiment in order to construct a standard curve necessary for the accurate and fast mRNA quantification. In order to estimate the reproducibility of the technique, the amplification of the same cRNA sample may be performed multiple times.

Other technologies for measuring the transcriptional state of a cell produce pools of restriction fragments of limited complexity for electrophoretic analysis, such as methods combining double restriction enzyme digestion with phasing primers (see, e.g., EP 0 534858 A1), or methods selecting restriction fragments with sites closest to a defined mRNA end. See, e.g., Prashar et al., Proc. Natl. Acad. Sci., USA, 93(2):659-663 (1996)).

Other methods statistically sample cDNA pools, such as by sequencing sufficient bases, e.g., 20-50 bases, in each of multiple cDNAs to identify each cDNA, or by sequencing short tags, e.g., 9-10 bases, which are generated at known positions relative to a defined mRNA end pathway pattern. See, e.g., Velculescu, Science, 270:484-487 (1995). The cDNA level(s) in the samples are quantified and the mean, average and standard deviation of each cDNA is determined using by standard statistical means well-known to those of skill in the art. Bailey NTJ, Statistical Methods In Biology, Third Edition (Cambridge University Press, 1995).

Alternatively, the expression level can be provided as a relative expression level. To determine a relative expression level of a marker gene, the level of expression of the marker is determined for 10 or more samples of normal versus disease biological samples, preferably 50 or more samples, prior to the determination of the expression level for the sample in question. The mean expression level of each of the genes assayed in the larger number of samples is determined and this is used as a baseline expression level for the marker. The expression level of the marker determined for the test sample (absolute level of expression) is then divided by the mean expression value obtained for that marker. This provides a relative expression level. Preferably, the samples used in the baseline determination will be from subjects who do not have the polymorphism. The choice of the cell source is dependent on the use of the relative expression level. Using expression found in normal tissues as a mean expression score aids in validating whether the marker assayed is specific (versus normal cells). In addition, as more data is accumulated, the mean expression value can be revised, providing improved relative expression values based on accumulated data.

*Determination of Biomarker Gene Translation.* In another embodiment of the present invention, a polypeptide corresponding to a marker is detected. The detection of the biomarker polypeptide *(a.k.a.,* biomarker, marker, marker protein or marker polypeptide) expression product of the biomarker gene in body fluids or tissues can be used to determine the presence or absence of the polymorphism, and the relative level of the biomarker polypeptide expression product can be used to determine if the polymorphism is present in a homozygous or heterozygous state (and hence the risk category of the individual). That is, in another embodiment of the present invention, a polypeptide corresponding to a marker (i.e., biomarker polypeptide) is detected. The level of this biomarker polypeptide gene expression product in body fluids or tissue sample may be determined by any means known in the art.

*Immunological Detection Methods.* Expression of the protein encoded by the gene(s) of the invention can be detected by a probe which is detectably labelled, or which can be subsequently labelled. Generally, the probe is an antibody that recognizes the expressed protein. A variety of formats can be employed to determine whether a sample contains a biomarker protein that binds to a given antibody. Immunoassay methods useful in the detection of biomarker polypeptides of the present invention include, but are not limited to, e.g., dot blotting, western blotting, protein chips, competitive and non-competitive protein binding assays, enzyme-linked immunosorbant assays (ELISA), immunohistochemistry, fluorescence activated cell sorting (FACS), and others commonly used and widely-described in scientific and patent literature, and many employed commercially. A skilled artisan can readily adapt known protein/antibody detection methods for use in determining whether cells express a marker of the present invention and the relative concentration of that specific polypeptide expression product in blood or other body tissues. Proteins from individuals can be isolated using techniques that are well-known to those of skill in the art. The protein isolation methods employed can, e.g., be such as those described in Harlow & Lane, Antibodies: A Laboratory Manual (Cold Spring Harbor, New York, 1988)).

An intact antibody, or a fragment thereof, e.g., Fab or $F(ab')_2$ can be used. Antibody fragments, which recognize specific epitopes, may be generated by known techniques. For example, such fragments include, but are not limited to, the $F(ab')_2$ fragments which can be produced by pepsin digestion of the antibody molecule and the Fab fragments which can be generated by reducing the disulfide bridges of the $F(ab')_2$ fragments. Alternatively, Fab expression libraries may be constructed (see Huse et al., Science, 246:1275-1281 (1989)), to allow rapid and easy identification of monoclonal Fab fragments with the desired specificity.

The term "labelled", with regard to the probe or antibody, is intended to encompass direct-labelling of the probe or antibody by coupling, *i.e.,* physically linking, a detectable substance to the probe or antibody, as well as indirect-labelling of the probe or antibody by reactivity with another reagent that is directly-labelled. Examples of indirect labelling include detection of a primary antibody using a fluorescently-labelled secondary antibody and end-labelling of a DNA probe with biotin such that it can be detected with fluorescently-labelled streptavidin.

Monoclonal antibodies (mAbs), which are homogeneous populations of antibodies to a particular antigen, may be obtained by any technique that provides for the production of antibody molecules by continuous cell lines in culture. These include, but are not limited to, the hybridoma technique of Kohler & Milstein, Nature, 256:495-497 (1975); and U.S. Pat. No. 4,376,110; the human B-cell hybridoma technique of Kosbor et al., Immunol. Today, 4:72 (1983); Cole et al., Proc. Natl. Acad. Sci., USA, 80:2026-2030 (1983); and the EBV-hybridoma technique, Cole et al., Monoclonal Antibodies and Cancer Therapy, pp. 77-96 (Alan R. Liss, Inc., 1985). Such antibodies may be of any immunoglobulin class including IgG, IgM, IgE, IgA, IgG and any subclass thereof. The hybridoma producing the mAb of this invention may be cultivated *in vitro or in vivo.* Production of high titres of mAbs *in vivo* makes this the presently preferred method of production.

In addition, techniques developed for the production of "chimaeric antibodies" (see Morrison et al., Proc. Natl Acad. Sci. USA, 81:6851-6855 (1984); Neuberger et al., Nature, 312: 604-608 (1984); and Takeda et al., Nature, 314: 452-454 (1985)), by splicing the genes from a mouse antibody molecule of appropriate antigen specificity together with genes from a human antibody molecule of appropriate biological activity can be used. A chimaeric antibody is a molecule in which different portions are derived from different animal species, such as those having a variable or hypervariable region derived form a murine mAb and a human immunoglobulin constant region.

Alternatively, techniques described for the production of single chain antibodies, U.S. Pat. No. 4,946,778; Bird, Science, 242:423-426 (1988); Huston et al., Proc. Natl Acad. Sci. USA, 85:5879-5883 (1988); and Ward et al., Nature, 334:544-546 (1989), can be adapted to produce differentially expressed gene single-chain antibodies. Single-chain antibodies are formed by linking the heavy- and light-chain fragments of the Fv region *via* an amino acid bridge, resulting in a single-chain polypeptide.

More preferably, techniques useful for the production of "humanized antibodies" can be adapted to produce antibodies to the proteins, fragments or derivatives thereof. Such techniques are disclosed in U.S. Pat, Nos. 5,932,448; 5,693,762; 5,693,761; 5,585,089; 5,530,101; 5,569,825; 5,625,126; 5,633,425; 5,789,650; 5,661,016; and 5,770,429. Antibody fragments, which recognize specific epitopes, may be generated by known techniques. For example, such fragments include, but are not limited to, the $F(ab')_2$ fragments which can be produced by pepsin digestion of the antibody molecule and the Fab fragments which can be generated by reducing the disulfide bridges of the $F(ab')_2$ fragments. Alternatively, Fab expression libraries may be constructed (see Huse et al., Science, 246:1275-1281 (1989)), to allow rapid aid easy identification of monoclonal Fab fragments with the desired specificity.

In one format, antibodies or antibody fragments can be used in methods, such as Western blots or immunofluorescence techniques, to detect the expressed proteins. In such uses, it is generally preferable to immobilize either the antibody or proteins on a solid support. Suitable solid phase supports or carriers include any support capable of binding an antigen or an antibody. Well-known supports or carriers include glass, polystyrene, polypropylene, polyethylene, dextran, nylon, amylases, natural and modified celluloses, polyacrylamides, gabbros and magnetite.

The extent to which the known proteins are expressed in a biological sample is determined by immunoassay methods that utilize the antibodies described above. Particularly preferred, for ease of detection, is the sandwich ELISA, of which a number of variations exist, all of which are intended to be used in the methods and assays of the present invention. For example, in a typical forward assay, unlabeled antibody is immobilized on a solid substrate and the sample to be tested brought into contact with the bound molecule after a suitable period of incubation, for a period of time sufficient to allow formation of an antibody-antigen binary complex. At this point, a second antibody, labelled with a reporter molecule capable of inducing a detectable signal, is then added and incubated, allowing time sufficient for the formation of a ternary complex of antibody-antigen-labelled antibody. Any unreacted material is washed away, and the presence of the antigen is determined by observation of a signal, or may be quantitated by comparing with a control sample containing known amounts of antigen. Variations on the forward assay include the simultaneous assay, in which both sample and antibody are added simultaneously to the bound antibody, or a reverse assay in which the labelled antibody and sample to be tested are first combined, incubated and added to the unlabelled surface bound antibody. These techniques are well-known to those skilled in the art, and the possibility of minor variations will be readily apparent. As used herein, "sandwich assay" is intended to encompass all variations on the basic two-site technique. For the immunoassays of the present invention, the only limiting factor is that the labelled antibody must be an antibody that is specific for the protein expressed by the gene of interest.

*Two-Dimensional Gel Electrophoresis.* Proteins can be separated by two-dimensional gel electrophoresis systems and then identified and/or quantified. Two-dimensional gel electrophoresis is well-known in the art and typically involves isoelectric focusing along a first dimension followed by SDS PAGE electrophoresis along a second dimension. (See, e.g., Hames et al., Gel Electrophoresis of Proteins: A Practical Approach (IRL Press, NY, 1990); Shevchenko et al., Proc Natl. Acad. Sci. USA, 93:14440-14445 (1996); Sagliocco et al., Yeast, 12:1519-1533 (1996); and Lander, Science 274: 536-539 (1996)). The resulting electropherograms can be analyzed by numerous techniques, including mass spectrometric techniques, western blotting and immunoblot analysis using polyclonal and monoclonal antibodies, and internal and N-terminal micro-sequencing. Using these techniques, it is possible to identify a substantial fraction of all the proteins produced under given physiological conditions, including in cells, e.g., in yeast, exposed to a drug, or in cells modified by, e.g., deletion or over-expression of a specific gene.

*Mass Spectroscopy.* The identity and the expression level of biomarker polypeptide can both be determined using mass spectroscopy technique (MS). MS-based analysis methodology is use for analysis of isolated biomarker polypeptide as well as analysis of biomarker polypeptide in a biological sample. MS formats for use in analyzing a biomarker polypeptide include ionization (I) techniques, such as, but not limited to, MALDI, continuous or pulsed ESI and related methods, such as ionspray or thermospray, and massive cluster impact (MCI). Such ion sources can be matched with detection formats, including linear or non-linear reflectron TOF, single or multiple quadrupole, single or multiple magnetic sector, Fourier transform ion cyclotron resonance (FTICR), ion trap and combinations thereof such as ion-trap/TOF. For ionization, numerous matrix/wavelength combinations (MALDI) or solvent combinations (ESI) can be employed. Sub-attomole levels of protein have been detected, e.g., using ESI MS (Valaskovic et al., Science, 273: 1199-1202 (1996)) and MALDI MS (Li et al., J Am. Chem. Soc., 118:1662-1663 (1996)).

For MS analysis, the biomarker polypeptide can be solubilised in an appropriate solution or reagent system. The selection of a solution or reagent system, e.g., an organic or inorganic solvent, will depend on the properties of the biomarker polypeptide and the type of MS performed, and is based on methods well-known in the art. See, e.g., Vorm et al., Anal. Chem., 61:3281 (1994) for MALDI; and Valaskovic et al., Anal. Chem., 67:3802 (1995), for ESI. MS of peptides also is described, e.g., in International PCT Application No. WO 93/24834 and U.S. Pat. No. 5,792,664. A solvent is selected that minimizes the risk that the biomarker polypeptide will be decomposed by the energy introduced for the vaporization process. A reduced risk of biomarker polypeptide decomposition can be achieved, e.g., by embedding the sample in a matrix. A suitable matrix can be an organic compound such as a sugar, e.g., a pentose or hexose, or a polysaccharide such as cellulose. Such compounds are decomposed thermolytically into $CO_2$ and $H_2O$ such that no residues are formed that can lead to chemical reactions. The matrix can also be an inorganic compound, such as nitrate of ammonium, which is decomposed essentially without leaving any residue. Use of these and other solvents is known to those of skill in the art. See, e.g., US. Pat. No. 5,062,935.

Electrospray MS has been described by Fenn et al., J. Phys. Chem., 88:4451-4459 (1984); and in PCT Application No. WO 90/14148; and current applications are summarized in review articles. See Smith et al., Anal. Chem., 62: 882-89 (1990); and Ardrey, Spectroscopy, 4:10-18 (1992). With ESI, the determination of molecular weights in femtomole amounts of sample is very accurate due to the presence of multiple ion peaks, all of which can be used for mass calculation.

Matrix Assisted Laser Desorption (MALDI) is one preferred method among the MS methods herein. Methods for performing MALDI are well-known to those of skill in the art. Numerous methods for improving resolution are also known. For example, resolution in MALDI-TOF-MS can be improved by reducing the number of high energy collisions during ion extraction. See, *e.g.,* Juhasz et al., Analysis, Anal. Chem., 68:941-946 (1996); see also, *e.g.,* U.S. Pat. No. 5,777,325; 5,742,049; 5,654,545; 5,641,959; 5,654,545, and 5,760,393 for descriptions of MALDI and delayed extraction protocols. MALDI-TOF: MS has been described by Hillenkamp *et al.,* Burlingame & McCloskey, eds., pp. 49-60 (Elsevier Science Publ., 1990).

In a preferred embodiment, the level of the biomarker protein in a biological sample, e.g., body fluid or tissue sample, maybe measured by means of mass spectrometric (MS) methods including, but not limited to, those techniques known in the art as matrix-assisted laser desorption/ionization, time-of-flight mass spectrometry (MALDI-TOF-MS) and surfaces enhanced for laser desorption/ionization, time-of-flight mass spectrometry (SELDI-TOF-MS) as further detailed below.

*MASLDI-TOF-MS Protein Detection Technique.* In some preferred embodiments, the detection of specific proteins or polypeptide gene expression products in a biological sample, e.g., body fluid or tissue sample, is performed by means of MS, especially matrix-assisted laser desorption/ionization, time-of flight mass spectrometry (MASLDI-TOF-MS). These techniques have been used to analyze macromolecules, such as proteins or biomolecules and utilize sample probe surface chemistries that enable the selective capture and desorption of analytes, including intact macromolecules, directly from the probe surface into the gas (vapour phase), and in the most preferred embodiments without added chemical matrix.

In other embodiments a variety of other techniques for marker detection using mass spectroscopy can be used. See Bordeaux Mass Spectrometry Conference Report, Hillenkamp, ed., pp. 354-362 (1988); Bordeaux Mass Spectrometry Conference Report, Karas & Hillenkamp, Eds., pp. 416-417 (1988); Karas & Hillenkamp, Anal. Chem., 60:2299-2301 (1988); and Karas et al., Biomed Environ Mass Spectrum, 18:841-843 (1989). The use of laser beams in TOF-MS is shown, e.g., in U.S. Pat. Nos., 4,694,167; 4,686,366; 4,295,046; and 5,045,694, which are incorporated herein by reference in their entireties. Other MS techniques allow the successful volatilization of high molecular weight biopolymers, without fragmentation, and have enabled a wide variety of biological macromolecules to be analyzed by mass spectrometry.

*Surfaces Enhanced for Laser Desorption/Ionization (SELDI).* In a preferred embodiment of the present invention, other techniques are used which employ new MS probe element compositions with surfaces that allow the probe element to actively participate in the capture and docking of specific analytes, described as Affinity Mass Spectrometry (AMS). Several types of new MS probe elements have been designed with Surfaces Enhanced for Affinity Capture (SEAC). See Hutchens & Yip, Rapid Common. Mass Spectrom., 7:576-580 (1993). SEAC probe elements have been used successfully to retrieve and tether different classes of biopolymers, particularly proteins, by exploiting what is known about protein surface structures and biospecific molecular recognition.

In another preferred embodiment of the present invention, the method of detection to be used with the methods of this invention uses a general category of probe elements, i.e., sample presenting means with surfaces enhanced for laser desorption/ionization (SELDI). See SELDI patents U.S. Pat. Nos. 5,719,060; 5,894,063; 6,020,208; 6,027,942; 6,124,137; and US. Patent Application No. U.S. 2003/0003465.

A polypeptide of interest can be attached directly to a support *via* a linker. Any linkers known to those of skill in the art to be suitable for linking peptides or amino acids to supports, either directly or *via* a spacer, may be used. For example, the polypeptide can be conjugated to a support, such as a bead, through means of a variable spacer. Linkers, include, Rink amide linkers (see, *e.g.,* Rink, Tetrahedron Lett., 28:3787 (1976)); trityl chloride linkers (see, e.g., Leznoff, Ace Chem. Res. 11:327 (1978)); and Merrifield linkers. (See, *e.g.,* Bodansky et al., Peptide Synthesis, Second Edition (Academic Press, New York, 1976)). For example, trityl linkers are known. (See, e.g., U.S. Pat. Nos. 5,410,068 and 5,612,474). Amino trityl linkers are also known, (See, e.g., U.S. Pat. No. 5,198,531). Other linkers include those that can be incorporated into fusion proteins and expressed in a host cell. Such linkers may be selected amino acids, enzyme substrates or any suitable peptide. The linker may be made, e.g., by appropriate selection of primers when isolating the nucleic acid. Alternatively, they may be added by post-translational modification of the protein of interest.

*Use of a Pin Tool to Immobilize a Polypeptide.* The immobilization of a polypeptide of interest to a solid support using a pin tool can be particularly advantageous. Pin tools include those disclosed herein or otherwise known in the art. See, e.g., U.S. Application Serial Nos. 08/786,988 and 08/787,639; and International PCT Application No. WO 98/20166. A pin tool in an array, e.g., a 4 x 4 array, can be applied to wells containing polypeptides of interest. Where the pin tool has a functional group attached to each pin tip, or a solid support, e.g., functionalized beads or paramagnetic beads, are attached to each pin, the polypeptides in a well can be captured (1 pmol capacity). Polypeptides of interest, particularly biomarker polypeptides, can be immobilized due to contact with the pin tool. Further immobilization can result by applying an electrical field to the pin tool. See, *e.g.,* Juhasz et al., Analysis, Anal. Chem., 68:941-946 (1996), and see also, *e.g.,* U.S. Patent Nos. 5,777,325;5,742,049; 5,654,545; 5,641,959; and 5,760,393 for descriptions of MALDI and delayed extraction protocols. Pin tools can be useful for immobilizing polypeptides of interest in spatially addressable manner on an array. Such spatially addressable or pre-addressable arrays are useful in a variety of processes, including, for example, quality control and amino acid sequence diagnostics. The pin tools described in the U.S. Application Nos. 08/786,988 and 08/787,639 and International PCT Application No. WO 98/20166 are serial and parallel dispensing tools that can be employed to generate multi-element arrays of polypeptides on a surface of tie solid support.

*Other Aspects of the Biological State.* In various embodiments of the present invention, aspects of the biological activity state, or mixed aspects can be measured in order to obtain drug and pathway responses. The activities of proteins relevant to the characterization of cell function can be measured, and embodiments of this invention can be based on such measurements. Activity measurements can be performed by any functional, biochemical or physical means appropriate to the particular activity being characterized. Where the activity involves a chemical transformation, the cellular protein can be contacted with natural substrates, and the rate of transformation measured. Where the activity involves association in multimeric units, e.g., association of an activated DNA binding complex with DNA, the amount of associated protein or secondary consequences of the association, such as amounts of mRNA transcribed, can be measured. Also, where only a functional activity is known, e.g., as in cell cycle control, performance of the function can be observed. However known and measured, the changes in protein activities form the response data analyzed by the methods of this invention. In alternative and non-limiting embodiments, response data may be formed of mixed aspects of the biological state of a cell. Response data can be constructed from, e.g., changes in certain mRNA abundances, changes in certain protein abundances and changes in certain protein activities.

The following EXAMPLES are presented in order to more fully illustrate the preferred embodiments of the invention. These EXAMPLES should in no way be construed as limiting the scope of the invention, as defined by the appended claims.

EXAMPLE 1

GENOMICS EXPLORATORY STUDY IN A RAT SPINAL CORD INJURY MODEL AFTER TREATMENT WITH ANTI-NOGO A ANTIBODY 11C7; MICROARRAY GENE EXPRESSION ANALYSIS

*Purpose.* The purpose of this EXAMPLE is to show gene expression changes resulting from anti-Nogo-A antibody-treatment after spinal cord injury in rats in order to identify biomarker candidates of treatment efficacy, mechanism of action or of any potential adverse effects.

*Study design.* The in life part of the EXAMPLE was performed as follows: A total of 40 adult female Lewis rats *(Rattus norwegicus,* 160-190 g) were obtained from a Specific Pathogen Free (SPF) breeding colony (R.Janvier, Le Genest-St-Isle, France) and kept as groups of 4 — 6 animals in standardized cages (type 4, Macrolon, Indulab, Hanstedt, Germany) on a 12 hour light/dark cycle on a standard regime with food and water *ad libitum.*

The rats were randomized to five groups: Two of 16 underwent spinal hemisection and received either IgG or anti-Nogo A antibody (11C7). The third group, a naïve group of eight, did not undergo surgery and did not receive any treatment, as follows:

Treatment groups:

1) IgG-treated 7 days
2) Nogo-A-treated 7 days
3) IgG-treated 14 days
4) Nogo-A-treated 14 days
5) Naïve controls

Animals were coded with random numbers and the experimenters were blind with regard to the treatments throughout all the steps and phases of the experiment. All the treatments, surgical procedure, and sacrifice and the initial data-analysis was carried out in blinded manner. The antibodies were coded "orange" and "yellow".

*Antibodies.* Anti Nogo-A antibody 11C7: Mouse monoclonal antibody (mAb) 11C7, raised against a 18aa peptide Nogo-A corresponding to rat sequence aa 623-640; used at a concentration of 3mg/ml in PBS. The control antibody was a mouse monoclonal IgG directed against plant lectin used at a concentration of 3mg/ml in PBS. The biochemical and neutralizing properties of both antibodies are described in Oertle T et al., J. Neurosci. 23:5393-5406 (2003).

*Surgical procedures.* Animals were anesthetized with a subcutaneous injection of Hypnorm (120 $\mu$l / 200 g body weight Janssen Pharmaceutics, Beerse, Belgium), and Dormicum (0.75 mg in 150 $\mu$l per 200 g body weight Roche Pharmaceuticals, Basle, Switzerland). Vitamin A containing eye ointment (Blache, Chauvin Novopharm AG, Switzerland) was applied to protect the eyes from dehydration during the relatively long operation procedure.

A T-shaped lesion to include the dorsal half of the spinal cord with the main as well as the dorso-lateral and ventro-medial projections of the CST with iridectomy scissors and a sharp, pointed blade was made at thoracic level T8.

A fine intrathecal catheter (32 gauge from RECATHCO, Allison Park, Pennsylvania, USA) was inserted from the lumbar level L2/L3 and pushed up to T9, delivering antibodies by osmotic minipumps (5 $\mu$/h, 3.1 $\mu$g/$\mu$l, Alzet© 2ML2, Charles River Laboratories, Les Oncins, France) to the lesion site for 2 weeks. After surgery, the animals were kept on a thermostatically regulated heating pad until completely awake. No pain killers or antibiotics were given in order not to influence the results. Ringer solution (Fresenius Kabi AG, Stans, Switzerland) was given subcutaneously when animals showed signs of dehydration.

*Sacrifice.* After 1 and 2 weeks respectively, the rats were slightly anesthetized with Isoflurane and decapitated.

The naïve animals were sacrificed together with the one week group.

1 ml of whole blood was collected into an EDTA tube, mixed, diluted with 1 ml NaCl 0.9% transferred to a tube containing Fas. The mixture was frozen on dry ice. Approx. 1 ml of whole blood was collected in a Lith/Hep tube, mixed and kept on ice before centrifuged at 2000x g for 10 min (cooled). The supernatant (plasma) was frozen on dry ice.

Brain and spinal cord were exposed, the specific tissue domains were sampled and immediately frozen on dry ice.

*Experimental animals.* Number of animals per group and sex: 8 females/group, total 40. Age: 8-9 weeks. Weight: 160-190 g.

TABLE 1

Study design, animal allocation and test item dosages.

|  | Group 1 | Group 2 | Group 3 | Group 4 | Group 5 |
|---|---|---|---|---|---|
| Compound | 11C7 | IgG | 11C7 | IgG | Naïve animals |
| Treatment duration | 7 days | 7 days | 14 days | 14 days | No treatment |
| Route and frequency of administration | i.t. Continuous via minipump. | i.t. Continuous via minipump. | i.t. Continuous via minipump. | i.t. Continuous via minipump. | No treatment. |
| Time between last dose and sacrifice | 0 h | 0 h | 0 h | 0 h | No treatment |
| Number of animals at treatment start | 8 | 8 | 8 | 8 | 8 |
| Animal numbers | 1-16 | 17-32 | 33-48 | 49-64 | 113-128 |

*Tissue sampling.* The following tissues were sampled:

1) Thoracic spinal cord at the level of lesion (T8)
2) Thoracic spinal cord above the lesion (T1-T7)
3) Cervical spinal cord
4) Lumbal spinal cord
5) Brain — frontal cortex
6) Brain — motor and somatosensory cortex
7) Brain- occipital cortex
8) Brain- striatum
9) Brain- hippocampus
10) Brainstem
12) Lumbal DRGs
13) Blood cells
14) Serum
15) CSF

Samples were stored on dry ice and subsequently in a deep-freezer at -80°C until further use. The following tissue samples were processed for gene expression profiling and analyzed:

1) Thoracic spinal cord at the level of lesion (T8)
2) Thoracic spinal cord above the lesion (T1-T7)
3) Lumbal spinal cord
4) Brain — frontal cortex
5) Brain — motor and somatosensory cortex
6) Blood cells

The brain was divided into two hemispheres and left was kept intact for further confirmation of the microarray findings using in situ hybridization/ immunohistochemistry while the right one to be used for dissection.

*RNA extraction and purification.* Briefly, total RNA was obtained by acid guanidinium thiocyanate-phenol-chloroform extraction (Trizol, Invitrogen Life Technologies) from each frozen tissue section and the total RNA was then purified on an affinity resin (Rneasy, Qiagen) according to the manufacturer's instructions. and quantified. Total RNA was quantified by the absorbance at $\lambda$ = 260 nm ($A_{260nm}$), and the purity was estimated by the ratio $A_{260nm}/A_{280nm}$. Integrity of the RNA molecules was confirmed by non-denaturing agarose gel electrophoresis using Agilent 2100 Bioanalyzer (Agilent Technologies, Palo Alto, California, USA). An aliquot of each individual RNA sample was kept for confirmation of microarray finding by real-time, fluorescence-based PCR (TAQMAN; Applera). RNA was stored at -80°C until analysis.

*Microarray experiment.* All microarray hybridizations were conducted as recommended by the manufacturer of the microarray system (Affymetrix, Santa Clara, California; Expression analysis technical manual). Six samples from each treatment group were individually hybridized (no pooling) on the rat genome RAE230 2.0 gene expression probe array set containing > 31 000 probe sets (Affymetrix, Inc., Santa Clara, California, USA).

Double stranded cDNA was synthesized with a starting amount of approximately 5 μg full-length total RNA using the Superscript Choice System (Invitrogen Life Technologies) in the presence of a T7-(dT) 24 DNA oligonucleotide primer. Following synthesis, the cDNA was purified by phenol/chloroform/isoamylalkohol extraction and ethanol precipitation. The purified cDNA was then transcribed in vitro using the BioArray® High Yield RNA Transcript Labelling Kit (ENZO) in the presence of biotinylated ribonucleotides form biotin labelled cRNA. The labelled cRNA was then purified on an affinity resin (RNeasy, Qiagen), quantified and fragmented. An amount of approximately 10 μg labelled cRNA was hybridized for approximately 16 hours at 45°C to an expression probe array. The array was then washed and stained twice with streptavidin-phycoerythrin (Molecular Probes) using the GeneChip Fluidics Workstation 400 (Affymetrix). The array was then scanned twice using a confocal laser scanner (GeneArray Scanner, Agilent) resulting in one scanned image. This resulting ".dat-file" was processed using the MASS program (Affymetrix) into a ".cel-file". Raw data was converted to expression levels using a "target intensity" of 150.

*Data analysis.* Initial data-analysis of the dataset for spinal cord tissues T8 (at the level of injury) and proximal to the injury, T1-7 was performed blindly. Analysis resulted in identifying samples coded "orange" as the 11 1C7-treated group after which the code was broken and the sample identity confirmed. Remaining of the analysis was not blinded.

*Quality control.* The following quality measures were studied for each sample: Scaling factor, background, percent present calls, AFFX-GAPDH 3': AFFX-GAPDH 5'-ratio, AFFX-GAPDH 3' variance, AFFX-Beta-actin 3': AFFX-Beta-actin 5'-ratio. Attention was paid to the homogeneity of the data. Average and standard deviation of the background noise level determined the raw data restriction value used in the consequent analysis. GAPDH 3' variance is a measure of variation among individual samples and can be used as a guideline for a reliable fold difference.

*Principal component analysis.* Principal component analysis (PCA) including all probe sets on Rat Genome 2.0 (n=15 866) as variables was performed to identify outlier microarrays after log-transformation and centralization of the data using Simca-P 10.0 software (Umetrics, Umeå, Sweden). After removal of technical outliers, PCA was repeated using GeneSpring (Silicon Genetics, Redwood City, California, USA) version 7.0.

*Data normalization.* After QC, MASS normalized microarray data was imported to GeneSpring version 7.0. (Silicon Genetics). Individual experiments were generated for each tissue separately. Each experiment was normalized as follows: Values below 0 were set to 0.1. Each measurement was divided by the 50.0th percentile of all measurements in that sample. Finally, per gene normalization was performed by normalizing to the expression value of the median of naïve samples.

*Identification of differentially expressed genes.* Differentially expressed genes between the vehicle and the treatments were identified within each experiment based on the following restrictions: (1) Prefiltering restrictions: Probe sets included in further analysis had to flagged present in 4/6 of replicates in any condition. Raw data signal intensity had to be minimum 50 in at least one of the treatment groups. (2) Statistical restriction: p<0.05 (Welch t-test (parametric)). Similar statistical restriction was always applied to different groups to be compared and is mentioned in each comparison.

*Gene Set Enrichment Analysis (GSEA).* An in-house implementation of the Gene Set Enrichment Analysis method was used to analyze microarray data. Genes with expression levels below 100 on more than 75% of the chips are discarded as low- or non-expressed. Microarray results are then analyzed in a series of pairwise comparisons between sets of condition (*e.g.* treated vs. control). Each gene's relative expression level under *condition,* and *condition$_2$* is computed as an expression ratio r$_i$

$$r_i = \frac{\mu_{i,1}}{\mu_{i,2}}$$

where $\mu i_j$ is the average expression value for gene i under *condition$_j$.* The genes are then sorted according to their expression ratios such that those genes with higher expression under $condition_1$ than $condition_2$ are at the top of the list. Next, the collection of available gene sets are projected onto the sorted list. This step in essence applies *a priori* biological knowledge to the experimental data to identify functionally related genes that are expressed in a coordinated fashion. Gene sets are processed one at a time. For gene set G each expression ratio $r_i$ is labelled 'in' the gene set if $gene_i \in$ G and 'out' of the gene set if $gene_j \notin$ G . A two-tailed Wilcoxon rank-sum test is calculated to determine if the genes labelled 'in' gene set G are enriched at either the top or bottom of the sorted list. The false discovery rate method of Storey JD & Tibshirani R, Proc Natl Acad Sci USA 100:9440-9445 (2003) is applied to transform p-values to multiple testing corrected q-values. The output from GSEA is a list of q-values $(q_1, q_2, ..., q_N)$ and labels $(l_1, l_2, ..., l_N)$, $l_i \in$ (*top, bottom)* that correspond to the N available gene sets. A small q-value $q_i$ indicates that the genes in gene set $G_i$ are significantly enriched at either the top or bottom of the list of expression ratios.

*Results.* Initial data-analysis of the dataset for spinal cord tissues T8 (at the level of injury) and proximal to the injury, T1-7 was performed blindly. Analysis resulted in identifying samples coded "orange" as the 11C7-treated group after which the code was broken and the sample identity confirmed. Remaining of the analysis was not blinded.

*Spinal cord T8 (At the level of injury).* Welch T-test comparing the IgG-treated group to the 11 C7-treated group resulted in 643 and 449 differentially expressed genes after one week and two weeks of treatment, respectively. The average fold change of the top 100 largest fold changes was 1.93±1.06 after one week of treatment and 1.31 ±0.07 after two weeks of treatment. The top 100 gene expression changes after one week of treatment are listed in TABLE 4 and after two weeks of treatment, in TABLE 5 in EXAMPLE 2. 90% of the top 20 transcripts were downregulated at one week after 11C7 treatment (whereas of the total differentially expressed ones, 41 % were downregulated). Interestingly, among those there were 7 transcripts encoding for proteins related to extracellular matrix (ECM) and wound healing and/or scarring (asporin precursor, dermatopontin, collagen), 2 secreted frizzled-like proteins (Sfr12 and 4), two IGF-binding proteins (Igfbp 5 and 6, negative regulators of IGF) and myocilin/TIGR, which has been recently shown to inhibit neurite outgrowth and to be upregulated in chronic glial scar after CNS injury. Jurynec MJ et al., Mol. Cell. Neurosci. 23: 69-80 (2003).

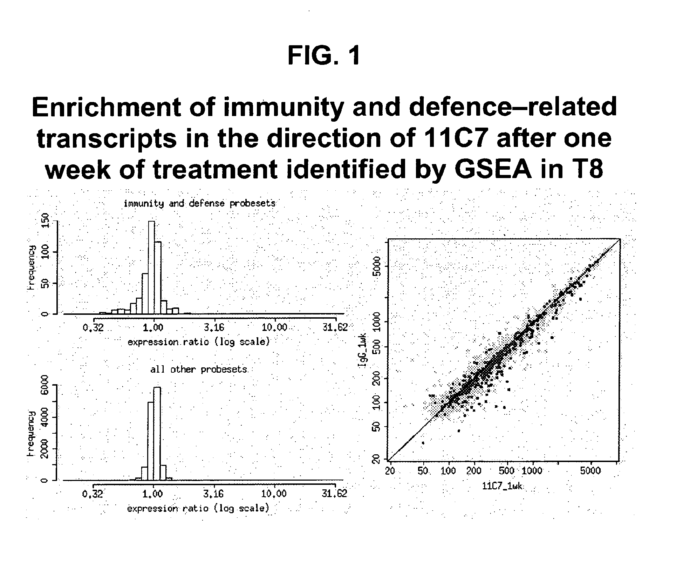

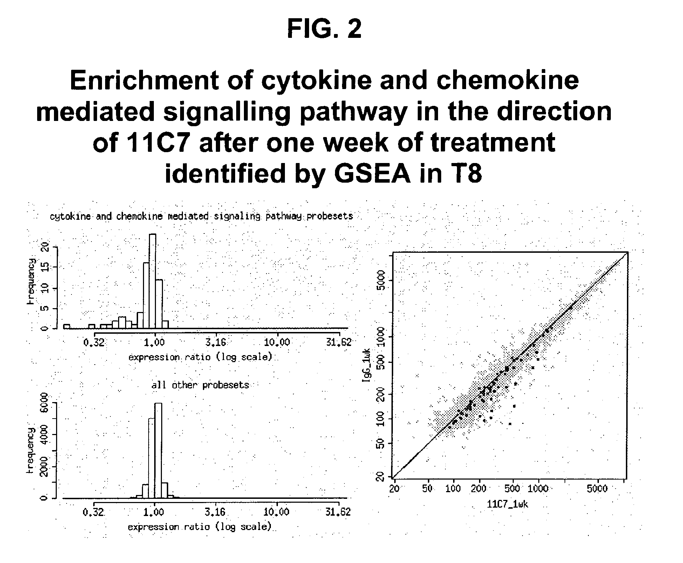

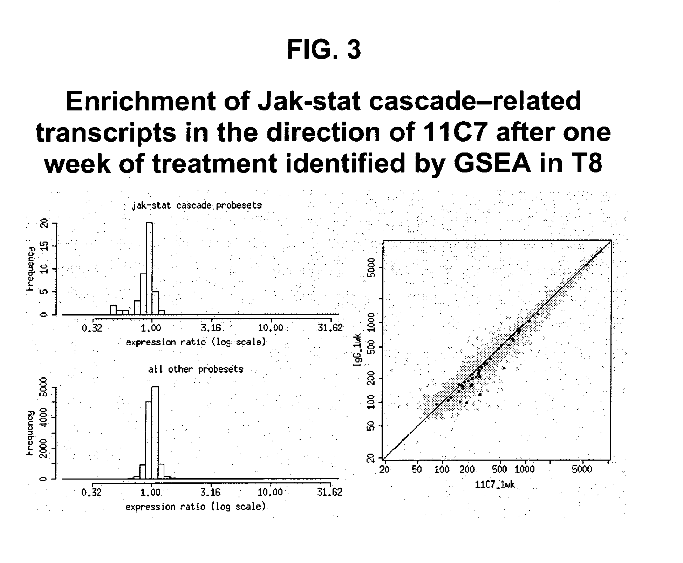

Gene Set Enrichment Analysis (GSEA) identified altogether 30 pathways with significant enrichment of differentially enriched transcripts after one week of treatment (TABLE 16 in EXAMPLE 3). Most significant enrichment was observed in immunity and defence -related transcripts (FIG. 1), cytokine and chemokine mediated signalling pathway (FIG. 2) and Jak-stat cascade (FIG. 3) all in the direction of 11C7. Of nervous system related pathways, neuronal activities, neurogenesis and nerve-nerve synaptic transmission were downregulated (q<0.001) and slit-robo-mediated axon guidance (q=0.018) upregulated in the 11C7-treated animals.

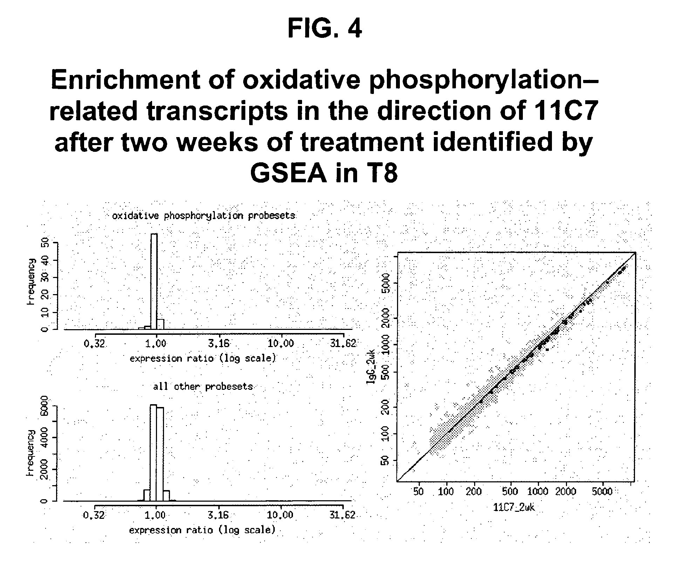

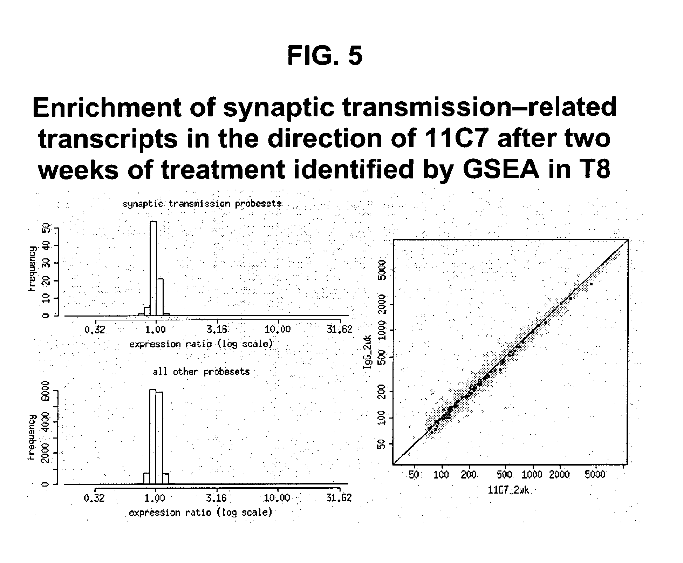

After two weeks of treatment, fold changes were significantly smaller than after 1 week of treatment. Only one transcript was > 1.5 fold significantly differentially regulated (p53-responsive gene 3, 1.6 fold upregulated after 11 C7). GSEA identified 19 pathways in which significant enrichment of differentially expressed transcripts were observed. Oxidative phosphorylation (FIG. 4), electron/ion/cation transport, blood coagulation, pre-mRNA processing and synaptic transmission (FIG. 5) were among the most significantly affected pathways (TABLE 21 in EXAMPLE 3).

*Spinal cord T1-7 (Proximal to the site of injury).* Welch T-test comparing the IgG-treated group to the 11C7-treated group resulted in 566 and 579 differentially expressed genes after one week and two weeks of treatment, respectively. The average fold change of the top 100 largest fold changes was 1.43±0.17 after one week of treatment and 1.56±0.98 after two weeks of treatment. The top 100 gene expression changes after one week of treatment are listed in TABLE 18 and after two weeks of treatment, in TABLE 19 in EXAMPLE 3.

The largest changes at one week after 11C7 treatment replicated the theme observed at the site of injury: eight of the top 20 changes were related to ECM (lumican, collagens 1a1-2 and 5a1, fibulin 2, tetranectin, Matrix glycoprotein SC1/ECM2) and downregulated after treatment with 11C7. After two weeks of treatment, fold changes were slightly larger than after 1 week of treatment. Some of the largest changes were related to downregulation of transcripts encoding for proteins expressed in lymphocytes

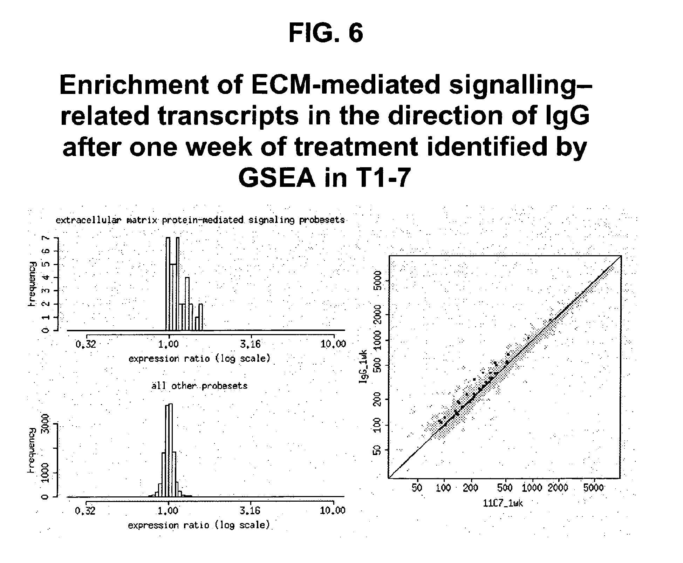

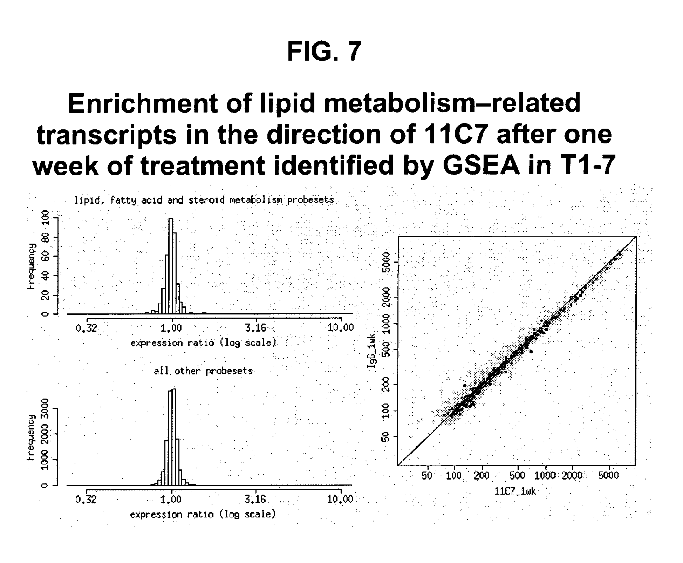

Gene Set Enrichment Analysis (GSEA) identified a significant enrichment in five pathways after one week of treatment (TABLE 18, EXAMPLE 3). No pathways were significantly affected (q<0.001) after two weeks of treatment. The most significantly affected pathways after one week were ECM-mediated signalling, lipid, fatty acid and sterol metabolism and growth factor homeostasis (FIGS. 6 to 8).

*Spinal cord L1-5 (Distal to the site of injury).* Welch T-test comparing the IgG-treated group to the 11C7-treated group resulted in 1303 and 1301 differentially expressed genes after one week and two weeks of treatment, respectively. The average fold change of the top 100 largest fold changes was 1.72±0.5 after one week of treatment and 1.91 ±2.0 after two weeks of treatment. The top 100 gene expression changes after one week of treatment are listed in Table 1-5 and after two weeks of treatment, in TABLE 21 in EXAMPLE 3.

The largest changes at one week after 11C7 treatment were related to transcripts expressed by lymphocytes (Similar to Ig gamma-2C chain C region (LOC362795), mRNA, secretory leukocyte protease inhibitor, lymphocyte selectin, lipocalin 2, thrombomodulin, chemokine (C-X-C motif) ligand 12) and as upregulated, could imply an increased lymphocyte trafficking into the tissue after 11C7 treatment. Also, Sfrp4 and ephrin B 1 were upregulated after 11C7.

After two weeks of treatment, top significantly changed transcripts included nuclear receptor MrgA10 RF-amide G protein-coupled receptor (Mrga10) and nuclear receptor coactivator 3 as well as immunity related transcripts which were downregulated after 11C7. A large number of significant changes were related to synaptic transmission or synaptic vesicle cycling (Synaptogenesis-related mRNA sequence 6, synaptic vesicle glycoprotein 2 b, synaptoporin) and were upregulated after 11C7.

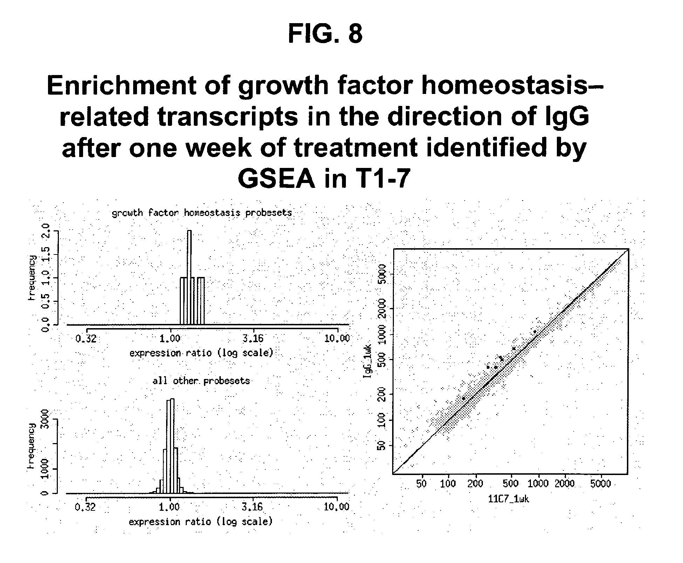

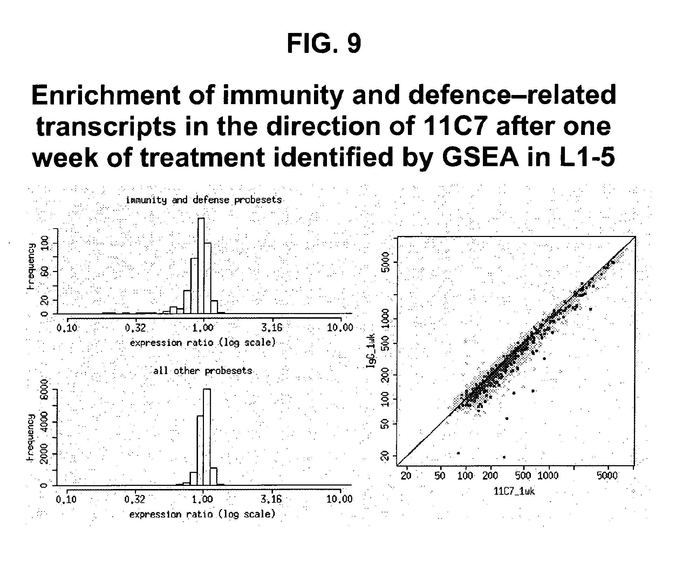

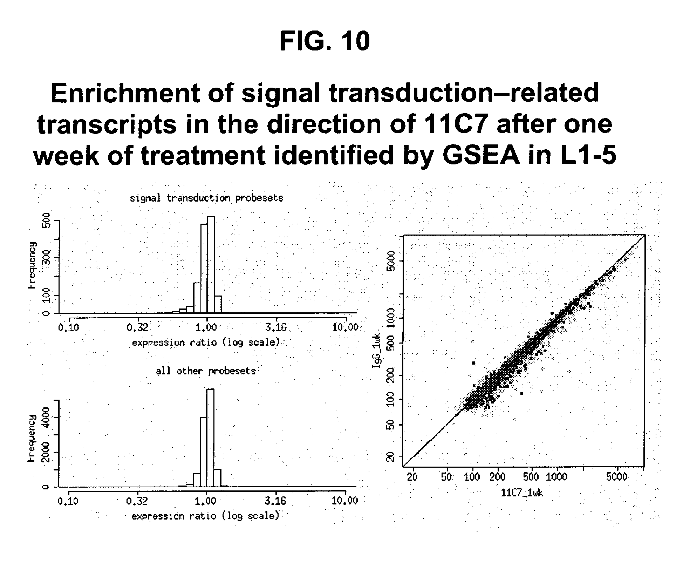

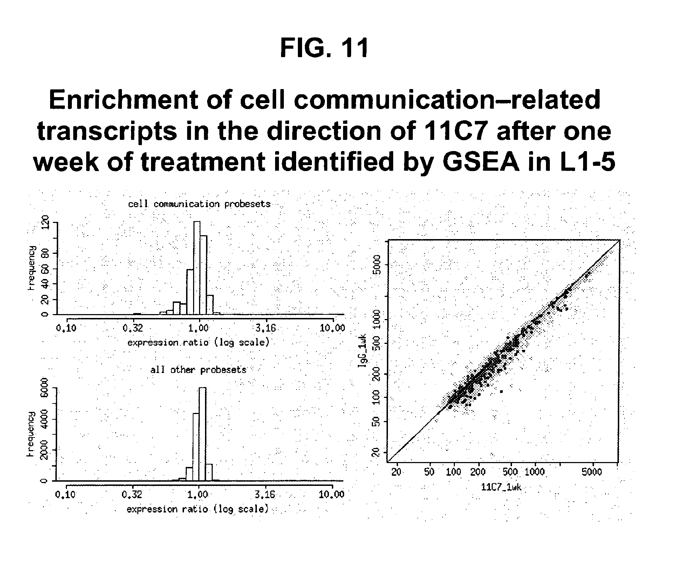

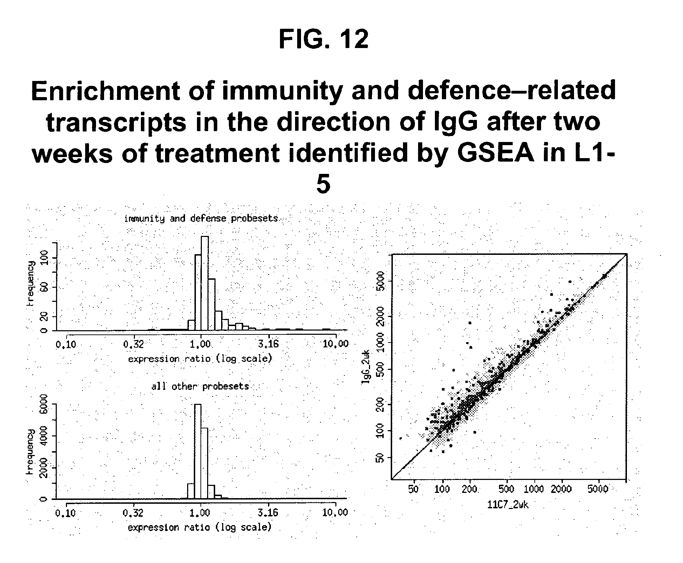

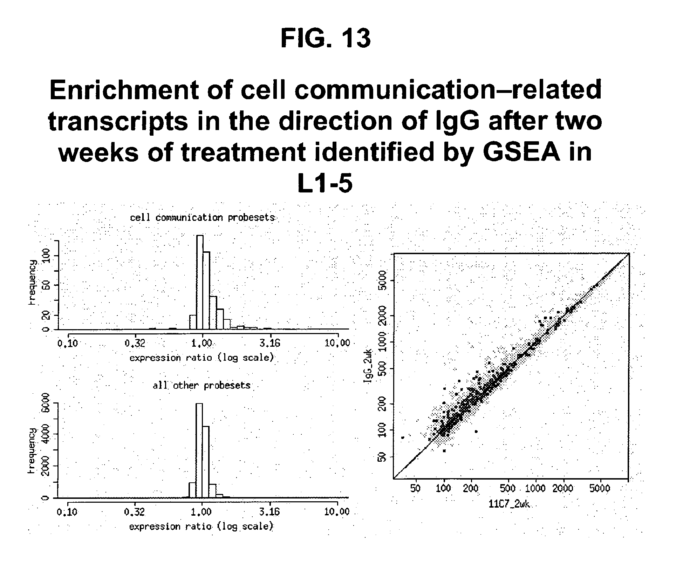

Gene Set Enrichment Analysis (GSEA) identified a significant enrichment in 58 pathways after one week of treatment (TABLE 19, EXAMPLE 3), and 48 pathways (TABLE 20, EXAMPLE 3) after two weeks of treatment. The most significantly affected pathways were immunity and defence, signal transduction and cell communication after one week of treatment (all upregulated in 11C7; FIGS. 8 to 10) and immunity and defence, cell communication and synaptic transmission after two weeks of treatment (FIGS. 11 to 13). Interestingly, immunity and defence —related pathway was highly significantly enriched in the direction of IgG—treated (downregulated after 11C7—treatment) after two weeks of treatment. Synaptic transmission, neuronal activities and neurotransmitter release-related pathways were significantly enriched (upregulated) after two weeks of 11C7-treatment.

*Motor-Somatosensory Cortex.* Welch T-test comparing the IgG-treated group to the 11C7-treated group resulted in 574 and 910 differentially expressed genes after one week and two weeks of treatment, respectively. The average fold change of the top 100 largest fold changes was $1.42\pm0.19$ after one week of treatment and $1.46\pm0.09$ after two weeks of treatment. The top 100 gene expression changes after one week of treatment are listed in TABLE 20 and after two weeks of treatment, in TABLE 21 in EXAMPLE 3.

70% of the top 100 changes in the motor/somatosensory cortex after one week treatment were ESTs thus complicating interpretation of the data. Among the top changed known transcripts were however S100 calcium-binding protein A9 (calgranulin B, expressed by macrophages, 3 fold upregulated after 11C7) and Crmp5 (Collapsin response mediator protein 5, upregulated after 11C7). Collapsin-response mediator proteins (CRMPs) are highly expressed in the developing brain where they take part in several aspects of neuronal differentiation. In adult, they are expressed in areas of persistent neurogenesis. Veyrac A et al., Eur. J Neurosci. 21:2635-2648 (2005). After two weeks of treatment, 80% of the top 100 changes were ESTs.

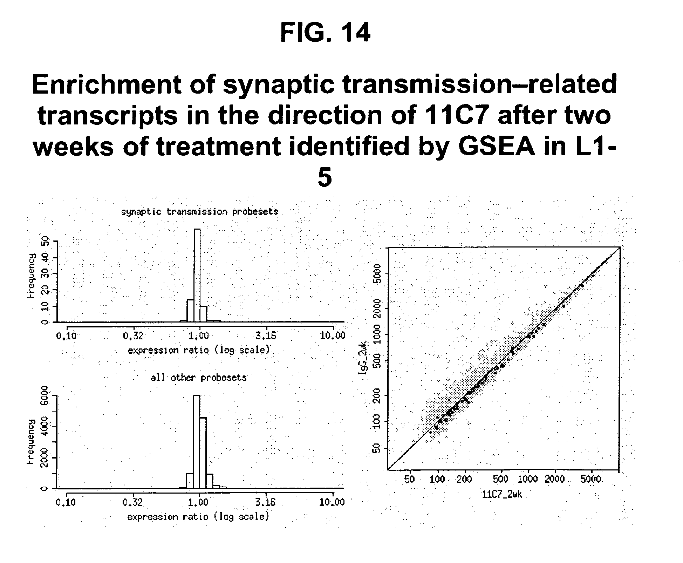

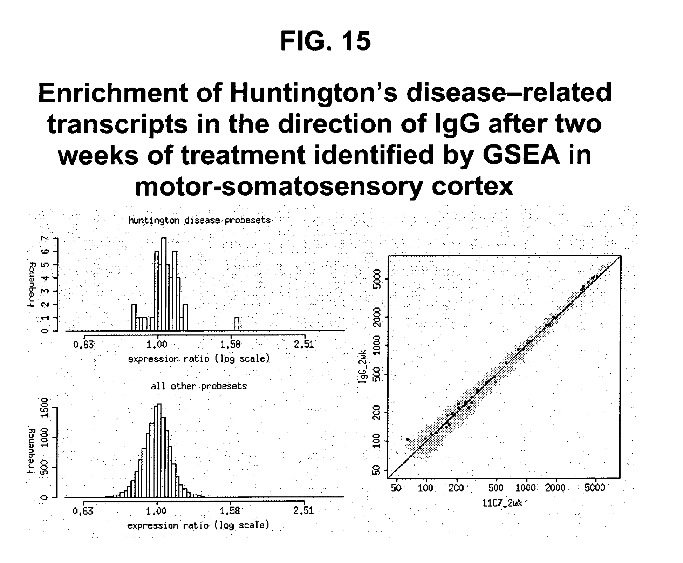

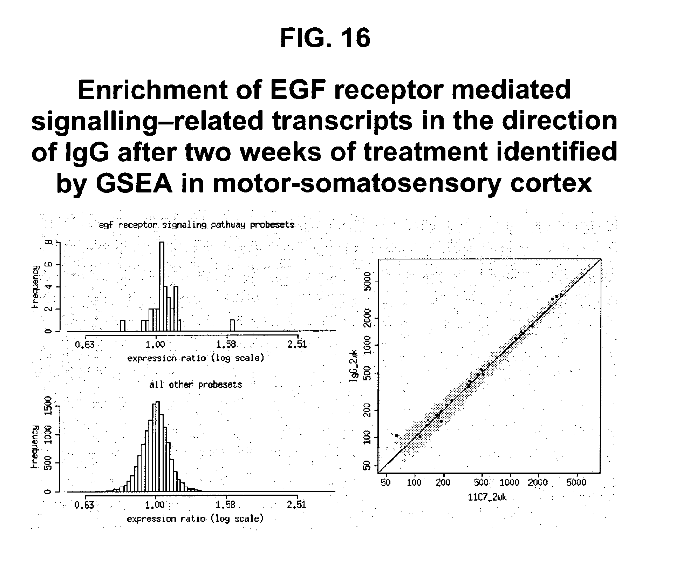

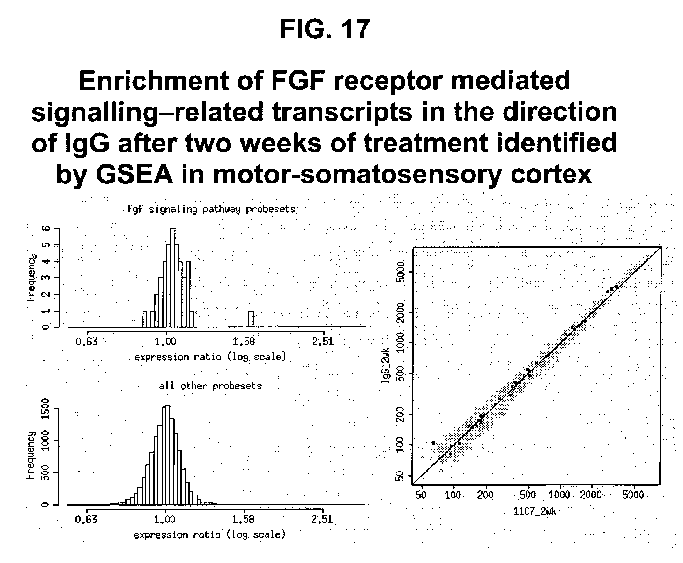

Based on multiple testing corrected analysis, GSEA identified no pathways with significant enrichment of differentially expressed transcripts after one week of treatment. After two weeks of treatment, the oxidative phosphorylation pathway showed a significant enrichment of differentially expressed genes (q<0.001; TABLE 21, EXAMPLE 3). Interestingly, the Huntington's disease, EGF-, FGF-, and NGF —signalling pathways were all affected but escaped the recommended level of significance (q<0.04 vs q<0.001). All were downregulated after 11C7 treatment (FIGS. 14 to 17). The small number of affected pathways is likely a reflection of the large number of ESTs differentially expressed in this dataset which cannot be assigned to any pathway.

*Frontal Cortex.* Welch t-test comparing the IgG-treated group to the 11C7-treated group resulted in 657 and 275 differentially expressed genes after one week and two weeks of treatment, respectively. The average fold change of the top 100 largest fold changes was $1.3\pm0.3$ after one week of treatment and $1.2\pm0.05$ after two weeks of treatment. The top 100 gene expression changes after one week of treatment are listed in Table 1-9 and after two weeks of treatment, in Table 1-10 in Annex-1. Only 13 transcripts after one week and 10 after two weeks of treatment was > 1.3 fold differentially expressed, thus indicating a very weak gene expression response to the treatment.

Among >1.3 fold changes were S100 calcium-binding protein A9 (calgranulin B) expressed by macrophages, c-fos oncogene, Dusp6 and Egr-1 related to cell differentiation after one week and stathmin 1, Nr2f2, G protein-coupled receptor 27 and myelin-associated oligodendrocytic basic protein (Mobp; 1.28 fold upregulated after 11C7) after two weeks of treatment.

GSEA was not performed for the frontal cortex dataset due to small number of significant changes.

*Blood.* Welch t-test comparing the IgG-treated group to the 11C7-treated group resulted in 389 and 427 differentially expressed genes after one week and two weeks of treatment, respectively. The average fold change of the top 100 largest fold changes was $2.1\pm0.56$ after one week of treatment and $1.80\pm0.40$ after two weeks of treatment. The top 100 gene expression changes after one week of treatment are listed in Table 1-11 and after two weeks of treatment, in Table 1-12 in Annex-1.

Among the largest changes at one week after 11C7 treatment were upregulation of matrix metalloproteinases Mmp8 and Mmp9, Hipk3, secretory leukocyte protease inhibitor (also upregulated after one week in L1-5) and calgranulin A. After two weeks of treatment, Similar to beta-amyloid binding protein (LOC362545), mRNA and Creb-binding protein were downregulated after 11C7 and neuroprotective mGluR8 and apoptosis-related Sfrp4 upregulated after 11C7.

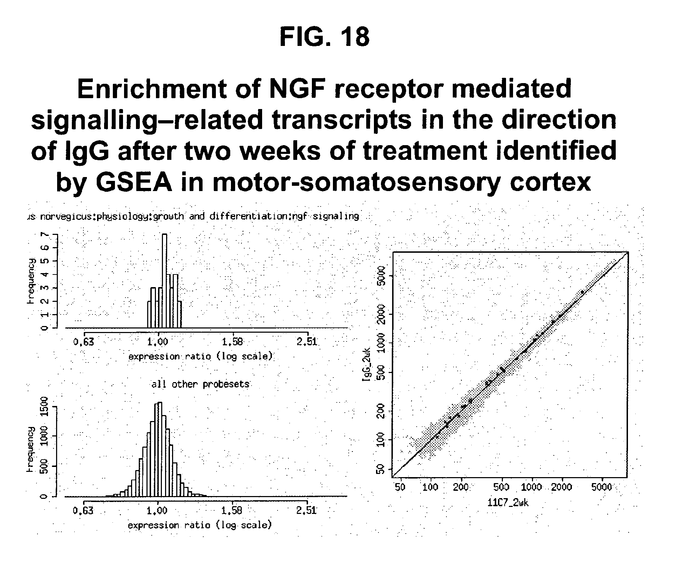

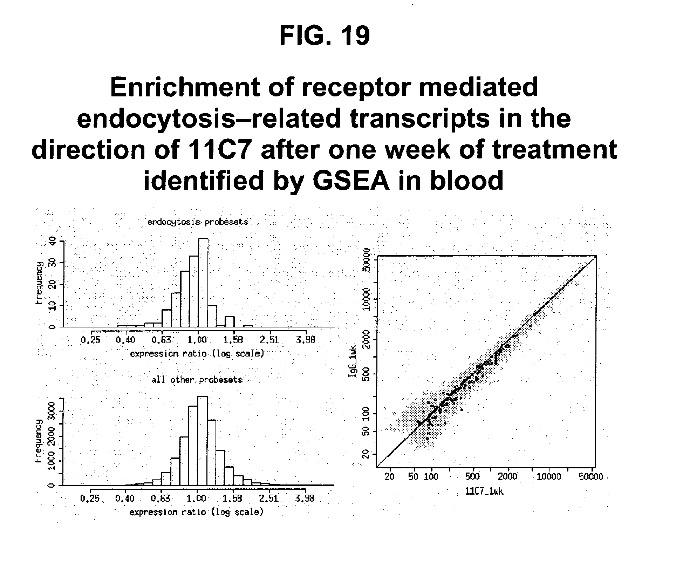

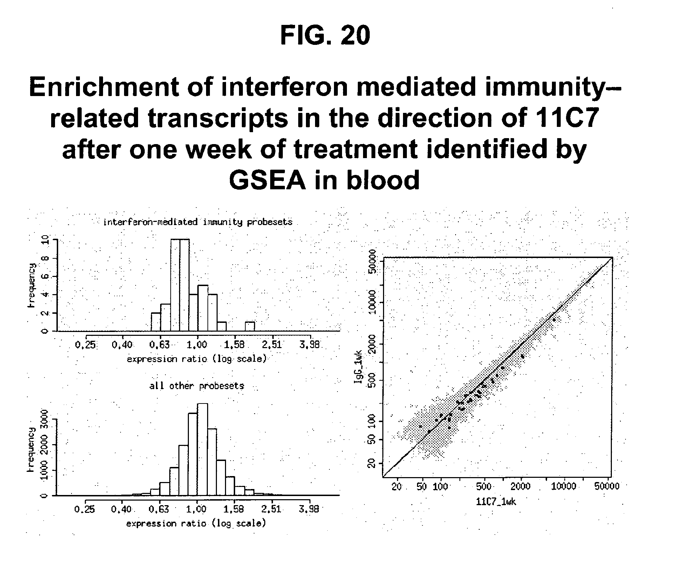

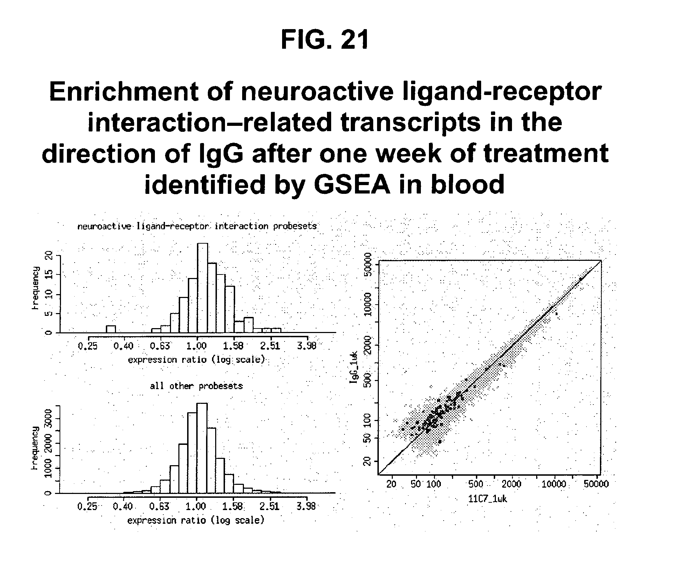

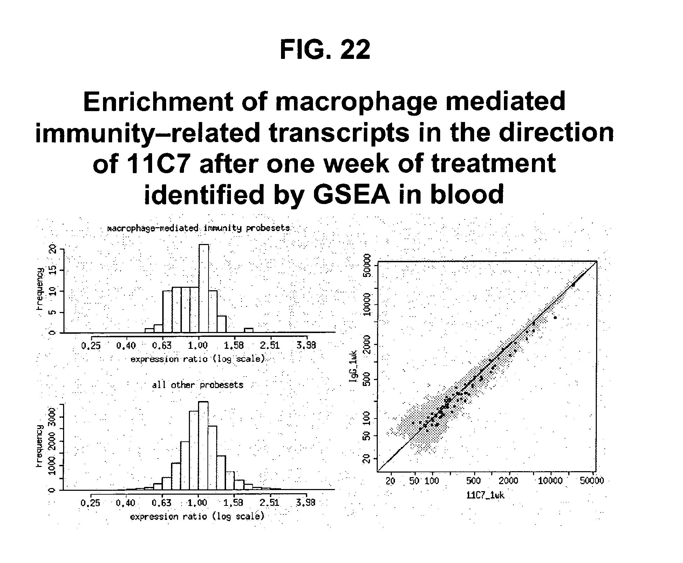

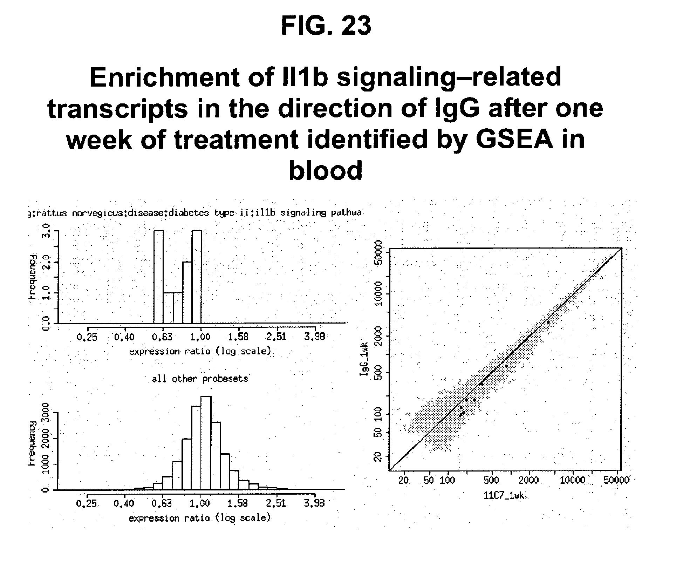

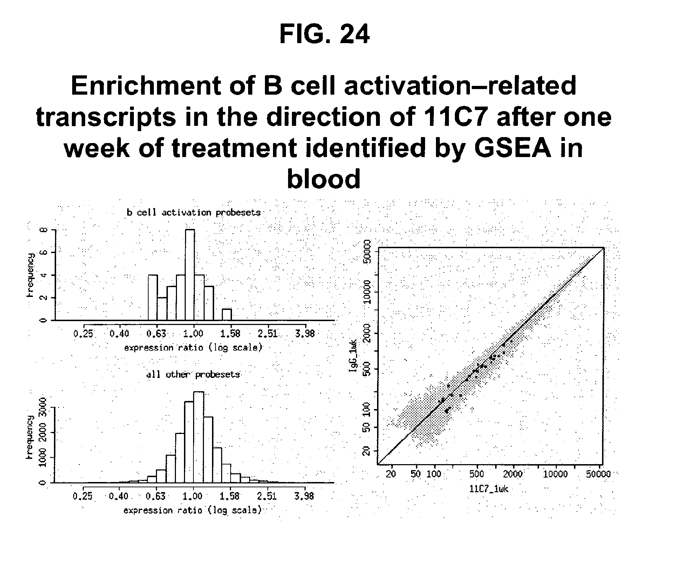

Based on multiple testing corrected analysis, GSEA identified six pathways with significant enrichment of differentially expressed transcripts after one week of treatment (q<0.001; Annex-2, Table 1-7). Endocytosis, intracellular protein traffic, receptor mediated endocytosis (FIG. 18), general vesicle transport, interferon mediated immunity (FIG. 19), neuroactive ligand-receptor interaction (FIG. 20), mapk signalling pathway, macrophage-mediated immunity (FIG. 21), followed by 11-1b and B-cell activation (FIGS. 22 and 23, respectively) were the most affected pathways. Interestingly, the enrichment direction in all of the above mentioned apart from the neuroactive ligand-receptor interaction, was in the direction of 11C7. This indicates upregulation of transcripts related to those pathways after 11C7 treatment. After two weeks of treatment, eight pathways showed a significant enrichment of differentially expressed genes (q<0.001; Annex-2, Table 1-8). Protein metabolism and modification, immunity and defence (FIG. 24) and protein modification were among the top affected pathways. All apart from one pathway after two weeks of treatment in blood were enriched in the direction of IgG.

*Discussion.* The purpose of this EXAMPLE was to identify treatment-related changes in rat after spinal cord hemisection after one week and two weeks of treatment with monoclonal mouse anti-Nogo-A antibody 11C7 in comparison to control treatment, mouse IgG antibody against plant lectin.

After one week of treatment, the most significant gene expression changes in terms of number and magnitude were observed distal to the site of injury (L1-5) followed by the site of injury (T8) and blood, whereas frontal cortex, motor-somatosensory cortex and spinal cord proximal to the site of injury (T1-7) were clearly less affected (TABLE 2). After two weeks of treatment, the largest effect size in terms of gene expression was observed at L1-5 followed by a relatively similar effect on motor-somatosensory cortex, spinal cord proximal to the site of injury (T1-7) and blood. Clearly less effect by the treatment was observed in T8 and in the frontal cortex after two weeks of treatment (TABLE 2).

TABLE 2

Summary of gene expression changes in tissues studied

| | 1 week | | | | 2 weeks | | |
|---|---|---|---|---|---|---|---|
| Tissue | Number of significant changes | Mean Fold Change of Top 100 | Effect size rank | Tissue | Number of significant changes | Mean Fold Change of Top 100 | Effect size rank |
| LI-5 | 1303 | 1.72±0.5 | 1 | L1-5 | 1301 | 1.91±2.0 | 1 |
| T8 | 643 | 1.93±1.06 | 2 | MCx | 910 | 1.46±0.09 | 2 |
| Blood | 389 | 2.1±0.56 | 3 | T1-7 | 579 | 1.56±0.98 | 2 |
| FCx | 657 | 1.3±0.3 | 4 | Blood | 427 | 1.8±0.04 | 3 |
| MCx | 574 | 1.42±0.19 | 5 | T8 | 449 | 1.31±0.07 | 4 |
| T1-7 | 566 | 1.43±0.17 | 5 | FCx | 275 | 1.2±0.05 | 5 |

Effect size rank is ranking the tissues studied based on the number of significant gene expression changes and the average fold change of the top 100 gene expression changes in that tissue.

A very strong effect by 11C7 was observed at the site of lesion downregulating transcripts related to extracellular matrix and wound healing after one week of treatment. Asporin precursor, dermatopontin, microfibril-associated glycoprotein-2 and several collagens were among the top downregulated changes as well as two secreted frizzled related proteins Sfrp2 and Sfrp4 whose expression has been found to correlate with apoptosis. Myocilin/TIGR, a secreted glycoprotein with upregulated expression in chronic glial scar after CNS injury and neurite outgrowth inhibiting effect on dorsal root ganglia neurons in vivo (Jurynec MJ et al., Mol. Cell. Neurosci. 23:69-80 (2003)) was found to be 2.67 fold downregulated after one week of 11C7-treatment. Myocilin is suggested to be a novel neurite outgrowth inhibiting molecule inhibited by anti-Nogo-A-treatment.

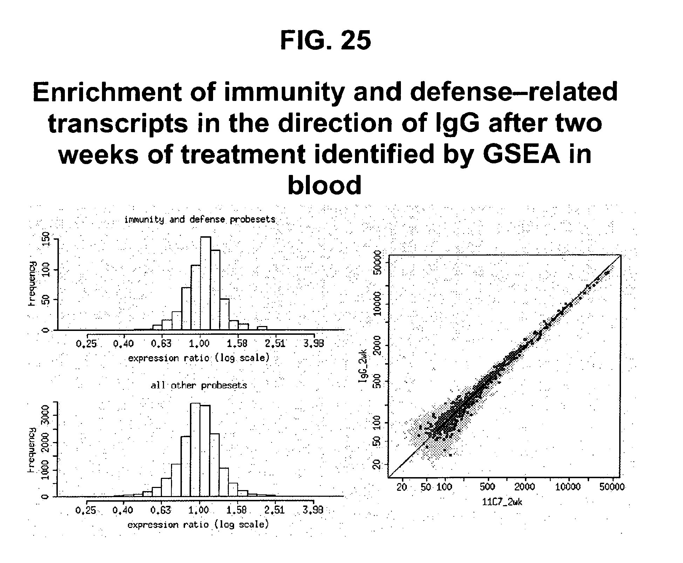

Other neurite outgrowth/axon guidance related changes included the slit-robo mediated axon guidance pathway related transcripts encoding for chemokine (C-X-C motif) ligand 12 and chemokine (C-X-C motif) receptor 4 identified by GSEA (q<0.02). Cxc112 and CXCR4 showed a concerted upregulation in all spinal cord segments studied after one week of treatment with 11C7 (FIG. 25). Activation of Cxcr4 by its soluble ligand Cxc112 (Sdfl) has been shown to influence growth cone motility and neurite extension *in vitro* (Arakawa Y et al., J. Cell. Biol. 161:381-391 (2003); Pujol F et al.,. J. Cell Sci. 118:1071-1080 (2005); Xiang Y et al., Nat. Neurosci. 5:843-848 (2002)). Interestingly, this action was suggested to be mediated by the Rho/ROCK pathway so that a low concentration of Cxc112 stimulated a Rho-dependent pathway that mediated facilitation of axon elongation. Arakawa Y et al., J. Cell. Biol. 161:381-391 (2003). Recently, Cxc112-CXCR4 chemokine signalling pathway was shown to define the initial trajectory of mammalian motor axons during the development. Lieberam I et al., Neuron 47:667-679 (2005). Our finding suggests, that this pathway could be upregulated as a result of 11C7 treatment and may thus contribute to the mechanism of action of anti-Nogo A during regeneration.

At the level of individual genes but not identified by GSEA, were changes related to semaphorin-collapsin mediated pathway: sema A/semaphorin 3A and collapsing response proteins 4 and 5 Crmp4/5 mediating repulsive cues to the migrating growth cones were seen downregulated after 1 week of treatment in T8 and in motor-somatosensory cortex.

GSEA was first described by Mootha VK et al., Nat. Genet. 34:267-273 (2003) as a method to identify coordinated transcriptional changes among functionally related groups of genes in microarray data. The gene set enrichment analysis method has been implemented in-house with several refinements to the original methodology [RD-2005-50762]. Often in the microarray data, changes at the level of single transcripts remain insignificant due to small fold changes while a large number of such changes affecting a whole pathway would be of significance. Due to small fold changes observed in nervous system in general (most likely due to a large gene dilution effect of heterogeneous cell populations), GSEA approach would be particularly interesting when interpreting data originating from nervous tissues. Pathway information introduced in the GSEA in this study has been collected from a variety of sources, including publicly available databases (KEGG) and proprietary (Celera, Pathart). Summary of the 24 pathways with significant (q<0.001) gene set enrichment in three or more tissues is presented in TABLE 3.

The most widely affected pathways overall were immunity and defence (4 tissues), protein metabolism and phosphorylation (4), nucleoside, nucleotide and nucleic acid metabolism (4) neuronal activities (4) and Jak-stat cascade (4).

GSEA revealed in this study a very clear effect in the immune defence pathways, including B- and T-cell mediated signalling, B-cell activation, macrophage-, NK-cell mediated as well as neutrophil mediated immunity, toll-like receptor pathway and cytokine and chemokine mediated signalling pathways. Interestingly, the immunity and defence mediated pathway was enriched in the direction of 11C7 after one week of treatment but in the direction of IgG after two weeks of treatment. Same pattern was observed also in all other immune mechanism —related pathways, such as B-cell, T-cell, macrophage and NK-cell mediated immunity pathways. Significant effect on the immunity-related pathways was observed most commonly in the spinal cord at the site of lesion (T8) and distal to it (L1-5) and in the blood, where the enrichment direction paralleled that of the spinal cord tissues. Although not studied in detail microscopically, this suggests an increase in the lymphocytes, macrophages and NK-cells after one week of treatment with 11C7 both in blood and in the injured spinal cord in comparison to the IgG -treated animals and possibly an increased trafficking of lymphocytes into the injured spinal cord. As antibodies targeting the extracellular portion of Nogo-A (Nogo-66) has been suggested to be of therapeutic potential in an animal model of multiple sclerosis (Karnezis T et al., Nat. Neurosci. 7: 736-744 (2004); Fontoura P et al., J Immunol. 173:6981-6992 (2004)), the possible involvement of immune related mechanisms in the compound action are of special interest.

Other significantly enriched pathways affected in more than three tissues studied include apoptosis and apoptosis signalling pathway, blood clotting/coagulation, cell adhesion-mediated signalling,, extracellular matrix protein-mediated signalling, growth factor homeostasis, , oncogene, oxidative phosphorylation and synaptic transmission. The enrichment direction in most of the pathways was similar to that observed in the immune related pathways, towards 11C7 after one week of treatment but in the direction of IgG after two weeks of treatment. An interesting exception is the synaptic transmission pathway, where after one week of treatment the pathway is downregulated after 11C7 treatment but upregulated after two weeks of treatment. Neuronal activities- and nerve-nerve-synaptic transmission pathways followed the same pattern and were significantly affected in spinal cord at the level of T8 and L1-5.

Identification of the several growth factor pathways, including EGF, FGF, NGF, PDGF and TGF beta-signalling pathways in the action of anti-Nogo-A antibody is of interest from several points: The EGF-receptor activation was recently reported to be the mediator of the inhibitory signals from myelin and chondroitin sulphate in axon regeneration and inhibition of the EGF receptor signalling resulted in regeneration resulted in regeneration of optic nerve after injury. He Z & Koprivica V, Annu. Rev. Neurosci. 27:341-368 (2004); Koprivica V et al., Science 310:106-110 (2005). In current dataset, EGF-receptor mediated signalling pathway was upregulated in blood and L 1-5 after 1 week of treatment with 11C7 but interestingly downregulated in motor-somatosensory cortex after 2 weeks of 11C7 treatment. PDGF signalling pathway was concomitantly upregulated after one week of treatment by 11C7 in spinal cord at all three levels studied (T8, T1-7, L1-5).

TABLE 3

Pathways with significant gene set enrichment in three or more tissues

| Pathway Name | Pathway Source | Tissue | Enrichment Direction |
|---|---|---|---|
| Apoptosis | Celera | T8 1 wk | 11C7 |
| | KEGG | T8 1 wk | 11C7 |
| | Celera | L1-51 wk | 11C7 |
| | Celera | L1-52 wk | IgG |
| apoptosis signalling | Celera public | T8 1 wk | 11C7 |
| pathway | Celera public | L1-51wk | 11C7 |
| | Celera public | L1-5 2 wk | IgG |
| B-cell- and antibody- | Celera | T8 1 wk | 11C7 |

(continued)

Pathways with significant gene set enrichment in three or more tissues

| Pathway Name | Pathway Source | Tissue | Enrichment Direction |
|---|---|---|---|
| mediated immunity | Celera | L1-51wk | 11C7 |
| | Celera | L1-5 2 wk | IgG |
| blood clotting | Celera | L1-51wk | 11C7 |
| | Celera | L1-5 2 wk | IgG |
| | Celera public | T8 2 wk | IgG |
| complement and | KEGG | T8 2 wk | IgG |
| coagulation cascades | KEGG | L1-51wk | 11C7 |
| | KEGG | L1-5 2 wk | IgG |
| cytokine and chemokine | Celera | T8 1 wk | 11C7 |
| mediated signalling | Celera | L 1-5 1 wk | 11C7 |
| pathway | Celera | L1-5 2 wk | IgG |
| | Celera | T8 1 wk | 11C7 |
| extracellular matrix | Celera | T1-71 1 wk | IgG |
| protein-mediated | Celera | L1-51wk | 11C7 |
| signalling | Celera | L1-5 2 wk | IgG |
| Growth factor | Celera | T1-7 1 wk | IgG |
| homeostasis | Celera | L1-51wk | 11C7 |
| | Celera | L1-5 2 wk | IgG |
| immunity and defence | Celera | Blood 2wk | IgG |
| | Celera | T8 1 wk | 11C7 |
| | Celera | L1-51wk | 11C7 |
| | Celera | L1-5 2 wk | IgG |
| interferon-mediated | Celera | Blood 1 wk | 11C7 |
| immunity | Celera | T8 1 wk | 11C7 |
| | Celera | L1-51wk | 11C7 |
| intracellular protein | Celera | Blood 2wk | IgG |
| traffic | Celera | Blood 1 wk | 11C7 |
| | Celera | T8 1 wk | 11C7 |
| Jak-stat cascade | Celera | T8 1 wk | 11C7 |
| | Celera | L1-51wk | 11C7 |
| | Celera | L1-5 2 wk | IgG |
| | Celera public | T8 1 wk | 11C7 |
| | Celera public | L1-51wk | 11C7 |
| macrophage-mediated | Celera | T8 1 wk | 11C7 |
| immunity | Celera | L 1-5 1 wk | 11C7 |
| | Celera | L1-5 2 wk | IgG |
| nerve-nerve synaptic | Celera | T8 2 wk | 11C7 |
| transmission | Celera | L 1-5 1 wk | IgG |
| | Celera | L1-52wk | 11C7 |
| neuronal activities | Celera | T8 1 wk | IgG |
| | Celera | T8 2 wk | 11C7 |
| | Celera | L 1-5 1 wk | IgG |
| | Celera | L1-5 2 wk | 11C7 |
| nucleoside, nucleotide | Celera | Blood 2wk | IgG |
| and nucleic acid | Celera | T8 1 wk | 11C7 |
| metabolism | Celera | T8 2 wk | IgG |
| | Celera | L1-51 wk | 11C7 |
| | Celera | L1-5 2 wk | IgG |
| oncogenesis | Celera | Blood 2wk | IgG |

(continued)

Pathways with significant gene set enrichment in three or more tissues

| Pathway Name | Pathway Source | Tissue | Enrichment Direction |
|---|---|---|---|
| | Celera | L 1-5 1 wk | 11C7 |
| | Celera | L1-5 2 wk | IgG |
| oxidative | KEGG | T8 2 wk | 11C7 |
| phosphorylation | Celera | T8 2 wk | 11C7 |
| | KEGG | L1-5 2 wk | 11C7 |
| | KEGG | MCx 1 wk | IgG |
| Protein metabolism and | Celera | Blood 2wk | IgG |
| modification | Celera | T8 1 wk | 11C7 |
| | Celera | L1-51 wk | 11C7 |
| | Celera | L1-5 2 wk | IgG |
| Protein modification | Celera | Blood 2wk | IgG |
| | Celera | T8 1 wk | 11C7 |
| | Celera | L 1-5 1 wk | 11C7 |
| | Celera | L1-5 2 wk | IgG |
| Proteolysis | Celera | T8 1 wk | 11C7 |
| | Celera | L 1-5 1 wk | 11C7 |
| | Celera | L1-5 2 wk | IgG |
| synaptic transmission | Celera | T8 2 wk | 11C7 |
| | Celera | L 1-5 1 wk | IgG |
| | Celera | L1-5 2 wk | 11C7 |
| T-cell mediated | Celera | T8 1 wk | 11C7 |
| immunity | Celera | L 1-5 1 wk | 11C7 |
| | Celera | L1-5 2 wk | IgG |
| toll receptor signalling | Celera public | L 1-5 1 wk | 11C7 |
| pathway | Celera public | L1-5 2 wk | IgG |
| | KEGG | T8 1 wk | 11C7 |
| | KEGG | L 1-5 1 wk | 11C7 |

*Conclusion.* The results confirm at the level of gene expression the injured spinal cord and motor cortex as the primary sites of action of the anti-Nogo-A antibody treatment applied intrathecally. The analysis identified novel molecular and pathways candidates as possible targets of anti-Nogo-A treatment, such as myocilin and the slit-robo pathway. The results also pointed to strong involvement of immune defence related pathways in the treatment effect.

The secreted proteins Sfrp4, Mmp9 and myocilin were selected to be further studied as candidate markers of treatment effect.

TAQMAN confirmation of selected findings was performed. All selected transcripts were confirmed (Sfrp2, Sfrp4, myocilin, asporin precursor, dermatopontin, Mmp9).

EXAMPLE 2

GENOMICS EXPLORATORY STUDY IN A RAT SPINAL CORD INJURY MODEL AFTER TREATMENT WITH ANTI-NOGO A ANTIBODY 11C7; MICROARRAY GENE EXPRESSION ANALYSIS, CONTINUED

*Gene Set Enrichment Analysis (GSEA).* Gene set enrichment analysis (GSEA) was performed as described by Mootha VK et al., Nat. Genet. 34:267-273 (2003). Shortly, GSEA determines if the members of a given gene set are enriched among the most differentially expressed genes between two classes. First, the genes are ordered on the basis of a difference metric. It can be the difference in means of the two classes divided by the sum of the standard deviations of the two diagnostic classes but other difference metrics can also be used.

For each gene set, an enrichment measure called the ES is made. This is a normalized Kolmogorov-Smirnov statistic. Consider the genes $R_1, .., R_N$ that are ordered on the basis of the difference metric between the two classes and a gene set S containing G members. We define

$$X_j = -\sqrt{\frac{G}{N-G}}$$

if Ri is not a member of S, or

$$X_j = \sqrt{\frac{N-G}{G}}$$

if Ri is a member of S. A running sum across all N genes is then computed. The ES is defined as

$$\max_{1 \le j \le N} \sum_{i=1}^{j} X_j$$

or the maximum observed positive deviation of the running sum. ES is measured for every gene set considered. Gene sets are based on pathway information from Celera, Pathart and KEGG. To determine whether any of the given gene sets shows association with the class phenotype distinction, the class labels are permuted 1,000 times, each time recording the maximum ES over all gene sets. In this regard, a single hypothesis is being tested. The null hypothesis is that no gene set is associated with the class distinction.

*Results.* Initial data-analysis of the dataset for spinal cord tissues T8 (at the level of injury) and proximal to the injury, T1-7 was performed blindly. Analysis resulted in identifying samples coded "orange" as the 11C7-treated group after which the code was broken and the sample identity confirmed. Remaining of the analysis was not blinded.

*Spinal cord T8 (At the level of injury).* Welch T-test comparing the IgG-treated group to the 11C7-treated group resulted in 643 and 449 differentially expressed genes after one week and two weeks of treatment, respectively. The average fold change of the top 100 largest fold changes was $1.93 \pm 1.06$ after one week of treatment and $1.31 \pm 0.07$ after two weeks of treatment. The top 20 gene expression changes after one week of treatment are listed in TABLE 4 and after two weeks of treatment, in TABLE 5. 90% of the top 20 transcripts were downregulated at one week after 11C7 treatment (whereas of the total differentially expressed ones, 41 % were downregulated). Interestingly, among them there were 7 transcripts encoding for proteins related to extracellular matrix (ECM) and wound healing and/or scarring (asporin precursor, dermatopontin, collagen), 2 secreted frizzled-like proteins (Sfr12 and 4), two Igf-binding proteins (Igfbp 5 and 6, negative regulators of Igf and myocilin/TIGR, which has been recently shown to inhibit neurite outgrowth and to be upregulated in chronic glial scar after CNS injury. Jurynec MJ et al., Mol. Cell. Neurosci. 23:69-80 (2003).

Gene Set Enrichment Analysis (GSEA) identified a significant enrichment in immunity and defence -related transcripts, cytokine and chemokine mediated signalling pathway, Jak-stat cascade, inhibition of apoptosis and in 90 other pathways after one week of treatment with 11C7 (TABLE 4). Of nervous system related pathways, neuronal activities, neurogenesis and nerve-nerve synaptic transmission were downregulated and slit-robo-mediated axon guidance upregulated in the 11 C7-treated animals.

TABLE 4

Top 20 gene expression changes in spinal cord at the level of injury (T8) after one week of treatment with the monoclonal mouse anti-Nogo A antibody 11C7

| Probe set name | p-value (ANOVA) | Fold change in anti-Nogo-A-treated vs. IgG treated | Gene Title | Common name |
|---|---|---|---|---|
| 1381504_at | 0.003869 | 0.1 | Similar to asporin precursor (LOC306805), mRNA | |
| 1380726_at | 0.001893 | 0.1 | Similar to asporin precursor (LOC306805), mRNA | |
| 1373674_at | 6.91 E-04 | 0.3 | Similar to microfibril-associated glycoprotein-2 (LOC362429), mRNA | |

(continued)

Top 20 gene expression changes in spinal cord at the level of injury (T8) after one week of treatment with the monoclonal mouse anti-Nogo A antibody 11C7

| Probe set name | p-value (ANOVA) | Fold change in anti-Nogo-A-treated vs. IgG treated | Gene Title | Common name |
|---|---|---|---|---|
| 1391946_at | 0.046399 | 2.9 | selectin, platelet | Selp |
| 1371732_at | 0.005726 | 0.3 | dermatopontin | Dpt |
| 1368394_at | 0.040183 | 0.3 | secreted frizzled-related protein 4 | Sfrp4 |
| 1392832_at | 0.002301 | 0.4 | Transcribed sequence with strong similarity to protein ref:NP_004664.1 (H. sapiens) angiopoietin-like 1 precursor; angiopoietin Y1; angiopoietin 3 [Homo sapiens] | |
| 1387313_at | 0.009335 | 0.4 | myocilin | Myoc, TIGR |
| 1373947_at | 0.005543 | 0.4 | dermatopontin | Dpt |
| 1372615_at | 0.013582 | 0.4 | amine oxidase, copper containing 3 | Aoc3 |
| 1387625_at | 0.001661 | 0.4 | insulin-like growth factor binding protein 6 | |
| 1390119_at | 0.037451 | 0.4 | secreted frizzled-related protein 2 | Sfrp2 |
| 1376105_at | 0.002882 | 0.4 | Similar to collagen type XIV (LOC314981 MRNA | |
| 1374070_at | 0.045385 | 2.4 | glutathione peroxidase 2 | |
| 1392965_a_at | 0.021555 | 0.4 | Transcribed sequence with weak similarity to protein ref:NP_071420.1 (H.sapiens) secreted modular calcium-binding protein 1 [Homo sapiens] | |
| 1397830_at | 0.035479 | 0.5 | insulin-like growth factor-binding protein 5 | lgfbp5 |
| 1383708_at | 0.005141 | 0.5 | Transcribed sequence with strong similarity to protein ref:NP_004782.1 (H.sapiens) integrin, beta-like | |
| 1372168_s_at | 0.001704 | 0.5 | insulin-like growth factor binding protein 6 | |
| 1374616_at | 5.66E-04 like | 0.5 | Similar to platelet-derived growth factor receptor-(LOC290771), mRNA | |
| 1374942_at | 0.023924 | 0.5 | Similar to carboxypeptidase X 2 (M14 family); carboxypeptidase X2; metallocarboxypeptidase 2 (LOC293566), mRNA | |

TABLE 5

GSEA performed on T8 dataset. Pathways with enriched genes either in IgG- or 11C7-treated after one week of treatment (q<0.05)

| Pathway Name | Source | Probe sets | q value | Enrichment Direction |
|---|---|---|---|---|
| expressed probesets that are unassigned to a pathway | gsea | 7048 | NA | NA |
| immunity and defence | Celera | 446 | 1.94E-21 | 11C7 |
| cytokine and chemokine mediated signalling pathway | Celera | 69 | 2.47E-12 | 11 C7 |

(continued)

GSEA performed on T8 dataset. Pathways with enriched genes either in IgG- or 11C7-treated after one week of treatment (q<0.05)

| Pathway Name | Source | Probe sets | q value | Enrichment Direction |
|---|---|---|---|---|
| Jak-stat cascade | Celera | 42 | 8.52E-10 | 11 C7 |
| protein metabolism and modification | Celera | 1420 | 1.56E-09 | 11C7 |
| interferon-mediated immunity | Celera | 32 | 1.17E-08 | 11 C7 |
| macrophage-mediated immunity | Celera | 58 | 1.77E-08 | 11 C7 |
| inhibition of apoptosis | Celera | 61 | 1.48E-07 | 11C7 |
| nucleoside, nucleotide and nucleic acid metabolism | Celera | 1325 | 4.38E-07 | 1C7 |
| NF-kappaB cascade | Celera | 33 | 5.42E-06 | 11 C7 |
| B-cell-and antibody-mediated immunity | Celera | 35 | 1.97E-05 | 11C7 |
| granulocyte-mediated immunity | Celera | 21 | 4.45E-05 | 11C7 |
| intracellular protein traffic | Celera | 623 | 4.45E-05 | 11 C7 |
| toll-like receptor signalling pathway | KEGG | 29 | 4.45E-05 | 11C7 |
| natural killer cell mediated immunity | Celera | 13 | 5.94E-05 | 11C7 |
| Apoptosis | Celera | 247 | 8.75E-05 | 11C7 |
| Proteolysis | Celera | 400 | 0.00032 | 11 C7 |
| ectoderm development | Celera | 153 | 0.00032 | IgG |
| cell motility | Celera | 99 | 0.00037 | 11C7 |
| Cytokine/chemokine mediated immunity | Celera | 31 | 0.000419 | 11C7 |
| apoptosis signalling pathway | Celera public | 51 | 0.000419 | 11C7 |
| DNA metabolism | Celera | 128 | 0.000419 | 11 C7 |
| Jak-stat signalling pathway | Celera public | 8 | 0.000455 | 11 C7 |
| protein modification | Celera | 588 | 0.000491 | 11C7 |
| Apoptosis | KEGG | 39 | 0.000501 | 11C7 |
| protein glycosylation | Celera | 88 | 0.000503 | 11 C7 |
| Endocytosis | Celera | 164 | 0.000894 | 11 C7 |
| T-cell mediated immunity | Celera | 58 | 0.00093 | 11C7 |
| cell cycle | Celera | 392 | 0.001 | 11C7 |
| neuronal activities | Celera | 227 | 0.001 | IgG |
| Neurogenesis | Celera | 143 | 0.0011 | IgG |
| Haematopoiesis | Celera | 53 | 0.00119 | 11 C7 |
| toll receptor signalling pathway | Celera public | 15 | 0.00174 | 11C7 |
| DNA replication | Celera | 47 | 0.0021 | 11 C7 |
| carbohydrate metabolism | Celera | 228 | 0.0021 | 11C7 |

(continued)

GSEA performed on T8 dataset. Pathways with enriched genes either in IgG- or 11C7-treated after one week of treatment (q<0.05)

| Pathway Name | Source | Probe sets | q value | Enrichment Direction |
|---|---|---|---|---|
| mapk signalling pathway | KEGG | 101 | 0.00232 | 11C7 |
| Huntington's disease | KEGG | 26 | 0.00356 | 11 C7 |
| Proteasome | KEGG | 19 | 0.0061 | 11C7 |
| MAPKKK cascade | Celera | 114 | 0.0061 | 11C7 |
| other immune and defence | Celera | 32 | 0.00647 | 11C7 |

| Pathway Name | Source | Probe sets | qvalue | Enrichment Direction |
|---|---|---|---|---|
| cell adhesion-mediated signalling | Celera | 128 | 0.00703 | IgG |
| signalling:Rattus norvegicus:disease: atherosclerosis:aif mediated pathway | Pathart | 5 | 0.00806 | 11C7 |
| Exocytosis | Celera | 131 | 0.00806 | 11C7 |
| receptor mediated endocytosis | Celera | 68 | 0.00806 | 11 C7 |
| pre-mRNA processing | Celera | 169 | 0.00927 | 11 C7 |
| cell structure | Celera | 267 | 0.0097 | IgG |
| signalling:Rattus norvegicus:disease: atherosclerosis:ifngamm a signalling pathway | Pathart | 4 | 0.0132 | 11 C7 |
| Glycolysis | Celera | 34 | 0.0137 | 11 C7 |
| signalling:Rattus norvegicus:disease: atherosclerosis:angioten sin signalling pathway | Pathart | 28 | 0.0137 | 11C7 |
| signalling:Rattus norvegicus:physiology: growth and differentiation:FGF2 signalling pathway | Pathart | 12 | 0.0137 | 11 C7 |
| signalling:Rattus norvegicus:physiology:cell adhesion:integrin signalling pathway | Pathart | 19 | 0.0137 | IgG |
| cell cycle control | Celera | 185 | 0.0146 | 11C7 |
| protein disulfide-isomerase reaction | Celera | 5 | 0.0155 | 11C7 |
| pi3 kinase pathway | Celera public | 24 | 0.0157 | 11 C7 |
| signalling:Rattus norvegicus:physiology: apoptosis:tnf signalling pathway | Pathart | 8 | 0.0157 | 11C7 |
| signalling:Rattus norvegicus:disease: rheumatoid | Pathart | 3 | 0.0164 | 11 C7 |

(continued)

| Pathway Name | Source | Probe sets | qvalue | Enrichment Direction |
|---|---|---|---|---|
| arthritis:interleukin signalling pathway | | | | |
| metabolism of cyclic nucleotides | Celera | 23 | 0.0164 | IgG |
| non-vertebrate process | Celera | 12 | 0.0164 | IgG |
| PDGF signalling pathway | Celera public | 19 | 0.0165 | 11 C7 |
| dentatorubropallidoluysian atrophy (drpla) | KEGG | 12 | 0.0177 | 11 C7 |
| starch and sucrose metabolism | KEGG | 25 | 0.0179 | 11C7 |
| axon guidance mediated by slit-robo | Celera public | 3 | 0.0183 | 11C7 |
| growth factor homeostasis | Celera | 8 | 0.0187 | IgG |
| other nucleoside, nucleotide and nucleic acid metabolism | Celera | 18 | 0.0204 | 11 C7 |
| signalling:Rattus norvegicus:disease: atherosclerosis:nfkb signalling pathway | Pathart | 3 | 0.0216 | 11 C7 |
| signalling:Rattus norvegicus:disease: atherosclerosis:ldl signalling pathway | Pathart | 17 | 0.0216 | 11 C7 |
| glycolysis / gluconeogenesis | KEGG | 29 | 0.0223 | 11C7 |
| nerve-nerve synaptic transmission | Celera | 24 | 0.0223 | IgG |
| glycosphingolipid metabolism | KEGG | 9 | 0.0223 | 11C7 |
| signalling:Raffus norvegicus:physiology: others:fcer1 signalling pathway | Pathart | 13 | 0.0236 | 11 C7 |
| intracellular signalling cascade | Celera | 438 | 0.0252 | 11 C7 |
| signalling:Rattus norvegicus:disease: atherosclerosisahrombo modulin signalling pathway | Pathart | 5 | 0.0262 | IgG |
| inflammation mediated by chemokine and cytokine signalling pathway | Celera public | 48 | 0.0281 | 11C7 |
| signalling:Rattus norvegicus:physiology: apoptosis:TGF beta induced apoptosis | Pathart | 23 | 0.0291 | 11 C7 |

(continued)

| Pathway Name | Source | Probe sets | qvalue | Enrichment Direction |
|---|---|---|---|---|
| Anterior/posterior patterning | Celera | 5 | 0.0293 | IgG |
| other polysaccharide metabolism | Celera | 56 | 0.0302 | 11C7 |
| Synaptic transmission | Celera | 81 | 0.0308 | IgG |
| n-glycan biosynthesis | KEGG | 8 | 0.0317 | 11C7 |
| signalling:Rattus norvegicus:disease: multiple sclerosis:responsive genes | Pathart | 3 | 0.032 | 11C7 |
| p53 pathway | Celera public | 12 | 0.032 | 11C7 |
| signalling:Rattus norvegicus:physiology: apoptosis:trail mediated apoptosis | Pathart | 5 | 0.034 | 11C7 |
| DNA recombination | Celera | 13 | 0.0378 | 11 C7 |
| regulated exocytosis | Celera | 50 | 0.0378 | 11C7 |
| blood circulation and gas exchange | Celera | 16 | 0.0378 | IgG |
| Histidine metabolism | KEGG | 10 | 0.0395 | IgG |
| complement-mediated immunity | Celera | 16 | 0.0401 | 11C7 |
| general vesicle transport | Celera | 180 | 0.0403 | 11 C7 |
| monosaccharide metabolism | Celera | 31 | 0.0428 | 11 C7 |
| Gamma-hexachlorocyclohexane degradation | KEGG | 5 | 0.0436 | 11 C7 |
| cholesterol biosynthesis | Celera public | 11 | 0.047 | 11 C7 |
| biosynthesis of steroids | KEGG | 14 | 0.0471 | 11C7 |
| signalling:Rattus norvegicus:disease: alzheimers:igf1 signalling pathway | Pathart | 4 | 0.049 | 11C7 |
| signalling:Rattus norvegicus:disease: atherosclerosis:il1 beta signalling pathway | Pathart | 2 | 0.0493 | 11C7 |
| b cell activation | Celera public | 26 | 0.0497 | 11C7 |

After two weeks of treatment, fold changes were significantly smaller than after 1 week of treatment. Only one transcript was>1.5 fold significantly differentially regulated (p53-responsive gene 3, 1.6 fold upregulated after 11C7). GSEA identified 45 pathways in which significant enrichment of differentially expressed transcripts were observed. Oxidative phosphorylation, electron/ion/cation transport, mRNA processing and synaptic transmission were among the most significantly affected pathways (TABLE 6).

TABLE 6

Top 20 gene expression changes in spinal cord at the level of injury (T8) after two weeks of treatment with the monoclonal mouse anti-Nogo A antibody 11C7

| Probe set name | p-value (ANOVA) | Fold change in anti-Nogo A-treated | Gene Title | Common name |
|---|---|---|---|---|
| 1383897_at | 0.022836 | 1.6 homologous | Similar to apoptosis-inducing factor (AIF)-mitochondrion-associated inducer of death; p53-responsive gene 3 (LOC361843), mRNA | |
| 1384687_at | 0.028576 | 0.7 (ENC-1) | Similar to Ectoderm-neural cortex-1 protein (LOC294674), mRNA | ENC-1 |
| 1398648_at | 0.002346 | 0.7 | Similar to malignant fibrous histiocytoma amplified sequence 1; MFH-amplified sequences with leucine-rich tandem repeats 1 (LOC306508), mRNA | |
| 1385349_at | 0.000320 | 0.7 | Similar to centrin 4 (LOC361934), mRNA | |
| 1369476_at | 0.040145 | 0.7 | ephrin B1 | Efnb1 |
| 1384863_at | 0.031062 | 1.4 | Similar to copine family member (LOC361433), mRNA | |
| 1380611_at | 0.048542 | 1.4 | Similar to FKBP51 (LOC361810), mRNA | |
| 1368726_a_at | 0.009647 factor | 0.7 | gonadotropin inducible ovarian transcription 2 | Giot2 |
| 1389666_at | 0.048066 1 | 0.7 | Similar to rod outer segment membrane protein (LOC309201), mRNA | |
| 1384950_at | 0.004045 beta; | 0.7 | Similar to phosphatidylinositol 4-kinase type 2 type II phosphatidylinositol 4-kinase beta (LOC305419), mRNA | |
| 1387606_at | 0.023480 | 0.7 | fibroblast growth factor 2 | FGF2 |

(continued)

Top 20 gene expression changes in spinal cord at the level of injury (T8) after two weeks of treatment with the monoclonal mouse anti-Nogo A antibody 11C7

| Probe set name | p-value (ANOVA) | Fold change in anti-Nogo A-treated | Gene Title | Common name |
|---|---|---|---|---|
| 1368911_at | 0.048008 | 0.7 | potassium inwardly-rectifying channel, subfamily J, member 8 | Kcnj8 |
| 1384437_at | 0.028250 | 0.7 | Similar to SWI/SNF-related matrix-associated actin-dependent regulator of chromatin a1 isoform a; sucrose nonfermenting 2-like protein 1; SNF2-like 1; global transcription activator homologous sequence (LOC317575), mRNA | |
| 1376828_at | 0.045858 | 0.7 (LOC312790), | Similar to retinoic acid inducible protein 3 mRNA | |
| 1395848_at | 0.022895 like | 1.3 | Similar to Down syndrome candidate region 1-protein 2 (LOC362627), mRNA | |
| 1374589_at | 0.031909 | 0.8 | Similar to Vezatin (LOC299738), mRNA | |
| 1375549_at | 0.035689 | 1.3 | ubiquitin specific protease 2 | |
| 1396214_at | 0.018671 | 0.8 | kit ligand | |
| 1382354_at | 0.021059 | 0.8 | Similar to Ab2-008 (LOC290270), mRNA | |
| 1396280_at | 0.036851 | 0.8 | Similar to T54 protein (LOC302560), mRNA | |

TABLE 7

GSEA performed on T8 dataset. Pathways with enriched genes either in IgG- or 11C7-treated after one week of treatment (q<0.05)

| Pathway Name | Pathway Source | Probesets | qvalue | Enrichment Direction |
|---|---|---|---|---|
| oxidative phosphorylation | KEGG | 64 | 8.76E-09 | 11C7 |
| | Sebastian | 45 | 4.52E-07 | IgG |
| electron transport | Celera | 89 | 1.03E-05 | 11 C7 |
| ion transport | Celera | 262 | 2.84E-05 | 11C7 |

(continued)

GSEA performed on T8 dataset. Pathways with enriched genes either in IgG- or 11C7-treated after one week of treatment (q<0.05)

| Pathway Name | Pathway Source | Probesets | qvalue | Enrichment Direction |
|---|---|---|---|---|
| nucleoside, nucleotide and nucleic acid metabolism | Celera | 1325 | 3.54E-05 | IgG |
| blood coagulation | Celera public | 10 | 5.67E-05 | IgG |
| cation transport | Celera | 203 | 5.79E-05 | 11 C7 |
| oxidative phosphorylation | Celera | 55 | 5.79E-05 | 11C7 |
| pre-mRNA processing | Celera | 169 | 9.62E-05 | IgG |
| synaptic transmission | Celera | 81 | 9.62E-05 | 11 C7 |
| expressed probesets that are unassigned to a pathway | gsea | 7048 | NA | NA |
| ribosome | KEGG | 51 | 0.000275 | 11 C7 |
| cholesterol biosynthesis | Celera | 11 | 0.00035 | 11 C7 |
| coagulation: anticoagulation | public Sebastian | 18 | 0.00035 | IgG |
| regulation of lipid, fatty acid and steroid metabolism | Celera | 17 | 0.000386 | 11 C7 |
| neuronal activities | Celera | 227 | 0.000536 | 11C7 |
| complement and coagulation cascades | KEGG | 24 | 0.000687 | IgG |
| coagulation: procoagulation | Sebastian | 27 | 0.000687 | IgG |
| nerve-nerve synaptic transmission | Celera | 24 | 0.000687 | 11C7 |
| mRNA splicing | Celera | 110 | 0.000885 | IgG |
| mRNA transcription regulation | Celera | 521 | 0.00106 | IgG |
| blood clotting | Celera | 30 | 0.00194 | IgG |
| ATP synthesis | KEGG | 20 | 0.00198 | 11C7 |
| cell adhesion | Celera | 230 | 0.00221 | IgG |
| cell communication | Celera | 388 | 0.00409 | IgG |
| coagulation: anticoagulation: anticoagulation | Sebastian | 8 | 0.0042 | IgG |
| immunity and defence | Celera | 446 | 0.00858 | IgG |
| DNA recombination | Celera | 13 | 0.00958 | IgG |
| mhci-mediated immunity | Celera | 15 | 0.0109 | 11C7 |

(continued)

GSEA performed on T8 dataset. Pathways with enriched genes either in IgG- or 11C7-treated after one week of treatment (q<0.05)

| Pathwav Name | Pathwav Source | Probesets | qvalue | Enrichment Direction |
|---|---|---|---|---|
| protein metabolism and modification | Celera | 1420 | 0.014 | IgG |
| prostaglandin and leukotriene metabolism | KEGG | 11 | 0.015 | IgG |
| stress response | Celera | 68 | 0.0155 | IgG |
| biosynthesis of steroids | KEGG | 14 | 0.0173 | 11C7 |
| coenzyme and prosthetic group metabolism | Celera | 44 | 0.0173 | IgG |
| mRNA transcription | Celera | 704 | 0.0213 | IgG |
| mhcii-mediated immunity | Celera | 10 | 0.0218 | 11C7 |
| vitamin/cofactor transport | Celera | 10 | 0.0218 | IgG |
| protein glycosylation | Celera | 88 | 0.024 | IgG |
| Jak-stat cascade | Celera | 42 | 0.0246 | IgG |
| Pathway Name | Pathway Source | Probesets | qvalue | Enrichment Direction |
| signalling:Rattus norvegicus:disease: atherosclerosis:tnf signalling pathway | Pathart | 11 | 0.0275 | IgG |
| pyrimidine metabolism | Celera | 32 | 0.0284 | IgG |
| transport | Celera | 481 | 0.0337 | 11C7 |
| cytokine and chemokine mediated signalling pathway | Celera | 69 | 0.0342 | IgG |
| nicotinic acetylcholine receptor signalling pathway | Celera / public | 23 | 0.0373 | 11C7 |
| mesoderm development | Celera | 171 | 0.0373 | IgG |
| coagulation: procoagulation: coagulation | Sebastian | 4 | 0.0377 | IgG |

*Spinal cord TI-7 (Proximal to the site of injury).* Welch T-test comparing the IgG-treated group to the 11C7-treated group resulted in 566 and 579 differentially expressed genes after one week and two weeks of treatment, respectively. The average fold change of the top 100 largest fold changes was $1.43 \pm 0.17$ after one week of treatment and $1.56 \pm 0.98$ after two weeks of treatment. The top 20 gene expression changes after one week of treatment are listed in TABLE 8 and after two weeks of treatment, in TABLE 9.

The largest changes at one week after 11C7 treatment replicated the theme observed at the site of injury: eight of the top 20 changes were related to ECM (lumican, collagens 1a1-2 and 5a1, fibulin 2, tetranectin, Matrix glycoprotein SC1/ECM2) and downregulated after treatment with 11C7. After two weeks of treatment, fold changes were slightly larger than after 1 week of treatment. Some of the largest changes were related to downregulation of transcripts encoding for proteins expressed in lymphocytes

Gene Set Enrichment Analysis (GSEA) identified a significant enrichment in 35 pathways after one week of treatment (TABLE 10), and 3 pathways (TABLE 11; q<0.05; 32 p<0.05) after two weeks of treatment. The most significantly affected pathways were ECM-mediated signalling, lipid metabolism and growth factor homeostasis after one week, and ion transport, growth factor homeostasis and mRNA transcription termination after two weeks of treatment.

TABLE 8

Top 20 gene expression chances in spinal cord at T1-7 (proximal to the site of injury) after one week of treatment with the monoclonal mouse anti-Nogo A antibody 11C7

| Probe Set ID | p-value (Welch t-test) | Fold change after 11C7 | Gene Title | Gene Symbol |
|---|---|---|---|---|
| 1396733_at | 0.012999 | 1.87 | Similar to tesmin (LOC309142), mRNA | --- |
| 1370493_a_at | 4.38E-04 | 1.85 | Paired Ig-like receptor-B (Pirb) mRNA, complete cds | --- |
| 1374616_at | 0.029271 | 0.55 | Similar to platelet-derived growth factor receptor-like (LOC290771), mRNA | --- |
| 1367749_at | 0.048803 | 0.56 | lumican | Lum |
| 1370775_a_at | 0.04171 | 0.56 | calcitonin/ calcitonin-related polypeptide, alpha | Calca |
| 1374334_at | 0.042824 heavy | 0.57 | Partial mRNA for immunoglobulin alpha chain (partial), complete constant region | --- |
| 1368420_at | 0.028017 | 1.74 | ceruloplasmin | Cp |
| 1370864_at | 0.024212 | 0.58 | collagen, type 1, alpha 1 | Col1a1 |
| 1393210_at | 0.015514 | 0.58 | Similar to Extracellular matrix protein 2 precursor (Matrix glycoprotein SC1/ECM2) (LOC291018), mRNA | --- |
| 1387854_at | 0.023509 | 0.59 | procollagen, type I, alpha 2 | Col1 a2 |
| 1377452_at | 0.047128 | 0.60 | Similar to tetranectin (LOC316099), mRNA | --- |
| 1370150_a_at | 0.021757 | 1.62 | thyroid hormone responsive protein | Thrsp |
| 1388116_at | 0.047749 | 0.63 | collagen, type 1, alpha 1 | Col1a1 |

(continued)

Top 20 gene expression chances in spinal cord at T1-7 (proximal to the site of injury) after one week of treatment with the monoclonal mouse anti-Nogo A antibody 11C7

| Probe Set ID | p-value (Welch t-test) | Fold change after 11C7 | Gene Title | Gene Symbol |
|---|---|---|---|---|
| 1371400_at | 0.015338 | 1.59 | thyroid hormone responsive protein | Thrsp |
| 1395333_at | 0.035054 | 0.66 | Similar to myelin P2 protein- mouse (LOC361918), mRNA | --- |
| 1368418_a_at | 0.026848 | 1.49 | ceruloplasmin | Cp |
| 1369955_at | 0.008515 | 0.68 | collagen, type V, alpha 1 | Col5a1 |
| 1389533_at | 0.048318 | 0.69 | fibulin 2 | Fbln2 |
| 1397180_at | 0.022456 | 0.70 | Similar to map kinase phosphatase-like protein MK-STYX (LOC360792), mRNA | --- |
| 1385430_at | 0.02245 | 1.42 (LOC309798), | Similar to Golgi coiled coil protein GCC185 mRNA | --- |

TABLE 9

Top 20 gene expression changes in spinal cord at T1-7 (proximal to the site of injury) after two weeks of treatment with the monoclonal mouse anti-Nogo A antibody 11C7

| Probe Set ID | p-value (Welch t-test) | Fold change after 11C7 | Gene Title | Gene Symbol |
|---|---|---|---|---|
| 1388272_at | 0.008604 | 0.13 (LOC299352), | Similar to lg gamma-2B chain C region mRNA | --- |
| 1371262_at | 0.019597 | 0.16 | Partial mRNA for immunoglobulin heavy chain variable region (IGHV gene), clone MZ1801-17 | --- |
| 1370394_at | 0.01089 | 0.17 | Rat anti-acetylcholine receptor antibody gene, rearranged lg gamma-2a chain, VDJC region, complete cds | --- |
| 1387902_a_at | 0.00679 | 0.20 | Rat anti-acetylcholine receptor antibody gene, kappa-chain, VJC region, complete cds | --- |

(continued)

Top 20 gene expression changes in spinal cord at T1-7 (proximal to the site of injury) after two weeks of treatment with the monoclonal mouse anti-Nogo A antibody 11C7

| Probe Set ID | p-value (Welch t-test) | Fold change after 11C7 | Gene Title | Gene Symbol |
|---|---|---|---|---|
| 1388149_at | 0.033528 family | 1.86 | transporter 1, ATP-binding cassette, sub-B (MDR/TAP) | Tap1 |
| 1398265_at | 0.036731 | 1.52 (CFTR/MRP), | ATP-binding cassette, sub-family C member 9 | Abcc9 |
| 1369304_at | 1.26E-04 | 1.51 | 6-pyruvoyl-tetrahydropterin synthase | Pts |
| 1368073_at | 0.027547 | 1.50 | interferon regulatory factor 1 | Irf1 |
| 1368472_at | 0.021049 | 1.50 | cadherin EGF LAG seven-pass G-type receptor 3 | Celsr3 |
| 1369885_at | 0.014586 | 1.46 | preoptic regulatory factor-1 | Porf1 |
| 1387242_at | 0.012609 | 1.45 | Protein kinase, interferon-inducible double stranded RNA dependent | Prkr |
| 1390340_a_at | 0.027697 factor | 0.69 | Similar to eukaryotic translation initiation 4G I (LOC287986), mRNA | --- |
| 1368000_at | 0.012805 | 0.69 | complement component 3 | C3 |
| 1384734_at | 0.00584 | 0.70 | neural cell adhesion molecule 2 | Ncam2 |
| 1395248_at | 0.033783 | 0.70 | Similar to ER degradation enhancing alpha mannosidase-like; A130059K23Rik (LOC297504), mRNA | --- |
| 1378219_at | 0.027976 | 0.71 tetratricopeptide | small glutamine rich protein with repeats 2 | Sgt2 |
| 1375765_at | 0.02259 2 | 0.71 | neural visinin-like Ca2+-binding protein type | Nvjp2 |
| 1382691_at | 0.006834 | 0.72 | splicing factor 3b, subunit 1, 155kD | Sf3b1 |
| 1384946_at | 0.013369 | 1.39 | Similar to toll-like receptor 1 (LOC305354), mRNA | --- |

(continued)

Top 20 gene expression changes in spinal cord at T1-7 (proximal to the site of injury) after two weeks of treatment with the monoclonal mouse anti-Nogo A antibody 11C7

| Probe Set ID | p-value (Welch t-test) | Fold change after 11C7 | Gene Title | Gene Symbol |
|---|---|---|---|---|
| 1391566_at | 0.041749 | 0.73 (Sentrin/SUMO-specific | Similar to Sentrin-specific protease 8 protease SENP8) (LOC315723), mRNA | --- |

TABLE 10

GSEA performed on T1-7 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| expressed probesets that are unassigned to a pathway | gsea | 6854 | NA | NA |
| extracellular matrix protein-mediated signalling | Celera | 37 | 1.11E-07 | IgG |
| lipid, fatty acid and steroid metabolism | Celera | 344 | 7.12E-07 | 11C7 |
| growth factor homeostasis | Celera | 7 | 0.000505 | IgG |
| glycolysis | Celera | 32 | 0.000687 | 11 C7 |
| glycolysis / gluconeogenesis | KEGG | 28 | 0.000913 | 11C7 |
| protein metabolism and modification | Celera | 1380 | 0.00267 | 11C7 |
| carbon fixation | KEGG | 13 | 0.00267 | 11C7 |
| carbohydrate metabolism | Celera | 221 | 0.00311 | 11 C7 |
| Alzheimer's disease | KEGG | 30 | 0.00397 | 11 C7 |
| intracellular protein traffic | Celera | 616 | 0.00397 | 11 C7 |
| endocytosis | Celera | 162 | 0.00423 | 11 C7 |
| amino acid metabolism | Celera | 121 | 0.00423 | 11 C7 |
| immunity and defence | Celera | 388 | 0.00476 | 11C7 |
| transport | Celera | 469 | 0.00565 | 11 C7 |
| cell communication | Celera | 360 | 0.00565 | IgG |
| stress response | Celera | 66 | 0.00615 | 11 C7 |
| amino acid transport | Celera | 32 | 0.00748 | 11C7 |
| Jak-stat cascade | Celera | 37 | 0.00748 | 11 C7 |
| purine metabolism | Celera | 56 | 0.00776 | 11 C7 |
| small molecule transport | Celera | 60 | 0.00816 | 11 C7 |
| cell adhesion-mediated signalling | Celera | 123 | 0.013 | IgG |

(continued)

GSEA performed on T1-7 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| cell structure | Celera | 261 | 0.0155 | 11C7 |
| exocytosis | Celera | 133 | 0.0155 | 11 C7 |
| alanine and aspartate metabolism | KEGG | 11 | 0.0161 | 11C7 |
| miscellaneous | Celera | 24 | 0.0176 | 11C7 |
| PDGF signalling pathway | Celera public | 16 | 0.0194 | 11 C7 |
| Alzheimer disease-presenilin pathway | Celera public | 32 | 0.0279 | 11C7 |
| signalling:Rattus norvegicus: disease:rheumatoid arthritis:gh signalling pathway | Pathart | 4 | 0.0285 | IgG |
| pentose phosphate pathway | KEGG | 13 | 0.0293 | 11C7 |
| signalling:Rattus norvegicus: disease:alzheimers: amyloidbeta-peptide signalling pathway | Pathart | 20 | 0.0332 | 11C7 |
| regulated exocytosis | Celera | 50 | 0.038 | 11 C7 |
| blood clotting | Celera | 25 | 0.038 | IgG |
| Huntington's disease | KEGG | 23 | 0.0443 | 11 C7 |
| purine metabolism | KEGG | 38 | 0.0443 | 11 C7 |
| amino acid biosynthesis | Celera | 33 | 0.0485 | 11 C7 |

TABLE 11

GSEA performed on T1-7 dataset. Pathways with enriched genes either in IgG- or 11C7- treated after two weeks of treatment (q<0.05)

| Pathway Name | Pathway Source | Probesets | pvalue | qvalue | Enrichment Direction |
|---|---|---|---|---|---|
| ion transport | Celera | 258 | 0.000252 | 0.0406 | IgG |
| growth factor homeostasis | Celera | 7 | 0.000278 | 0.0406 | 11 C7 |
| mRNA transcription termination | Celera | 7 | 0.000308 | 0.0406 | 11C7 |

*Spinal cord L1-5 (Distal to the site of injury).* Welch T-test comparing the IgG-treated group to the 11 C7-treated group resulted in 1303 and 1301 differentially expressed genes after one week and two weeks of treatment, respectively.

The average fold change of the top 100 largest fold changes was 1.72±0.5 after one week of treatment and 1.91±2.0 after two weeks of treatment. The top 20 gene expression changes after one week of treatment are listed in TABLE 12 and after two weeks of treatment, in TABLE 13.

The largest changes at one week after 11C7 treatment replicated the theme observed at the site of injury: eight of the top 20 changes were related to ECM (lumican, collagens 1a1-2 and 5a1, fibulin 2, tetranectin, Matrix glycoprotein SC1/ECM2) and downregulated after treatment with 11C7. After two weeks of treatment, fold changes were slightly larger than after 1 week of treatment. Some of the largest changes were related to downregulation of transcripts encoding for proteins expressed in lymphocytes

Gene Set Enrichment Analysis (GSEA) identified a significant enrichment in 151 pathways after one week of treatment (TABLE 14), and 116 pathways (TABLE 15) after two weeks of treatment. Very interestingly, immunity and defence —related pathway was highly significantly enriched in the direction of IgG —treated (downregulated after 11C7 —treatment) after two weeks of treatment, whereas transcripts in synaptic transmission, neuronal activities and neurotransmitter release —related pathways were significantly enriched (upregulated) after 11C7—treatment.

TABLE 12

Top 20 gene expression changes in spinal cord at L1-5 (distal to the site of injury) after one week of treatment with the monoclonal mouse anti-Nogo A antibody 11C7

| Probe set name | p-value (Welch t-test | Fold change after 11C7 | Gene Title | Common name |
|---|---|---|---|---|
| 1384218_at | 0.048806 | 4.6 | Similar to IG GAMMA-2C CHAIN C REGION (LOC362795), mRNA | --- |
| 1367998_at | 0.036222 | 3.8 | secretory leukocyte protease inhibitor | Slpi |
| 1369801_at | 0.036995 | 3.5 | selectin, lymphocyte | Sell |
| 1368441_at | 0.03155 | 2.9 | mesothelin | Msln |
| 1374070_at | 0.033238 | 2.9 | glutathione peroxidase 2 | Gpx2 |
| 1387868_at | 0.02313 | 2.7 | lipopolysaccharide binding protein | Lbp |
| 1384580_at | 0.025395 | 2.3 | complement component 6 | C6 |
| 1368448_at | 0.046104 binding | 2.3 | latent transforming growth factor beta protein 2 | Ltbp2 |
| 1387011_at | 0.030364 | 2.3 | lipocalin 2 | Lcn2 |
| 1385397_at | 0.02158 | 2.2 | Ab1-219 mRNA, complete cds | --- |
| 1398589_at | 0.044363 | 2.1 | Similar to cell surface receptor FDF03 (LOC288568), mRNA | --- |
| 1368900_at | 0.008563 | 2.1 | thrombomodulin | Thbd |
| 1374779_at | 0.008626 | 2.0 | coagulation factor XIIIa | F13a |
| 1387655_at | 0.01132 | 1.9 | chemokine (C-X-C motif) ligand 12 | Cxcl12 |

(continued)

Top 20 gene expression changes in spinal cord at L1-5 (distal to the site of injury) after one week of treatment with the monoclonal mouse anti-Nogo A antibody 11C7

| Probe set name | p-value (Welch t-test | Fold change after 11C7 | Gene Title | Common name |
|---|---|---|---|---|
| 1393891_at | 0.021901 | 1.9 | Similar to Collagen alpha 1 (VIII) chain precursor (LOC304021), mRNA | --- |
| 1369301_at | 0.032784 | 1.9 | angiotensin receptor-like 1 | Agtrl 1 |
| 1367712_at | 0.043348 | 1.8 | tissue inhibitor of metalloproteinase 1 | Timp1 |
| 1368394_at | 0.04073 | 1.8 | secreted frizzled-related protein 4 | Sfrp4 |
| 1372889_at | 0.020139 | 1.8 | matrin F/G 1 | Matr1 |
| 1374626_at | 0.010198 | 1.8 | Similar to leucine-rich alpha-2-glycoprotein (LOC367455), mRNA | --- |

TABLE 13

Top 20 gene expression changes in spinal cord at L1-5 (distal to the site of injury) after two weeks of treatment with the monoclonal mouse anti-Nogo A antibody 11C7

| Probe set name | p-value (Welch T-test | Fold change after 11C7 | Gene Title | Common name |
|---|---|---|---|---|
| 1385350_at | 0.039469 | 0.1 | nuclear receptor MrgA10 RF-amide G protein-coupled receptor | Mrga10 |
| 1383637_at | 0.019342 | 0.1 | tubulin, beta 5 | Tubb5 |
| 1382194_at | 0.045985 | 0.1 | nuclear receptor coactivator 3 | Ncoa3 |
| 1370933_at | 0.044151 | 0.1 | protein phosphatase 4, regulatory subunit 1 | Ppp4r1 |
| 1370919_at | 0.044097 | 0.3 | RT1 class II, locus DMa | RT1-DMa |
| 1388108_at | 0.020241 | 2.1 | fatty acid elongase 2 | rEL02 |
| 1379091_at | 0.045963 | 0.5 | Transcribed sequence with weak similarity to protein sp:O75325 (H.sapiens) GAC1_HUMAN Glioma amplified on chromosome 1 protein precursor | --- |

(continued)

Top 20 gene expression changes in spinal cord at L1-5 (distal to the site of injury) after two weeks of treatment with the monoclonal mouse anti-Nogo A antibody 11C7

| Probe set name | p-value (Welch T-test | Fold change after 11C7 | Gene Title | Common name |
|---|---|---|---|---|
| 1381310_at | 0.041052 | 2.0 | Similar to ubiquitin associated protein (LOC300788), mRNA | --- |
| 1387592_at | 0.014777 GOA - | 0.5 | Similar to ring finger B-box coiled-coil protein, human (LOC303683), mRNA | --- |
| 1375884_at | 0.039489 | 1.9 | Synaptogenesis-related mRNA sequence 6 | --- |
| 1371828_at | 0.018834 | 0.6 | BCL2/adenovirus E1B 19 kDa-interacting protein 3, nuclear gene for mitochondrial product | Bnip3 |
| 1396175_at | 0.04858 | 0.6 | synaptic vesicle glycoprotein 2 b | Sv2b |
| 1367940_at | 0.018827 | 0.6 | cathepsin S | Ctss |
| 1383478_at | 0.042591 | 0.6 | interferon-gamma inducible gene, Puma-g | Pumag |
| 1370697_a_at | 0.019342 | 1.6 | coagulation factor VIII | F8 |
| 1368982_at | 0.028419 | 1.6 | myosin IE | Myo1e |
| 1378377_at | 0.034481 | 1.6 | Similar to gamma-filamin (LOC362332), mRNA | --- |
| 1368565_at | 0.02172 | 0.6 | Similar to BAG-family molecular chaperone regulator-3 (BCL-2 binding athanogene-3) (BAG-3) (Bcl-2-binding protein Bis) (LOC293524), mRNA | --- |
| 1384878_at | 0.036861 | 1.6 | synaptoporin | Synpr |
| 1370972_x_at | 0.016236 | 1.5 | heterogeneous nuclear ribonucleoprotein M | Hnrpm |

TABLE 14

GSEA performed on L1-5 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| expressed probesets that are unassigned to a pathway | gsea | 6794 | NA | NA |
| immunity and defence | Celera | 393 | 3.44E-40 | 11C7 |
| signal transduction | Celera | 1336 | 5.02E-15 | 11C7 |
| cell communication | Celera | 350 | 5.14E-15 | 11C7 |
| ribosome | KEGG | 51 | 3.23E-12 | 11C7 |
| protein metabolism and modification | Celera | 1358 | 4.63E-12 | 11C7 |
| Jak-stat cascade | Celera | 38 | 5.02E-09 | 11 C7 |
| macrophage-mediated immunity | Celera | 52 | 5.02E-09 | 11C7 |
| integrin signalling pathway | Celera public | 48 | 5.02E-09 | 11 C7 |
| mesoderm development | Celera | 161 | 5.02E-09 | 11C7 |
| synaptic transmission | Celera | 84 | 2.13E-08 | IgG |
| cell structure and motility | Celera | 417 | 2.13E-08 | 11 C7 |
| extracellular matrix protein-mediated signalling | Celera | 36 | 2.75E-08 | 11C7 |
| cell surface receptor mediated signal transduction | Celera | 515 | 1.24E-07 | 11C7 |
| B-cell- and antibody- mediated immunity | Celera | 30 | 1.35E-07 | 11 C7 |
| complement and coagulation cascades | KEGG | 17 | 3.46E-07 | 11C7 |
| cytokine and chemokine mediated signalling pathway | Celera | 58 | 7.40E-07 | 11C7 |
| | Sebastian | 37 | 7.99E-07 | 11 C7 |
| granulocyte-mediated immunity | Celera | 18 | 8.20E-07 | 11C7 |
| blood clotting | Celera | 24 | 8.89E-07 | 11 C7 |
| proteolysis | Celera | 376 | 8.89E-07 | 11 C7 |
| signalling:Rattus norvegicus:disease: atherosclerosis: angiotensin signalling pathway | Pathart | 27 | 1.24E-06 | 11C7 |
| protein biosynthesis | Celera | 207 | 2.60E-06 | 11 C7 |
| skeletal development | Celera | 29 | 3.58E-06 | 11 C7 |
| apoptosis signalling pathway | Celera public | 46 | 3.59E-06 | 11 C7 |
| apoptosis | Celera | 228 | 3.59E-06 | 11 C7 |
| nerve-nerve synaptic transmission | Celera | 26 | 5.35E-06 | IgG |

(continued)

GSEA performed on L1-5 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| complement-mediated immunity | Celera | 15 | 5.53E-06 | 11C7 |
| interferon-mediated immunity | Celera | 29 | 6.32E-06 | 11C7 |
| developmental processes | Celera | 507 | 1.46E-05 | 11 C7 |
| oncogenesis | Celera | 280 | 1.61 E-05 | 11 C7 |
| other polysaccharide metabolism | Celera | 52 | 2.76E-05 | 11 C7 |
| cell adhesion-mediated signalling | Celera | 120 | 3.07E-05 | 11C7 |
| T-cell mediated immunity | Celera | 49 | 4.35E-05 | 11C7 |
| neuronal activities | Celera | 230 | 4.35E-05 | IgG |
| nucleoside, nucleotide and nucleic acid metabolism | Celera | 1255 | 4.43E-05 | 11C7 |
| cell structure | Celera | 258 | 6.22E-05 | 11C7 |
| toll receptor signalling pathway | Celera public | 14 | 6.44E-05 | 11C7 |
| ligand-mediated signalling | Celera | 131 | 9.47E-05 | 11C7 |
| signalling:Rattus norvegicus:physiology: growth and differentiation:NGF signalling pathway | Pathart | 33 | 0.000135 | 11C7 |

| Pathway Name | Pathway Source | Probesets | qvalue | Enrichment Direction |
|---|---|---|---|---|
| signalling:Rattus norvegicus:physiology: growth and differentiation:TGFbeta signalling pathway | Pathart | 15 | 0.000148 | 11C7 |
| coagulation: procoagulation | Sebastian | 24 | 0.000163 | 11C7 |
| angiogenesis | Celera public | 57 | 0.000163 | 11C7 |
| mapk signalling pathway | KEGG | 90 | 0.000246 | 11C7 |
| TGF-beta signalling pathway | Celera public | 29 | 0.000249 | 11C7 |
| b cell activation | Celera public | 26 | 0.000257 | 11 C7 |
| signalling:Rattus norvegicus:physiology: skeletal development:FGF signalling pathway | Pathart | 20 | 0.000287 | 11C7 |

(continued)

| Pathway Name | Pathway Source | Probesets | qvalue | Enrichment Direction |
|---|---|---|---|---|
| protein modification | Celera | 558 | 0.000308 | 11C7 |
| cell adhesion | Celera | 217 | 0.000401 | 11C7 |
| pi3 kinase pathway | Celera public | 25 | 0.000433 | 11 C7 |
| signalling:Rattus norvegicus:disease: obesity:responsive genes | Pathart | 16 | 0.000439 | 11C7 |
| signalling:Raftus norvegicus:disease: atherosclerosis:ldl signalling pathway | Pathart | 15 | 0.00044 | 11C7 |
| inflammation mediated by chemokine and cytokine signalling pathway | Celera public | 46 | 0.000453 | 11 C7 |
| toll-like receptor signalling pathway | KEGG | 27 | 0.000552 | 11C7 |
| hematopoesis | Celera | 48 | 0.00056 | 11 C7 |
| signalling:Rattus norvegicus:physiology: apoptosis:TGF beta induced apoptosis | Pathart | 20 | 0.000686 | 11C7 |
| Jak-stat signalling pathway | Celera public | 6 | 0.000768 | 11C7 |
| mRNA transcription regulation | Celera | 480 | 0.000768 | 11C7 |
| natural killer cell mediated immunity | Celera | 11 | 0.00086 | 11 C7 |
| growth factor homeostasis | Celera | 7 | 0.00115 | 11 C7 |
| signalling:Rattus norvegicus:physiology: cell adhesion:integrin signalling pathway | Pathart | 18 | 0.00128 | 11C7 |
| TGF-beta signalling pathway | KEGG | 25 | 0.0015 | 11C7 |
| signalling:Rattus norvegicus:disease: diabetes type ii:il1b signalling pathway | Pathart | 9 | 0.0015 | 11C7 |
| signalling:Rattus norvegicus:disease: Parkinsons disease:dopamine signalling pathway | Pathart | 26 | 0.00156 | 11C7 |
| inhibition of apoptosis | Celera | 54 | 0.00159 | 11 C7 |
| mRNA transcription | Celera | 660 | 0.00173 | 11C7 |
| signalling:Rattus | Pathart | 12 | 0.00173 | 11C7 |

(continued)

| Pathway Name | Pathway Source | Probesets | qvalue | Enrichment Direction |
|---|---|---|---|---|
| norvegicus:physiology: others:fcer1 signalling pathway | | | | |
| coagulation: anticoagulation | Sebastian | 13 | 0.00203 | 11 C7 |
| signalling:Rattus norvegicus:disease: alzheimers:amyloidbeta- peptide signalling pathway | Pathart | 19 | 0.00254 | 11C7 |
| cell motility | Celera | 94 | 0.00275 | 11C7 |
| coagulation: anticoagulation: anticoagulation | Sebastian | 6 | 0.00277 | 11C7 |
| signalling:Rattus norvegicus:disease: atherosclerosis:tnf signalling pathway | Pathart | 10 | 0.00322 | 11C7 |
| Huntington's disease | KEGG | 24 | 0.00343 | 11 C7 |
| cation transport | Celera | 197 | 0.00343 | IgG |
| NF-kappaB cascade | Celera | 29 | 0.00389 | 11 C7 |
| lipid, fatty acid and steroid metabolism | Celera | 341 | 0.00389 | 11C7 |
| Alzheimer disease- presenilin pathway | Celera public | 31 | 0.00433 | 11C7 |
| blood coagulation | Celera public | 7 | 0.00433 | 11 C7 |
| protein glycosylation | Celera | 83 | 0.00443 | 11 C7 |
| ion transport | Celera | 257 | 0.00464 | IgG |
| induction of apoptosis | Celera | 97 | 0.00513 | 11 C7 |
| endocytosis | Celera | 161 | 0.00541 | 11C7 |
| general vesicle transport | Celera | 178 | 0.00548 | 11 C7 |
| signalling:Rattus norvegicus:disease: atherosclerosis:insulin signalling pathway | Pathart | 7 | 0.00586 | 11C7 |
| p53 pathway | Celera public | 11 | 0.00592 | 11 C7 |
| apoptosis | KEGG | 31 | 0.0064 | 11C7 |
| fas signalling pathway | Celera public | 15 | 0.0066 | 11C7 |
| intracellular protein traffic | Celera | 611 | 0.00718 | 11C7 |
| signalling:Rattus norvegicus:physiology: growth and differentiation:PDGF signalling pathway | Pathart | 8 | 0.00718 | 11C7 |

(continued)

| Pathway Name | Pathway Source | Probesets | qvalue | Enrichment Direction |
|---|---|---|---|---|
| intracellular signalling cascade | Celera | 420 | 0.00882 | 11C7 |
| signalling:Rattus norvegicus:disease: obesity:leptin signalling pathway | Pathart | 24 | 0.00882 | 11C7 |
| other immune and defence | Celera | 29 | 0.00886 | 11C7 |
| axon guidance mediated by slit-robo | Celera public | 3 | 0.00909 | 11C7 |
| signalling:Rattus norvegicus:disease: diabetes type ii:ffa signalling pathway | Pathart | 13 | 0.00932 | 11C7 |
| signalling:Rattus norvegicus:physiology: growth and differentiation:FGF2 signalling pathway | Pathart | 11 | 0.00932 | 11C7 |
| neurotransmitter release | Celera | 19 | 0.00962 | IgG |
| stress response | Celera | 65 | 0.00985 | 11 C7 |
| signalling:Rattus norvegicus:disease: atherosclerosis:il1 signalling pathway | Pathart | 10 | 0.0102 | 11C7 |
| signalling:Rattus norvegicus:physiology: apoptosis:NGF signalling pathway | Pathart | 17 | 0.0112 | 11C7 |
| signalling:Rattus norvegicus:physiology: apoptosis:FGF signalling pathway | Pathart | 8 | 0.0112 | 11C7 |
| oxidative stress response | Celera public | 13 | 0.0136 | 11C7 |
| protein disulfide-isomerase reaction | Celera | 6 | 0.0136 | 11 C7 |
| Parkinson disease | Celera public | 48 | 0.0143 | 11 C7 |
| signalling:Rattus norvegicus:disease: alzheimers:igf1 signalling pathway | Pathart | 4 | 0.0152 | 11C7 |
| glycolysis / gluconeogenesis | KEGG | 27 | 0.0165 | 11C7 |
| T-cell activation | Celera public | 29 | 0.0165 | 11C7 |
| other transport | Celera | 26 | 0.0169 | 11 C7 |

(continued)

| Pathway Name | Pathway Source | Probesets | qvalue | Enrichment Direction |
|---|---|---|---|---|
| oncogene | Celera | 54 | 0.0169 | 11C7 |
| prostaglandin and leukotriene metabolism | KEGG | 7 | 0.0169 | 11C7 |
| PDGF signalling pathway | Celera public | 15 | 0.0173 | 11 C7 |
| mRNA splicing | Celera | 107 | 0.0177 | 11C7 |
| signalling:Rattus norvegicus:disease: obesity:cntf signalling pathway | Pathart | 6 | 0.0179 | 11C7 |
| cytokine/chemokine mediated immunity | Celera | 23 | 0.0203 | 11C7 |
| carbohydrate metabolism | Celera | 215 | 0.0203 | 11 C7 |
| porphyrin and chlorophyll metabolism | KEGG | 7 | 0.0203 | 11C7 |
| prion disease | KEGG | 6 | 0.0219 | 11C7 |
| n-glycan biosynthesis | KEGG | 8 | 0.0231 | 11C7 |
| signalling:Rattus norvegicus:disease: atherosclerosis:linoleic acid signalling pathway | Pathart | 3 | 0.0234 | 11 C7 |
| signalling:Rattus norvegicus:disease: atherosclerosis:aif mediated pathway | Pathart | 5 | 0.0236 | 11C7 |
| coagulation: procoagulation: jackson lab bleeding mice | Sebastian | 7 | 0.027 | 11C7 |
| other apoptosis | Celera | 9 | 0.027 | 11C7 |
| Huntington disease | Celera public | 44 | 0.0277 | 11C7 |
| signalling:Rattus norvegicus:disease: atherosclerosis:PDGF signalling pathway | Pathart | 8 | 0.0278 | 11C7 |
| nicotinic acetylcholine receptor signalling pathway | Celera public | 23 | 0.0296 | 11C7 |
| vitamin/cofactor transport | Celera | 9 | 0.0296 | 11 C7 |
| wnt signalling pathway | KEGG | 58 | 0.0303 | 11C7 |
| signalling:Rattus norvegicus:disease: alzheimers:hydrogen peroxide signalling pathway | Pathart | 8 | 0.0319 | 11C7 |
| other oncogenesis | Celera | 44 | 0.032 | 11 C7 |

(continued)

| Pathway Name | Pathway Source | Probesets | qvalue | Enrichment Direction |
|---|---|---|---|---|
| cell cycle | Celera public | 5 | 0.032 | 11C7 |
| signalling:Rattus norvegicus:disease: rheumatoid arthritis:interleukin signalling pathway | Pathart | 2 | 0.0323 | 11C7 |
| cell proliferation and differentiation | Celera | 138 | 0.0334 | 11C7 |
| urea cycle and metabolism of amino groups | KEGG | 11 | 0.0368 | 11C7 |
| other receptor mediated signalling pathway | Celera | 33 | 0.0369 | 11C7 |
| peptidoglycan biosynthesis | KEGG | 3 | 0.0377 | 11 C7 |
| lipid and fatty acid transport | Celera | 51 | 0.0404 | 11C7 |
| dentatorubropallidoluysian atrophy (drpla) | KEGG | 8 | 0.0404 | 11C7 |
| oxidative phosphorylation | Celera | 56 | 0.0404 | IgG |
| signalling:Rattus norvegicus:physiology: inflammation:il1 signalling pathway | Pathart | 2 | 0.0404 | 11 C7 |
| other protein metabolism | Celera | 27 | 0.0404 | IgG |
| EGF receptor signalling pathway | Celera | 36 | 0.0405 | 11C7 |
| signalling:Rattus norvegicus:disease: diabetes type ii:hexosamine mediated pathway | public Pathart | 16 | 0.0423 | 11C7 |
| gamma-hexachlorocyclohexane degradation | KEGG | 4 | 0.0429 | 11 C7 |
| metabotropic glutamate receptor group ii pathway | Celera | 9 | 0.0431 | 11 C7 |
| phagocytosis | public Celera | 16 | 0.0443 | 11C7 |
| signalling:Rattus norvegicus:physiology: apoptosis:wnt signalling pathway | Pathart | 7 | 0.0458 | 11C7 |
| signalling:Rattus norvegicus:disease: atherosclerosis:ifngamma signalling pathway | Pathart | 2 | 0.0458 | 11C7 |

(continued)

| Pathway Name | Pathway Source | Probesets | qvalue | Enrichment Direction |
|---|---|---|---|---|
| receptor protein serine/ threonine kinase signalling pathway | Celera | 28 | 0.046 | 11C7 |
| hypoxia response via hif activation | Celera | 13 | 0.0465 | 11C7 |
| arginine and proline metabolism | public KEGG | 20 | 0.0465 | 11C7 |
| glycolysis | Celera | 32 | 0.0465 | 11 C7 |
| signalling:Rattus norvegicus:disease: alzheimers:NGF signalling pathway | Pathart | 8 | 0.0473 | 11C7 |
| signalling:Rattus norvegicus:disease: alzheimers:icaml signalling pathway | Pathart | 5 | 0.0473 | 11C7 |

TABLE 15

GSEA performed on L1-5 dataset. Pathways with enriched genes either in IgG- or 11C7- treated after two weeks of treatment (q<0.05)

| Pathway Name | Pathway Source | Probesets | qvalue | Enrichment Direction |
|---|---|---|---|---|
| immunity and defence | Celera | 393 | 0 | IgG |
| expressed probesets that are unassigned to a pathway | gsea | 6794 | NA | NA |
| cell communication | Celera | 350 | 5.49E-11 | IgG |
| synaptic transmission | Celera | 84 | 1.15E-10 | 11 C7 |
| protein metabolism and modification | Celera | 1358 | 1.92E-10 | IgG |
| extracellular matrix protein-mediated signalling | Celera | 36 | 1.08E-09 | IgG |
| neuronal activities | Celera | 230 | 1.89E-09 | 11C7 |
| signal transduction | Celera | 1336 | 2.28E-08 | IgG |
| B-cell- and antibody-mediated immunity | Celera | 30 | 5.37E-08 | IgG |
| macrophage-mediated immunity | Celera | 52 | 5.72E-08 | IgG |
| T-cell mediated immunity | Celera | 49 | 1.66E-07 | IgG |
| blood clotting | Celera | 24 | 6.46E-07 | IgG |

(continued)

GSEA performed on L1-5 dataset. Pathways with enriched genes either in IgG- or 11C7- treated after two weeks of treatment (q<0.05)

| Pathway Name | Pathway Source | Probesets | qvalue | Enrichment Direction |
|---|---|---|---|---|
| integrin signalling pathway | Celera | 48 | 8.72E-07 | IgG |
| complement and coagulation cascades | public KEGG | 17 | 8.79E-07 | IgG |
| oncogene | Celera | 54 | 2.21 E-06 | IgG |
| cation transport | Celera | 197 | 4.01 E-06 | 11 C7 |
| oncogenesis | Celera | 280 | 6.38E-06 | IgG |
| ion transport | Celera | 257 | 6.92E-06 | 11 C7 |
| proteolysis | Celera | 376 | 1.24E-05 | IgG |
| | Sebastian | 37 | 2.01 E-05 | IgG |
| cytokine and chemokine mediated signalling pathway | Celera | 58 | 2.40E-05 | IgG |
| neurotransmitter release | Celera | 19 | 2.40E-05 | 11 C7 |
| protein modification | Celera | 558 | 8.85E-05 | IgG |
| apoptosis | Celera | 228 | 8.85E-05 | IgG |
| cell adhesion-mediated signalling | Celera | 120 | 9.26E-05 | IgG |
| neuroactive ligand-receptor interaction | KEGG | 52 | 0.000111 | 11 C7 |
| mhcii-mediated immunity | Celera | 10 | 0.000115 | IgG |
| other polysaccharide metabolism | Celera | 52 | 0.000144 | IgG |
| nucleoside, nucleotide and nucleic acid metabolism | Celera | 1255 | 0.000191 | IgG |
| nerve-nerve synaptic transmission | Celera | 26 | 0.000245 | 11 C7 |
| complement-mediated immunity | Celera | 15 | 0.000245 | IgG |
| ionotropic glutamate receptor pathway | Celera | 24 | 0.000245 | 11 C7 |
| T-cell activation | public Celera public | 29 | 0.000245 | IgG |
| ligand-mediated signalling | Celera | 131 | 0.000245 | IgG |
| skeletal development | Celera | 29 | 0.000282 | IgG |
| mesoderm development | Celera | 161 | 0.000296 | IgG |

(continued)

GSEA performed on L1-5 dataset. Pathways with enriched genes either in IgG- or 11C7- treated after two weeks of treatment (q<0.05)

| Pathway Name | Pathway Source | Probesets | qvalue | Enrichment Direction |
|---|---|---|---|---|
| apoptosis signalling pathway | Celera public | 46 | 0.000296 | IgG |
| inflammation mediated by chemokine and cytokine signalling pathway | Celera public | 46 | 0.000304 | IgG |
| growth factor homeostasis | Celera | 7 | 0.000316 | IgG |
| protein glycosylation | Celera | 83 | 0.000341 | IgG |
| p53 pathway | Celera public | 11 | 0.000393 | IgG |

| Pathway Name | Pathway Source | Probesets | value | Enrichment Direction |
|---|---|---|---|---|
| inhibition of apoptosis | Celera | 54 | 0.000439 | IgG |
| toll receptor signalling pathway | Celera public | 14 | 0.000465 | IgG |
| Jak-stat cascade | Celera | 38 | 0.000533 | IgG |
| NF-kappaB cascade | Celera | 29 | 0.000538 | IgG |
| B-cell activation | Celera public | 26 | 0.000611 | IgG |
| signalling:Rattus norvegicus:physiology:cell adhesion:integrin signalling pathway | Pathart | 18 | 0.000633 | IgG |
| cell adhesion | Celera | 217 | 0.000905 | IgG |
| nicotinate and nicotinamide metabolism | KEGG | 16 | 0.000962 | IgG |
| insulin-igf pathway-protein kinase b signalling cascade | Celera public | 18 | 0.00119 | IgG |
| oxidative phosphorylation | KEGG | 65 | 0.00139 | 11C7 |
| cell structure and motility | Celera | 417 | 0.00145 | IgG |
| oxidative phosphorylation | Celera | 56 | 0.00151 | 11 C7 |
| pre-mRNA processing | Celera | 162 | 0.00158 | IgG |
| coagulation: anticoagulation | Sebastian | 13 | 0.00192 | IgG |

(continued)

| Pathway Name | Pathway Source | Probesets | value | Enrichment Direction |
|---|---|---|---|---|
| cell motility | Celera | 94 | 0.00256 | IgG |
| coagulation: procoagulation | Sebastian | 24 | 0.00375 | IgG |
| protein disulfide-isomerase reaction | Celera | 6 | 0.00375 | IgG |
| toll-like receptor signalling pathway | KEGG | 27 | 0.00421 | IgG |
| granulocyte-mediated immunity | Celera | 18 | 0.00473 | IgG |
| apoptosis | KEGG | 31 | 0.00588 | IgG |
| signalling:Rattus norvegicus: disease: rheumatoid arthritis:gh signalling pathway | Pathart | 4 | 0.00611 | IgG |
| signalling:Rattus norvegicus: disease: atherosclerosis: angiotensin signalling pathway | Pathart | 27 | 0.00652 | IgG |
| transport | Celera | 464 | 0.0069 | 11 C7 |
| signalling:Rattus norvegicus: physiology:others: fcer1 signalling pathway | Pathart | 12 | 0.0071 | IgG |
| n-glycan biosynthesis | KEGG | 8 | 0.00736 | IgG |
| signalling:Rattus norvegicus: disease: atherosclerosisanf signalling pathway | Pathart | 10 | 0.00752 | IgG |
| other apoptosis | Celera | 9 | 0.00783 | IgG |
| metabotropic glutamate receptor group iii pathway | Celera public | 19 | 0.00783 | 11 C7 |
| hypoxia response via hif activation | Celera public | 13 | 0.00806 | IgG |
| mRNA transcription regulation | Celera | 480 | 0.00921 | IgG |
| signalling:Rattus norvegicus: physiology:growth and differentiation:NGF signalling pathway | Pathart | 33 | 0.00998 | IgG |

(continued)

| Pathway Name | Pathway Source | Probesets | value | Enrichment Direction |
|---|---|---|---|---|
| TGF-beta signalling pathway | Celera | 29 | 0.0112 | IgG |
| Parkinson's disease | public KEGG | 16 | 0.0112 | 11 C7 |
| angiogenesis | Celera | 57 | 0.0114 | IgG |

GSEA performed on L 1-5 dataset. Pathways with enriched genes either in IgG- or 11C7- treated after two weeks of treatment (q<0.05)

| Pathway Name | Pathway Source | Probesets | qvalue | Enrichment Direction |
|---|---|---|---|---|
| signalling:Rattus norvegicus: disease:diabetes type ii:il1b signalling pathway | public Pathart | 9 | 0.0117 | IgG |
| electron transport | Celera | 89 | 0.0131 | 11C7 |
| insulin-igf pathway-mitogen activated protein kinase kinase-map kinase cascade | Celera | 14 | 0.0133 | IgG |
| signalling:Rattus norvegicus: disease: atherosclerosis:ldl signalling pathway | public Pathart | 15 | 0.0136 | IgG |
| natural killer cell mediated immunity | Celera | 11 | 0.0138 | IgG |
| axon guidance mediated by slit-robo | Celera | 3 | 0.0139 | IgG |
| monosaccharide metabolism | public Celera | 27 | 0.0141 | IgG |
| starch and sucrose metabolism | KEGG | 20 | 0.0141 | IgG |
| stress response | Celera | 65 | 0.0141 | IgG |
| lipid, fatty acid and steroid metabolism | Celera | 341 | 0.0142 | IgG |
| blood coagulation | Celera | 7 | 0.0144 | IgG |
| inositol phosphate metabolism | public KEGG | 22 | 0.0144 | IgG |
| extracellular transport and import | Celera | 35 | 0.0144 | 11C7 |
| mRNA splicing | Celera | 107 | 0.0152 | IgG |
| signalling:Rattus | Pathart | 16 | 0.0152 | IgG |

(continued)

GSEA performed on L 1-5 dataset. Pathways with enriched genes either in IgG- or 11C7- treated after two weeks of treatment (q<0.05)

| Pathway Name | Pathway Source | Probesets | qvalue | Enrichment Direction |
|---|---|---|---|---|
| norvegicus: disease:obesity: responsive genes | | | | |
| pi3 kinase pathway | Celera public | 25 | 0.016 | IgG |
| signalling:Rattus norvegicus: disease: alzheimers: amyloidbeta- peptide signalling pathway | Pathart | 19 | 0.0165 | IgG |
| receptor protein serine/threonine kinase signalling pathway | Celera | 28 | 0.0165 | IgG |
| MAPKKK cascade | Celera | 111 | 0.0178 | IgG |
| fas signalling pathway | Celera public | 15 | 0.0179 | IgG |
| glycosphingolipid metabolism | KEGG | 9 | 0.0188 | IgG |
| ribosome | KEGG | 51 | 0.02 | IgG |
| intracellular signalling cascade | Celera | 420 | 0.023 | IgG |
| protein biosynthesis | Celera | 207 | 0.0232 | IgG |
| interleukin signalling pathway | Celera public | 23 | 0.0249 | IgG |
| coagulation: anticoagulation: anticoagulation | Sebastian | 6 | 0.0253 | IgG |
| signalling:Rattus norvegicus: physiology: apoptosis:TGF beta induced apoptosis | Pathart | 20 | 0.0256 | IgG |
| other immune and defence | Celera | 29 | 0.0266 | IgG |
| signalling:Rattus norvegicus: disease:obesity: leptin signalling pathway | Pathart | 24 | 0.0273 | IgG |
| bile acid biosynthesis | KEGG | 10 | 0.0277 | IgG |
| carbohydrate metabolism | Celera | 215 | 0.0288 | IgG |

(continued)

GSEA performed on L1-5 dataset. Pathways with enriched genes either in IgG- or 11C7- treated after two weeks of treatment (q<0.05)

| Pathway Name | Pathway Source | Probesets | qvalue | Enrichment Direction |
|---|---|---|---|---|
| signalling:Rattus norvegicus: disease: atherosclerosis: insulin signalling pathway | Pathart | 7 | 0.0327 | IgG |
| induction of apoptosis | Celera | 97 | 0.0332 | IgG |
| benzoate degradation via coa ligation | KEGG | 19 | 0.0334 | IgG |
| phagocytosis | Celera | 16 | 0.0337 | IgG |
| cell surface receptor mediated signal transduction | Celera | 515 | 0.0351 | IgG |
| signalling:Rattus norvegicus: disease: atherosclerosis: linoleic acid signalling pathway | Pathart | 3 | 0.0356 | IgG |
| coagulation: procoagulation: possible positive modulators platelet aggr. | Sebastian | 3 | 0.0387 | IgG |
| coagulation: procoagulation: synthesis and transport | Sebastian | 3 | 0.0397 | IgG |
| signalling:Rattus norvegicus: physiology: inflammation:il1 signalling pathway | Pathart | 2 | 0.0403 | IgG |
| phospholipid metabolism | Celera | 52 | 0.0403 | IgG |
| signalling:Rattus norvegicus: physiology:growth and differentiation:akt mediated pathway | Pathart | 4 | 0.0456 | IgG |

*Motor-Somatosensory Cortex.* Welch T-test comparing the IgG-treated group to the 11C7-treated group resulted in 1303 and 1301 differentially expressed genes after one week and two weeks of treatment, respectively. The average fold change of the top 100 largest fold changes was 1.72±0.5 after one week of treatment and 1.91±2.0 after two weeks of treatment. The top 20 gene expression changes after one week of treatment are listed in TABLE 12 and after two weeks of treatment, in TABLE 13.

The largest changes at one week after 11C7 treatment replicated the theme observed at the site of injury: eight of the top 20 changes were related to ECM (lumican, collagens 1al-2 and 5al, fibulin 2, tetranectin, Matrix glycoprotein SC1/ECM2) and downregulated after treatment with 11C7. After two weeks of treatment, fold changes were slightly larger than after 1 week of treatment. Some of the largest changes were related to downregulation of transcripts encoding for proteins expressed in lymphocytes

Gene Set Enrichment Analysis (GSEA) identified a significant enrichment in 151 pathways after one week of treatment (TABLE 14), and 116 pathways (TABLE 15) after two weeks of treatment. The most significantly affected pathways were ECM-mediated signalling, lipid metabolism and growth factor homeostasis after one week, and ion transport, growth factor homeostasis and mRNA transcription termination after two weeks of treatment.

EXAMPLE 3

LISTS OF PATHWAYS WITH SIGNIFICANT GENE ENRICHMENT IDENTIFIED BY GENE SET ENRICHMENT ANALYSIS (GSEA)

TABLE 16

GSEA performed on T8 dataset. Pathways with enriched genes either in IgG- or 11 C7- treated after one week of treatment (q<0.05)

| Pathway Name | Source | Probe sets | qvalue | Enrichment Direction |
|---|---|---|---|---|
| expressed probesets that are unassigned to a pathway | gsea | 7048 | NA | NA |
| immunity and defence | Celera | 446 | 1.94E-21 | 11C7 |
| cytokine and chemokine mediated signalling pathway | Celera | 69 | 2.47E-12 | 11C7 |
| Jak-stat cascade | Celera | 42 | 8.52E-10 | 11 C7 |
| protein metabolism and modification | Celera | 1420 | 1.56E-09 | 11C7 |
| interferon-mediated immunity | Celera | 32 | 1.17E-08 | 11 C7 |
| macrophage-mediated immunity | Celera | 58 | 1.77E-08 | 11 C7 |
| inhibition of apoptosis | Celera | 61 | 1.48E-07 | 11 C7 |
| nucleoside, nucleotide and nucleic acid metabolism | Celera | 1325 | 4.38E-07 | 11C7 |
| NF-kappaB cascade | Celera | 33 | 5.42E-06 | 11C7 |
| B-cell- and antibody-mediated immunity | Celera | 35 | 1.97E-05 | 11C7 |
| granulocyte-mediated immunity | Celera | 21 | 4.45E-05 | 11 C7 |
| intracellular protein traffic | Celera | 623 | 4.45E-05 | 11 C7 |
| toll-like receptor signalling pathway | KEGG | 29 | 4.45E-05 | 11C7 |
| natural killer cell mediated immunity | Celera | 13 | 5.94E-05 | 11C7 |
| Apoptosis | Celera | 247 | 8.75E-05 | 11C7 |
| Proteolysis | Celera | 400 | 0.00032 | 11 C7 |
| ectoderm development | Celera | 153 | 0.00032 | IgG |
| cell motility | Celera | 99 | 0.00037 | 11C7 |

(continued)

GSEA performed on T8 dataset. Pathways with enriched genes either in IgG- or 11 C7- treated after one week of treatment (q<0.05)

| Pathway Name | Source | Probe sets | qvalue | Enrichment Direction |
|---|---|---|---|---|
| Cytokine/chemokine mediated immunity | Celera | 31 | 0.000419 | 11C7 |
| apoptosis signalling pathway | Celera | 51 | 0.000419 | 11C7 |
| | public | | | |
| DNA metabolism | Celera | 128 | 0.000419 | 11C7 |
| Jak-stat signalling pathway | Celera | 8 | 0.000455 | 11C7 |
| | public | | | |
| protein modification | Celera | 588 | 0.000491 | 11 C7 |
| Apoptosis | KEGG | 39 | 0.000501 | 11 C7 |
| protein glycosylation | Celera | 88 | 0.000503 | 11C7 |
| Endocytosis | Celera | 164 | 0.000894 | 11 C7 |
| T-cell mediated immunity | Celera | 58 | 0.00093 | 11 C7 |
| cell cycle | Celera | 392 | 0.001 | 11C7 |
| neuronal activities | Celera | 227 | 0.001 | IgG |
| Neurogenesis | Celera | 143 | 0.0011 | IgG |

GSEA performed on T8 dataset. Pathways with enriched genes either in IgG- or 11C7- treated after one week of treatment (q<0.05)

| Pathway Name | Source | Probe sets | qvalue | Enrichment Direction |
|---|---|---|---|---|
| Hematopoesis | Celera | 53 | 0.00119 | 11C7 |
| toll receptor signalling pathway | Celera | 15 | 0.00174 | 11C7 |
| | public | | | |
| DNA replication | Celera | 47 | 0.0021 | 11C7 |
| carbohydrate metabolism | Celera | 228 | 0.0021 | 11C7 |
| mapk signalling pathway | KEGG | 101 | 0.00232 | 11C7 |
| Huntington's disease | KEGG | 26 | 0.00356 | 11C7 |
| Proteasome | KEGG | 19 | 0.0061 | 11C7 |
| MAPKKK cascade | Celera | 114 | 0.0061 | 11C7 |
| other immune and defence | Celera | 32 | 0.00647 | 11 C7 |
| cell adhesion-mediated signalling | Celera | 128 | 0.00703 | IgG |
| signalling:Rattus norvegicus:disease: atherosclerosis:aif mediated pathway | Pathart | 5 | 0.00806 | 11C7 |
| Exocytosis | Celera | 131 | 0.00806 | 11C7 |
| receptor mediated endocytosis | Celera | 68 | 0.00806 | 11 C7 |
| pre-mRNA processing | Celera | 169 | 0.00927 | 11 C7 |
| cell structure | Celera | 267 | 0.0097 | IgG |
| signalling:Rattus norvegicus:disease: atherosclerosis:ifngam | Pathart | 4 | 0.0132 | 11C7 |

(continued)

GSEA performed on T8 dataset. Pathways with enriched genes either in IgG- or 11C7- treated after one week of treatment (q<0.05)

| Pathway Name | Source | Probe sets | qvalue | Enrichment Direction |
|---|---|---|---|---|
| ma signalling pathway | | | | |
| Glycolysis | Celera | 34 | 0.0137 | 11C7 |
| signalling:Rattus norvegicus:disease: atherosclerosis:angiotensin signalling pathway | Pathart | 28 | 0.0137 | 11C7 |
| signalling:Rattus norvegicus:physiology: growth and differentiation:FGF2 signalling pathway | Pathart | 12 | 0.0137 | 11C7 |
| signalling:Rattus norvegicus:physiology: cell adhesion:integrin signalling pathway | Pathart | 19 | 0.0137 | IgG |
| cell cycle control | Celera | 185 | 0.0146 | 11C7 |
| protein disulfide-isomerase reaction | Celera | 5 | 0.0155 | 11C7 |
| pi3 kinase pathway | Celera public | 24 | 0.0157 | 11C7 |
| signalling:Rattus norvegicus: physiology: apoptosisanf signalling pathway | Pathart | 8 | 0.0157 | 11 C7 |
| signalling:Rattus norvegicus:disease: rheumatoid arthritis:interleukin signalling pathway | Pathart | 3 | 0.0164 | 11C7 |
| metabolism of cyclic nucleotides | Celera | 23 | 0.0164 | IgG |
| non-vertebrate process | Celera | 12 | 0.0164 | IgG |
| PDGF signalling pathway | Celera public | 19 | 0.0165 | 11 C7 |
| dentatorubropallidoluysian atrophy (drpla) | KEGG | 12 | 0.0177 | 11C7 |

GSEA performed on T8 dataset. Pathways with enriched genes either in IgG- or 11C7- treated after one week of treatment (q<0.051)

| Pathway Name | Source | Probe sets | qvalue | Enrichment Direction |
|---|---|---|---|---|
| starch and sucrose metabolism | KEGG | 25 | 0.0179 | 11 C7 |
| axon guidance mediated by slit-robo | Celera public | 3 | 0.0183 | 11C7 |
| growth factor homeostasis | Celera | 8 | 0.0187 | IgG |

(continued)

GSEA performed on T8 dataset. Pathways with enriched genes either in IgG- or 11C7- treated after one week of treatment (q<0.051)

| Pathway Name | Source | Probe sets | qvalue | Enrichment Direction |
|---|---|---|---|---|
| other nucleoside, nucleotide and nucleic acid metabolism | Celera | 18 | 0.0204 | 11C7 |
| signalling:Rattus norvegicus:disease: atherosclerosis:nfkb signalling pathway | Pathart | 3 | 0.0216 | 11 C7 |
| signalling:Rattus norvegicus:disease: atherosclerosis:ldl signalling pathway | Pathart | 17 | 0.0216 | 11C7 |
| glycolysis / gluconeogenesis | KEGG | 29 | 0.0223 | 11 C7 |
| nerve-nerve synaptic transmission | Celera | 24 | 0.0223 | IgG |
| glycosphingolipid metabolism | KEGG | 9 | 0.0223 | 11C7 |
| signalling:Rattus norvegicus:physiology: others:fcer1 signalling pathway | Pathart | 13 | 0.0236 | 11C7 |
| intracellular signalling cascade | Celera | 438 | 0.0252 | 11C7 |
| signalling:Rattus norvegicus:disease: atherosclerosis:throm bomodulin signalling pathway | Pathart | 5 | 0.0262 | IgG |
| inflammation mediated by chemokine and cytokine signalling pathway | Celera public | 48 | 0.0281 | 11C7 |
| signalling:Rattus norvegicus:physiology: apoptosis:TGF beta induced apoptosis | Pathart | 23 | 0.0291 | 11 C7 |
| Anterior/posterior patterning | Celera | 5 | 0.0293 | IgG |
| other polysaccharide metabolism | Celera | 56 | 0.0302 | 11 C7 |
| Synaptic transmission | Celera | 81 | 0.0308 | IgG |
| n-glycan biosynthesis | KEGG | 8 | 0.0317 | 11C7 |
| signalling:Rattus norvegicus:disease: multiple sclerosis: responsive genes | Pathart | 3 | 0.032 | 11C7 |
| p53 pathway | Celera | 12 | 0.032 | 11 C7 |

(continued)

GSEA performed on T8 dataset. Pathways with enriched genes either in IgG- or 11C7- treated after one week of treatment (q<0.051)

| Pathway Name | Source | Probe sets | qvalue | Enrichment Direction |
|---|---|---|---|---|
| | public | | | |
| signalling:Rattus norvegicus: physiology: apoptosisarail mediated apoptosis | Pathart | 5 | 0.034 | 11C7 |
| DNA recombination | Celera | 13 | 0.0378 | 11 C7 |
| regulated exocytosis | Celera | 50 | 0.0378 | 11C7 |
| blood circulation and gas exchange | Celera | 16 | 0.0378 | IgG |
| Histidine metabolism | KEGG | 10 | 0.0395 | IgG |
| complement-mediated immunity | Celera | 16 | 0.0401 | 11C7 |
| general vesicle transport | Celera | 180 | 0.0403 | 11C7 |
| monosaccharide metabolism | Celera | 31 | 0.0428 | 11 C7 |

GSEA performed on T8 dataset. Pathways with enriched genes either in IgG- or 11C7- treated after one week of treatment (q<0.05)

| Pathway Name | Source | Probe sets | qvalue | Enrichment Direction |
|---|---|---|---|---|
| Gamma-hexachlorocyclohexane degradation | KEGG | 5 | 0.0436 | 11 C7 |
| cholesterol biosynthesis | Celera public | 11 | 0.047 | 11 C7 |
| biosynthesis of steroids | KEGG | 14 | 0.0471 | 11C7 |
| signalling:Rattus norvegicus:disease: alzheimers:igf1 signalling pathway | Pathart | 4 | 0.049 | 11 C7 |
| signalling:Rattus norvegicus:disease: atherosclerosis:il1 beta signalling pathway | Pathart | 2 | 0.0493 | 11 C7 |
| b cell activation | Celera public | 26 | 0.0497 | 11 C7 |

TABLE 17

GSEA performed on T8 dataset. Pathways with enriched genes either in IgG- or 11C7-treated after two weeks of treatment (q<0.05)

| Pathway Name | Pathway Source | Probesets | qvalue | Enrichment Direction |
|---|---|---|---|---|
| oxidative phosphorylation | KEGG | 64 | 8.76E-09 | 11C7 |
| | Sebastian | 45 | 4.52E-07 | IgG |
| electron transport | Celera | 89 | 1.03E-05 | 11 C7 |
| ion transport | Celera | 262 | 2.84E-05 | 11 C7 |
| nucleoside, nucleotide and nucleic acid | Celera | 1325 | 3.54E-05 | IgG |

(continued)

GSEA performed on T8 dataset. Pathways with enriched genes either in IgG- or 11C7-treated after two weeks of treatment (q<0.05)

| Pathway Name | Pathway Source | Probesets | qvalue | Enrichment Direction |
|---|---|---|---|---|
| metabolism | | | | |
| blood coagulation | Celera public | 10 | 5.67E-05 | IgG |
| cation transport | Celera | 203 | 5.79E-05 | 11 C7 |
| oxidative phosphorylation | Celera | 55 | 5.79E-05 | 11 C7 |
| pre-mRNA processing | Celera | 169 | 9.62E-05 | IgG |
| synaptic transmission | Celera | 81 | 9.62E-05 | 11 C7 |
| expressed probesets that are unassigned to a pathway | gsea | 7048 | NA | NA |
| ribosome | KEGG | 51 | 0.000275 | 11 C7 |
| cholesterol biosynthesis | Celera public | 11 | 0.00035 | 11 C7 |
| coagulation: anticoagulation | Sebastian | 18 | 0.00035 | IgG |
| regulation of lipid, fatty acid and steroid metabolism | Celera | 17 | 0.000386 | 11C7 |
| neuronal activities | Celera | 227 | 0.000536 | 11C7 |
| complement and coagulation cascades | KEGG | 24 | 0.000687 | IgG |
| coagulation: procoagulation | Sebastian | 27 | 0.000687 | IgG |
| nerve-nerve synaptic transmission | Celera | 24 | 0.000687 | 11 C7 |
| mRNA splicing | Celera | 110 | 0.000885 | IgG |
| mRNA transcription regulation | Celera | 521 | 0.00106 | IgG |
| blood clotting | Celera | 30 | 0.00194 | IgG |
| ATP synthesis | KEGG | 20 | 0.00198 | 11C7 |
| cell adhesion | Celera | 230 | 0.00221 | IgG |
| cell communication | Celera | 388 | 0.00409 | IgG |
| coagulation: anticoagulation: anticoagulation | Sebastian | 8 | 0.0042 | IgG |
| immunity and defence | Celera | 446 | 0.00858 | IgG |
| DNA recombination | Celera | 13 | 0.00958 | IgG |
| mhci-mediated immunity | Celera | 15 | 0.0109 | 11C7 |
| protein metabolism and modification | Celera | 1420 | 0.014 | IgG |

(continued)

GSEA performed on T8 dataset. Pathways with enriched genes either in IgG- or 11C7-treated after two weeks of treatment (q<0.05)

| Pathway Name | Pathway Source | Probesets | qvalue | Enrichment Direction |
|---|---|---|---|---|
| prostaglandin and leukotriene metabolism | KEGG | 11 | 0.015 | IgG |
| stress response | Celera | 68 | 0.0155 | IgG |
| biosynthesis of steroids | KEGG | 14 | 0.0173 | 11 C7 |
| coenzyme and prosthetic group metabolism | Celera | 44 | 0.0173 | IgG |
| mRNA transcription | Celera | 704 | 0.0213 | IgG |
| mhcii-mediated immunity | Celera | 10 | 0.0218 | 11C7 |
| vitamin/cofactor transport | Celera | 10 | 0.0218 | IgG |
| protein glycosylation | Celera | 88 | 0.024 | IgG |
| Jak-stat cascade | Celera | 42 | 0.0246 | IgG |
| signalling:Rattus norvegicus: disease: atherosclerosisanf signalling pathway | Pathart | 11 | 0.0275 | IgG |
| pyrimidine metabolism | Celera | 32 | 0.0284 | IgG |
| transport | Celera | 481 | 0.0337 | 11 C7 |
| cytokine and chemokine mediated signalling pathway | Celera | 69 | 0.0342 | IgG |
| nicotinic acetylcholine receptor signalling pathway | Celera public | 23 | 0.0373 | 11C7 |
| mesoderm development | Celera | 171 | 0.0373 | IgG |
| coagulation: procoagulation: coagulation | Sebastian | 4 | 0.0377 | IgG |

TABLE 18

GSEA performed on T1-7 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| expressed probesets that are unassigned to a pathway | gsea | 6854 | NA | NA |

(continued)

GSEA performed on T1-7 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| extracellular matrix protein-mediated signalling | Celera | 37 | 1.11E-07 | IgG |
| lipid, fatty acid and steroid metabolism | Celera | 344 | 7.12E-07 | 11C7 |
| growth factor homeostasis | Celera | 7 | 0.000505 | IgG |
| glycolysis | Celera | 32 | 0.000687 | 11 C7 |
| glycolysis / gluconeogenesis | KEGG | 28 | 0.000913 | 11 C7 |
| protein metabolism and modification | Celera | 1380 | 0.00267 | 11 C7 |
| carbon fixation | KEGG | 13 | 0.00267 | 11 C7 |
| carbohydrate metabolism | Celera | 221 | 0.00311 | 11 C7 |
| Alzheimer's disease | KEGG | 30 | 0.00397 | 11 C7 |
| intracellular protein traffic | Celera | 616 | 0.00397 | 11C7 |
| endocytosis | Celera | 162 | 0.00423 | 11 C7 |
| amino acid metabolism | Celera | 121 | 0.00423 | 11 C7 |
| immunity and defence | Celera | 388 | 0.00476 | 11C7 |
| transport | Celera | 469 | 0.00565 | 11C7 |
| cell communication | Celera | 360 | 0.00565 | IgG |
| stress response | Celera | 66 | 0.00615 | 11 C7 |
| amino acid transport | Celera | 32 | 0.00748 | 11C7 |
| Jak-stat cascade | Celera | 37 | 0.00748 | 11C7 |
| purine metabolism | Celera | 56 | 0.00776 | 11 C7 |
| small molecule transport | Celera | 60 | 0.00816 | 11 C7 |
| cell adhesion-mediated signalling | Celera | 123 | 0.013 | IgG |
| cell structure | Celera | 261 | 0.0155 | 11C7 |
| exocytosis | Celera | 133 | 0.0155 | 11C7 |
| alanine and aspartate metabolism | KEGG | 11 | 0.0161 | 11C7 |
| miscellaneous | Celera | 24 | 0.0176 | 11C7 |
| PDGF signalling pathway | Celera public | 16 | 0.0194 | 11C7 |
| Alzheimer disease-presenilin pathway | Celera public | 32 | 0.0279 | 11C7 |
| signalling:Rattus norvegicus: disease:rheumatoid arthritis:gh signalling pathway | Pathart | 4 | 0.0285 | IgG |

(continued)

GSEA performed on T1-7 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| pentose phosphate pathway | KEGG | 13 | 0.0293 | 11C7 |
| signalling:Rattus norvegicus: disease:alzheimers: amyloidbeta-peptide signalling pathway | Pathart | 20 | 0.0332 | 11C7 |
| regulated exocytosis | Celera | 50 | 0.038 | 11C7 |
| blood clotting | Celera | 25 | 0.038 | IgG |
| Huntington's disease | KEGG | 23 | 0.0443 | 11 C7 |
| purine metabolism | KEGG | 38 | 0.0443 | 11C7 |
| amino acid biosynthesis | Celera | 33 | 0.0485 | 11 C7 |

TABLE 19

GSEA performed on T1-7 dataset. Pathwavs with enriched genes either in IgG- or 11C7- treated after two weeks of treatment (q<0.05)

| Pathway Name | Pathway Source | Probesets | pvalue | qvalue | Enrichment Direction |
|---|---|---|---|---|---|
| ion transport | Celera | 258 | 0.000252 | 0.0406 | IgG |
| growth factor homeostasis | Celera | 7 | 0.000278 | 0.0406 | 11 C7 |
| mRNA transcription termination | Celera | 7 | 0.000308 | 0.0406 | 11C7 |

TABLE 20

GSEA performed on L1-5 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| expressed probesets that are unassigned to a pathway | gsea | 6794 | NA | NA |
| immunity and defence | Celera | 393 | 3.44E-40 | 11C7 |
| signal transduction | Celera | 1336 | 5.02E-15 | 11 C7 |
| cell communication | Celera | 350 | 5.14E-15 | 11C7 |
| ribosome | KEGG | 51 | 3.23E-12 | 11C7 |
| protein metabolism and modification | Celera | 1358 | 4.63E-12 | 11C7 |
| Jak-stat cascade | Celera | 38 | 5.02E-09 | 11 C7 |
| macrophage-mediated immunity | Celera | 52 | 5.02E-09 | 11 C7 |

(continued)

GSEA performed on L1-5 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| integrin signalling pathway | Celera public | 48 | 5.02E-09 | 11C7 |
| mesoderm development | Celera | 161 | 5.02E-09 | 11 C7 |
| synaptic transmission | Celera | 84 | 2.13E-08 | IgG |
| cell structure and motility | Celera | 417 | 2.13E-08 | 11C7 |
| extracellular matrix protein-mediated signalling | Celera | 36 | 2.75E-08 | 11C7 |
| cell surface receptor mediated signal transduction | Celera | 515 | 1.24E-07 | 11 C7 |
| B-cell-and antibody-mediated immunity | Celera | 30 | 1.35E-07 | 11C7 |
| complement and coagulation cascades | KEGG | 17 | 3.46E-07 | 11 C7 |
| cytokine and chemokine mediated signalling pathway | Celera | 58 | 7.40E-07 | 11C7 |
| | Sebastian | 37 | 7.99E-07 | 11C7 |
| granulocyte-mediated immunity | Celera | 18 | 8.20E-07 | 11 C7 |
| blood clotting | Celera | 24 | 8.89E-07 | 11 C7 |
| proteolysis | Celera | 376 | 8.89E-07 | 11 C7 |
| signalling:Rattus norvegicus:disease: atherosclerosis: angiotensin signalling pathway | Pathart | 27 | 1.24E-06 | 11 C7 |
| protein biosynthesis | Celera | 207 | 2.60E-06 | 11 C7 |
| skeletal development | Celera | 29 | 3.58E-06 | 11 C7 |
| apoptosis signalling pathway | Celera public | 46 | 3.59E-06 | 11C7 |
| apoptosis | Celera | 228 | 3.59E-06 | 11 C7 |
| nerve-nerve synaptic transmission | Celera | 26 | 5.35E-06 | IgG |
| complement-mediated immunity | Celera | 15 | 5.53E-06 | 11C7 |
| interferon-mediated immunity | Celera | 29 | 6.32E-06 | 11 C7 |
| developmental processes | Celera | 507 | 1.46E-05 | 11 C7 |
| oncogenesis | Celera | 280 | 1.61 E-05 | 11 C7 |
| other polysaccharide metabolism | Celera | 52 | 2.76E-05 | 11 C7 |
| cell adhesion-mediated signalling | Celera | 120 | 3.07E-05 | 11 C7 |
| T-cell mediated immunity | Celera | 49 | 4.35E-05 | 11 C7 |
| neuronal activities | Celera | 230 | 4.35E-05 | IgG |

(continued)

GSEA performed on L1-5 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| nucleoside, nucleotide and nucleic acid metabolism | Celera | 1255 | 4.43E-05 | 11 C7 |
| cell structure | Celera | 258 | 6.22E-05 | 11 C7 |
| toll receptor signalling pathway | Celera public | 14 | 6.44E-05 | 11C7 |
| ligand-mediated signalling | Celera | 131 | 9.47E-05 | 11C7 |
| signalling:Rattus norvegicus:physiology: growth and differentiation:NGF signalling pathway | Pathart | 33 | 0.000135 | 11C7 |
| signalling:Rattus norvegicus:physiology: growth and differentiation:TGFbeta signalling pathway | Pathart | 15 | 0.000148 | 11C7 |
| coagulation: procoagulation | Sebastian | 24 | 0.000163 | 11 C7 |
| angiogenesis | Celera public | 57 | 0.000163 | 11C7 |
| mapk signalling pathway | KEGG | 90 | 0.000246 | 11 C7 |
| TGF-beta signalling pathway | Celera public | 29 | 0.000249 | 11C7 |
| b cell activation | Celera public | 26 | 0.000257 | 11 C7 |
| signalling:Rattus norvegicus: physiologyakeletal development:FGF signalling pathway | Pathart | 20 | 0.000287 | 11C7 |
| protein modification | Celera | 558 | 0.000308 | 11C7 |
| cell adhesion | Celera | 217 | 0.000401 | 11C7 |
| pi3 kinase pathway | Celera public | 25 | 0.000433 | 11 C7 |
| signalling:Rattus norvegicus:disease: obesity:responsive genes | Pathart | 16 | 0.000439 | 11C7 |
| signalling:Rattus norvegicus:disease: atherosclerosis:ldl signalling pathway | Pathart | 15 | 0.00044 | 11C7 |
| inflammation mediated by chemokine and cytokine signalling pathway | Celera public | 46 | 0.000453 | 11C7 |

(continued)

GSEA performed on L1-5 dataset. Pathways with enriched genes either in IgG- or 11C7- treated after one week of treatment (q<0.05)

| Pathway Name | Pathway Source | Probesets | qvalue | Enrichment Direction |
|---|---|---|---|---|
| toll-like receptor signalling pathway | KEGG | 27 | 0.000552 | 11C7 |
| hematopoesis | Celera | 48 | 0.00056 | 11 C7 |
| signalling:Rattus norvegicus:physiology: apoptosis:TGF beta induced apoptosis | Pathart | 20 | 0.000686 | 11C7 |
| Jak-stat signalling pathway | Celera public | 6 | 0.000768 | 11C7 |
| mRNA transcription regulation | Celera | 480 | 0.000768 | 11C7 |
| natural killer cell mediated immunity | Celera | 11 | 0.00086 | 11C7 |
| growth factor homeostasis | Celera | 7 | 0.00115 | 11C7 |
| signalling:Rattus norvegicus:physiology: cell adhesion:integrin signalling pathway | Pathart | 18 | 0.00128 | 11C7 |
| TGF-beta signalling pathway | KEGG | 25 | 0.0015 | 11C7 |
| signalling:Rattus norvegicus:disease: diabetes type ii:il1b signalling pathway | Pathart | 9 | 0.0015 | 11C7 |
| signalling:Rattus norvegicus:disease: Parkinsons disease:dopamine signalling pathway | Pathart | 26 | 0.00156 | 11C7 |
| inhibition of apoptosis | Celera | 54 | 0.00159 | 11C7 |
| mRNA transcription | Celera | 660 | 0.00173 | 11C7 |
| signalling:Rattus norvegicus:physiology: others:fcer1 signalling pathway | Pathart | 12 | 0.00173 | 11C7 |
| coagulation: anticoagulation | Sebastian | 13 | 0.00203 | 11C7 |
| signalling:Rattus norvegicus:disease: alzheimers:amyloidbeta- peptide signalling pathway | Pathart | 19 | 0.00254 | 11C7 |

(continued)

| Pathway Name | Pathway Source | Probesets | q value | Enrichment Direction |
|---|---|---|---|---|
| cell motility | Celera | 94 | 0.00275 | 11C7 |
| coagulation: anticoagulation: anticoagulation | Sebastian | 6 | 0.00277 | 11C7 |
| signalling:Rattus norvegicus:disease: atherosclerosisanf signalling pathway | Pathart | 10 | 0.00322 | 11 C7 |
| Huntington's disease | KEGG | 24 | 0.00343 | 11C7 |
| cation transport | Celera | 197 | 0.00343 | IgG |
| NF-kappaB cascade | Celera | 29 | 0.00389 | 11 C7 |
| lipid, fatty acid and steroid metabolism | Celera | 341 | 0.00389 | 11C7 |
| Alzheimer disease-presenilin pathway | Celera public | 31 | 0.00433 | 11C7 |
| blood coagulation | Celera public | 7 | 0.00433 | 11C7 |
| protein glycosylation | Celera | 83 | 0.00443 | 11C7 |
| ion transport | Celera | 257 | 0.00464 | IgG |
| induction of apoptosis | Celera | 97 | 0.00513 | 11 C7 |
| endocytosis | Celera | 161 | 0.00541 | 11C7 |
| general vesicle transport | Celera | 178 | 0.00548 | 11 C7 |
| signalling:Rattus norvegicus:disease: atherosclerosis:insulin signalling pathway | Pathart | 7 | 0.00586 | 11C7 |
| p53 pathway | Celera public | 11 | 0.00592 | 11C7 |
| apoptosis | KEGG | 31 | 0.0064 | 11C7 |
| fas signalling pathway | Celera public | 15 | 0.0066 | 11C7 |
| intracellular protein traffic | Celera | 611 | 0.00718 | 11C7 |
| signalling:Rattus norvegicus:physiology: growth and differentiation:PDGF signalling pathway | Pathart | 8 | 0.00718 | 11C7 |
| intracellular signalling, cascade | Celera | 420 | 0.00882 | 11C7 |
| signalling:Rattus norvegicus:disease: obesity:leptin signalling pathway | Pathart | 24 | 0.00882 | 11C7 |
| other immune and defence | Celera | 29 | 0.00886 | 11C7 |
| axon guidance mediated by slit-robo | Celera public | 3 | 0.00909 | 11C7 |

(continued)

| Pathway Name | Pathway Source | Probesets | q value | Enrichment Direction |
|---|---|---|---|---|
| signalling:Rattus norvegicus:disease: diabetes type ii:ffa signalling pathway | Pathart | 13 | 0.00932 | 11C7 |
| signalling:Rattus norvegicus:physiology: growth and differentiation:FGF2 signalling pathway | Pathart | 11 | 0.00932 | 11C7 |
| neurotransmitter release | Celera | 19 | 0.00962 | IgG |
| stress response | Celera | 65 | 0.00985 | 11 C7 |
| signalling:Rattus norvegicus:disease: atherosclerosis:il1 signalling pathway | Pathart | 10 | 0.0102 | 11C7 |
| signalling:Rattus norvegicus:physiology: apoptosis:NGF signalling pathway | Pathart | 17 | 0.0112 | 11C7 |
| signalling:Rattus norvegicus:physiology: apoptosis:FGF signalling | Pathart | 8 | 0.0112 | 11C7 |
| Pathway Name | Pathway Source | Probesets | qvalue | Enrichment Direction |
| pathway | | | | |
| oxidative stress response | Celera public | 13 | 0.0136 | 11 C7 |
| protein disulfide-isomerase reaction | Celera | 6 | 0.0136 | 11 C7 |
| Parkinson disease | Celera public | 48 | 0.0143 | 11C7 |
| signalling:Rattus norvegicus:disease: alzheimers:igf1 signalling pathway | Pathart | 4 | 0.0152 | 11C7 |
| glycolysis / gluconeogenesis | KEGG | 27 | 0.0165 | 11C7 |
| T-cell activation | Celera public | 29 | 0.0165 | 11C7 |
| other transport | Celera | 26 | 0.0169 | 11C7 |
| oncogene | Celera | 54 | 0.0169 | 11C7 |
| prostaglandin and leukotriene metabolism | KEGG | 7 | 0.0169 | 11C7 |
| PDGF signalling pathway | Celera public | 15 | 0.0173 | 11C7 |
| mRNA splicing | Celera | 107 | 0.0177 | 11C7 |

(continued)

| Pathway Name | Pathway Source | Probesets | qvalue | Enrichment Direction |
|---|---|---|---|---|
| signalling:Rattus norvegicus:disease: obesity:cntf signalling pathway | Pathart | 6 | 0.0179 | 11C7 |
| cytokine/chemokine mediated immunity | Celera | 23 | 0.0203 | 11C7 |
| carbohydrate metabolism | Celera | 215 | 0.0203 | 11C7 |
| porphyrin and chlorophyll metabolism | KEGG | 7 | 0.0203 | 11C7 |
| prion disease | KEGG | 6 | 0.0219 | 11C7 |
| n-glycan biosynthesis | KEGG | 8 | 0.0231 | 11C7 |
| signalling:Rattus norvegicus:disease: atherosclerosis:linoleic acid signalling pathway | Pathart | 3 | 0.0234 | 11 C7 |
| signalling:Rattus norvegicus:disease: atherosclerosis:aif mediated pathway | Pathart | 5 | 0.0236 | 11C7 |
| coagulation: procoagulation: jackson lab bleeding mice | Sebastian | 7 | 0.027 | 11C7 |
| other apoptosis | Celera | 9 | 0.027 | 11C7 |
| Huntington disease | Celera public | 44 | 0.0277 | 11C7 |
| signalling:Rattus norvegicus:disease: atherosclerosis:PDGF signalling pathway | Pathart | 8 | 0.0278 | 11C7 |
| nicotinic acetylcholine receptor signalling pathway | Celera public | 23 | 0.0296 | 11C7 |
| vitamin/cofactor transport | Celera | 9 | 0.0296 | 11 C7 |
| wnt signalling pathway | KEGG | 58 | 0.0303 | 11C7 |
| signalling:Rattus norvegicus:disease: alzheimers:hydrogen peroxide signalling pathway | Pathart | 8 | 0.0319 | 11C7 |
| other oncogenesis | Celera | 44 | 0.032 | 11 C7 |
| cell cycle | Celera public | 5 | 0.032 | 11C7 |
| signalling:Rattus norvegicus:disease: rheumatoid | Pathart | 2 | 0.0323 | 11C7 |

(continued)

| Pathway Name | Pathway Source | Probesets | qvalue | Enrichment Direction |
|---|---|---|---|---|
| arthritis:interleukin signalling pathway | | | | |
| cell proliferation and differentiation | Celera | 138 | 0.0334 | 11C7 |
| urea cycle and metabolism of amino groups | KEGG | 11 | 0.0368 | 11C7 |
| other receptor mediated signalling pathway | Celera | 33 | 0.0369 | 11C7 |
| peptidoglycan biosynthesis | KEGG | 3 | 0.0377 | 11 C7 |
| lipid and fatty acid transport | Celera | 51 | 0.0404 | 11 C7 |
| dentatorubropallidoluysian atrophy (drpla) | KEGG | 8 | 0.0404 | 11C7 |
| oxidative phosphorylation | Celera | 56 | 0.0404 | IgG |
| signalling:Rattus norvegicus:physiology: inflammation:il1 signalling pathway | Pathart | 2 | 0.0404 | 11 C7 |
| other protein metabolism | Celera | 27 | 0.0404 | IgG |
| EGF receptor signalling pathway | Celera | 36 | 0.0405 | 11C7 |
| signalling:Rattus norvegicus:disease: diabetes type ii:hexosamine mediated pathway | Pathart public | 16 | 0.0423 | 11C7 |
| gamma-hexachlorocyclohexane degradation | KEGG | 4 | 0.0429 | 11 C7 |
| metabotropic glutamate receptor group ii pathway | Celera public | 9 | 0.0431 | 11C7 |
| phagocytosis | Celera | 16 | 0.0443 | 11C7 |
| signalling:Rattus norvegicus:physiology: apoptosis:wnt signalling pathway | Pathart | 7 | 0.0458 | 11C7 |
| signalling:Rattus norvegicus:disease: atherosclerosis:ifngamma signalling pathway | Pathart | 2 | 0.0458 | 11C7 |
| receptor protein serine/ threonine kinase signalling pathway | Celera | 28 | 0.046 | 11C7 |
| hypoxia response via hif activation | Celera public | 13 | 0.0465 | 11C7 |

(continued)

| Pathway Name | Pathway Source | Probesets | qvalue | Enrichment Direction |
|---|---|---|---|---|
| arginine and proline metabolism | KEGG | 20 | 0.0465 | 11C7 |
| glycolysis | Celera | 32 | 0.0465 | 11 C7 |
| signalling:Rattus norvegicus:disease: alzheimers:NGF signalling pathway | Pathart | 8 | 0.0473 | 11C7 |
| signalling:Rattus norvegicus:disease: alzheimers:icam1 signalling pathway | Pathart | 5 | 0.0473 | 11C7 |

## TABLE 21

GSEA performed on L1-5 dataset. Pathways with enriched genes either in IgG- or 11C7- treated after two weeks of treatment (q<0.05)

| Pathway Name | Pathway Source | Probesets | qvalue | Enrichment Direction |
|---|---|---|---|---|
| immunity and defence | Celera | 393 | 0 | IgG |
| expressed probesets that are unassigned to a pathway | gsea | 6794 | NA | NA |
| cell communication | Celera | 350 | 5.49E-11 | IgG |
| synaptic transmission | Celera | 84 | 1.15E-10 | 11C7 |
| protein metabolism and modification | Celera | 1358 | 1.92E-10 | IgG |
| extracellular matrix protein-mediated signalling | Celera | 36 | 1.08E-09 | IgG |
| neuronal activities | Celera | 230 | 1.89E-09 | 11 C7 |
| signal transduction | Celera | 1336 | 2.28E-08 | IgG |
| B-cell- and antibody-mediated immunity | Celera | 30 | 5.37E-08 | IgG |
| macrophage-mediated immunity | Celera | 52 | 5.72E-08 | IgG |
| T-cell mediated immunity | Celera | 49 | 1.66E-07 | IgG |
| blood clotting | Celera | 24 | 6.46E-07 | IgG |
| integrin signalling pathway | Celera | 48 | 8.72E-07 | IgG |
| complement and coagulation cascades | public KEGG | 17 | 8.79E-07 | IgG |
| oncogene | Celera | 54 | 2.21 E-06 | IgG |

(continued)

GSEA performed on L1-5 dataset. Pathways with enriched genes either in IgG- or 11C7- treated after two weeks of treatment (q<0.05)

| Pathway Name | Pathway Source | Probesets | qvalue | Enrichment Direction |
|---|---|---|---|---|
| cation transport | Celera | 197 | 4.01E-06 | 11C7 |
| oncogenesis | Celera | 280 | 6.38E-06 | IgG |
| ion transport | Celera | 257 | 6.92E-06 | 11C7 |
| proteolysis | Celera | 376 | 1.24E-05 | IgG |
| | Sebastian | 37 | 2.01 E-05 | IgG |
| cytokine and chemokine mediated signalling pathway | Celera | 58 | 2.40E-05 | IgG |
| neurotransmitter release | Celera | 19 | 2.40E-05 | 11C7 |
| protein modification | Celera | 558 | 8.85E-05 | IgG |
| apoptosis | Celera | 228 | 8.85E-05 | IgG |
| cell adhesion-mediated signalling | Celera | 120 | 9.26E-05 | IgG |
| neuroactive ligand-receptor interaction | KEGG | 52 | 0.000111 | 11C7 |
| mhcii-mediated immunity | Celera | 10 | 0.000115 | IgG |
| other polysaccharide metabolism | Celera | 52 | 0.000144 | IgG |
| nucleoside, nucleotide and nucleic acid metabolism | Celera | 1255 | 0.000191 | IgG |
| nerve-nerve synaptic transmission | Celera | 26 | 0.000245 | 11 C7 |
| complement-mediated immunity | Celera | 15 | 0.000245 | IgG |
| ionotropic glutamate receptor pathway | Celera public | 24 | 0.000245 | 11C7 |
| T-cell activation | Celera public | 29 | 0.000245 | IgG |
| ligand-mediated signalling | Celera | 131 | 0.000245 | IgG |
| skeletal development | Celera | 29 | 0.000282 | IgG |
| mesoderm development | Celera | 161 | 0.000296 | IgG |
| apoptosis signalling pathway | Celera | 46 | 0.000296 | IgG |
| inflammation mediated by chemokine and cytokine | public Celera | 46 | 0.000304 | IgG |

(continued)

GSEA performed on L1-5 dataset. Pathways with enriched genes either in IgG- or 11C7- treated after two weeks of treatment (q<0.05)

| Pathway Name | Pathway Source | Probesets | qvalue | Enrichment Direction |
|---|---|---|---|---|
| signalling pathway | public | | | |
| growth factor homeostasis | Celera | 7 | 0.000316 | IgG |
| protein glycosylation | Celera | 83 | 0.000341 | IgG |
| p53 pathway | Celera public | 11 | 0.000393 | IgG |
| Pathway Name | Pathwav Source | Probesets | qvalue | Enrichment Direction |
| inhibition of apoptosis | Celera | 54 | 0.000439 | IgG |
| toll receptor signalling pathway | Celera public | 14 | 0.000465 | IgG |
| Jak-stat cascade | Celera | 38 | 0.000533 | IgG |
| NF-kappaB cascade | Celera | 29 | 0.000538 | IgG |
| b cell activation | Celera public | 26 | 0.000611 | IgG |
| signalling:Rattus norvegicus:physiology: cell adhesion:integrin signalling pathway | Pathart | 18 | 0.000633 | IgG |
| cell adhesion | Celera | 217 | 0.000905 | IgG |
| nicotinate and nicotinamide metabolism | KEGG | 16 | 0.000962 | IgG |
| insulin-igf pathway-protein kinase b signalling cascade | Celera public | 18 | 0.00119 | IgG |
| oxidative phosphorylation | KEGG | 65 | 0.00139 | 11C7 |
| cell structure and motility | Celera | 417 | 0.00145 | IgG |
| oxidative phosphorylation | Celera | 56 | 0.00151 | 11C7 |
| pre-mRNA processing | Celera | 162 | 0.00158 | IgG |
| coagulation: anticoagulation | Sebastian | 13 | 0.00192 | IgG |
| cell motility | Celera | 94 | 0.00256 | IgG |
| coagulation: procoagulation | Sebastian | 24 | 0.00375 | IgG |
| protein disulfide-isomerase reaction | Celera | 6 | 0.00375 | IgG |
| toll-like receptor signalling pathway | KEGG | 27 | 0.00421 | IgG |

(continued)

| Pathway Name | Pathway Source | Probesets | qvalue | Enrichment Direction |
|---|---|---|---|---|
| granulocyte-mediated immunity | Celera | 18 | 0.00473 | IgG |
| apoptosis | KEGG | 31 | 0.00588 | IgG |
| signalling:Rattus norvegicus:disease: rheumatoid arthritis:gh signalling pathway | Pathart | 4 | 0.00611 | IgG |
| signalling:Rattus norvegicus:disease: atherosclerosis: angiotensin signalling pathway | Pathart | 27 | 0.00652 | IgG |
| transport | Celera | 464 | 0.0069 | 11 C7 |
| signalling:Rattus norvegicus:physiology: others:fcer1 signalling pathway | Pathart | 12 | 0.0071 | IgG |
| n-glycan biosynthesis | KEGG | 8 | 0.00736 | IgG |
| signalling:Rattus norvegicus:disease: atherosclerosis:tnf signalling pathway | Pathart | 10 | 0.00752 | IgG |
| other apoptosis | Celera | 9 | 0.00783 | IgG |
| metabotropic glutamate receptor group iii pathway | Celera public | 19 | 0.00783 | 11C7 |
| hypoxia response via hif activation | Celera public | 13 | 0.00806 | IgG |
| mRNA transcription regulation | Celera | 480 | 0.00921 | IgG |
| signalling:Rattus norvegicus:physiology: growth and differentiation:NGF signalling pathway | Pathart | 33 | 0.00998 | IgG |
| TGF-beta signalling pathway | Celera public | 29 | 0.0112 | IgG |
| Parkinson's disease | KEGG | 16 | 0.0112 | 11C7 |
| angiogenesis | Celera | 57 | 0.0114 | IgG |

(continued)

| Pathway Name | Pathway Source | Probesets | qvalue | Enrichment Direction |
|---|---|---|---|---|
| signalling:Rattus norvegicus:disease: diabetes type ii:il1b signalling pathway | public Pathart | 9 | 0.0117 | IgG |
| electron transport | Celera | 89 | 0.0131 | 11C7 |
| insulin-igf pathway-mitogen activated protein kinase kinase-map kinase cascade | Celera | 14 | 0.0133 | IgG |
| signalling:Rattus norvegicus:disease: atherosclerosis:ldl signalling pathway | public Pathart | 15 | 0.0136 | IgG |
| natural killer cell mediated immunity | Celera | 11 | 0.0138 | IgG |
| axon guidance mediated by slit-robo | Celera | 3 | 0.0139 | IgG |
| monosaccharide metabolism | public Celera | 27 | 0.0141 | IgG |
| starch and sucrose metabolism | KEGG | 20 | 0.0141 | IgG |
| stress response | Celera | 65 | 0.0141 | IgG |
| lipid, fatty acid and steroid metabolism | Celera | 341 | 0.0142 | IgG |
| blood coagulation | Celera | 7 | 0.0144 | IgG |
| inositol phosphate metabolism | public KEGG | 22 | 0.0144 | IgG |
| extracellular transport and import | Celera | 35 | 0.0144 | 11C7 |
| mRNA splicing | Celera | 107 | 0.0152 | IgG |
| signalling:Rattus norvegicus:disease: obesity:responsive genes | Pathart | 16 | 0.0152 | IgG |
| pi3 kinase pathway | Celera | 25 | 0.016 | IgG |
| signalling:Rattus norvegicus:disease: alzheimers: amyloidbeta-peptide signalling pathway | public Pathart | 19 | 0.0165 | IgG |

(continued)

| Pathway Name | Pathway Source | Probesets | qvalue | Enrichment Direction |
|---|---|---|---|---|
| receptor protein serine/ threonine kinase signalling pathway | Celera | 28 | 0.0165 | IgG |
| MAPKKK cascade | Celera | 111 | 0.0178 | IgG |
| fas signalling pathway | Celera public | 15 | 0.0179 | IgG |
| glycosphingolipid metabolism | KEGG | 9 | 0.0188 | IgG |
| ribosome | KEGG | 51 | 0.02 | IgG |
| intracellular signalling cascade | Celera | 420 | 0.023 | IgG |
| protein biosynthesis | Celera | 207 | 0.0232 | IgG |
| interleukin signalling pathway | Celera public | 23 | 0.0249 | IgG |
| coagulation: anticoagulation: anticoagulation | Sebastian | 6 | 0.0253 | IgG |
| signalling:Rattus norvegicus:physiology: apoptosis:TGF beta induced apoptosis | Pathart | 20 | 0.0256 | IgG |
| other immune and defence | Celera | 29 | 0.0266 | IgG |
| signalling:Rattus norvegicus:disease: obesity:leptin signalling pathway | Pathart | 24 | 0.0273 | IgG |
| bile acid biosynthesis | KEGG | 10 | 0.0277 | IgG |
| carbohydrate metabolism | Celera | 215 | 0.0288 | IgG |
| signalling:Rattus norvegicus:disease: atherosclerosis:insulin signalling pathway | Pathart | 7 | 0.0327 | IgG |
| induction of apoptosis | Celera | 97 | 0.0332 | IgG |
| benzoate degradation via coa ligation | KEGG | 19 | 0.0334 | IgG |
| phagocytosis | Celera | 16 | 0.0337 | IgG |
| cell surface receptor mediated signal transduction | Celera | 515 | 0.0351 | IgG |

(continued)

| Pathway Name | Pathway Source | Probesets | qvalue | Enrichment Direction |
|---|---|---|---|---|
| signalling:Rattus norvegicus:disease: atherosclerosis:linoleic acid signalling pathway | Pathart | 3 | 0.0356 | IgG |
| coagulation: procoagulation: possible positive modulators platelet aggr. | Sebastian | 3 | 0.0387 | IgG |
| coagulation: procoagulation: synthesis and transport | Sebastian | 3 | 0.0397 | IgG |
| signalling:Rattus norvegicus:physiology: inflammation:il1 signalling pathway | Pathart | 2 | 0.0403 | IgG |
| phospholipid metabolism | Celera | 52 | 0.0403 | IgG |
| signalling:Rattus norvegicus:physiology: growth and differentiation:akt mediated pathway | Pathart | 4 | 0.0456 | IgG |

EXAMPLE 4

PATHWAYS WITH SIGNIFICANT GENE SET ENRICHMENT IN THREE OR MORE TISSUES

TABLE 22. Pathways with significant gene set enrichment in three or more tissue.

| Pathway Name | Pathway Source | Tissue | Enrichment Direction |
|---|---|---|---|
| Apoptosis | Celera | T8 1 wk | 11 C7 |
|  | KEGG | T8 1 wk | 11C7 |
|  | Celera | L 1-5 1 wk | 11C7 |
|  | Celera | L1-5 2 wk | IgG |
| apoptosis signalling pathway | Celera public | T8 1 wk | 11C7 |
|  | Celera public | L 1-5 1 wk | 11C7 |
|  | Celera public | L1-5 2 wk | IgG |
| B-cell- and antibody-mediated immunity | Celera | T8 1 wk | 11C7 |
|  | Celera | L 1-5 1 wk | 11C7 |
|  | Celera | L1-5 2 wk | IgG |
| blood clotting | Celera | L 1-5 1 wk | 11C7 |
|  | Celera | L1-5 2 wk | IgG |
|  | Celera public | T8 2 wk | IgG |

(continued)

| Pathway Name | Pathway Source | Tissue | Enrichment Direction |
|---|---|---|---|
| complement and coagulation cascades | KEGG | T8 2 wk | IgG |
| | KEGG | L 1-5 1 wk | 11C7 |
| | KEGG | L1-5 2 wk | IgG |
| cytokine and chemokine mediated signalling pathway | Celera | T8 1 wk | 11C7 |
| | Celera | L 1-5 1 wk | 11C7 |
| | Celera | L1-5 2 wk | IgG |
| extracellular matrix protein-mediated signalling | Celera | T1-7 1 wk | IgG |
| | Celera | L 1-5 1 wk | 11C7 |
| | Celera | L1-5 2 wk | IgG |
| growth factor homeostasis | Celera | T1-7 1 wk | IgG |
| | Celera | L 1-5 1 wk | 11C7 |
| | Celera | L1-5 2 wk | IgG |
| immunity and defence | Celera | Blood 2wk | IgG |
| | Celera | T8 1 wk | 11C7 |
| | Celera | L 1-5 1 wk | 11C7 |
| | Celera | L1-5 2 wk | IgG |
| interferon-mediated immunity | Celera | Blood 1 wk | 11C7 |
| | Celera | T8 1 wk | 11C7 |
| | Celera | L 1-5 1 wk | 11C7 |
| intracellular protein traffic | Celera | Blood 2wk | IgG |
| | Celera | Blood 1 wk | 11C7 |
| | Celera | T8 1 wk | 11C7 |
| Jak-stat cascade | Celera | T8 1 wk | 11C7 |
| | Celera | L 1-5 1 wk | 11C7 |
| | Celera | L1-5 2 wk | IgG |
| | Celera public | T8 1 wk | 11C7 |
| | Celera public | L 1-5 1 wk | 11C7 |
| macrophage-mediated immunity | Celera | T8 1 wk | 11C7 |
| | Celera | L 1-5 1 wk | 11C7 |
| | Celera | L1-5 2 wk | IgG |
| nerve-nerve synaptic transmission | Celera | T8 2 wk | 11C7 |
| | Celera | L 1-5 1 wk | IgG |
| | Celera | L1-5 2 wk | 11C7 |
| neuronal activities | Celera | T8 1 wk | IgG |
| | Celera | T8 2 wk | 11C7 |
| | Celera | L 1-5 1 wk | IgG |
| | Celera | L1-5 2 wk | 11C7 |
| nucleoside, nucleotide and nucleic acid metabolism | Celera | Blood 2wk | IgG |
| | Celera | T8 1 wk | 11C7 |
| | Celera | T8 2 wk | IgG |
| | Celera | L 1-5 1 wk | 11C7 |
| | Celera | L1-5 2 wk | IgG |
| oncogenesis | Celera | Blood 2wk | IgG |
| | Celera | L 1-5 1 wk | 11C7 |
| | Celera | L1-5 2 wk | IgG |
| oxidative phosphorylation | KEGG | T8 2 wk | 11C7 |
| | Celera | T8 2 wk | 11C7 |
| | KEGG | L1-5 2 wk | 11C7 |

(continued)

| Pathway Name | Pathway Source | Tissue | Enrichment Direction |
|---|---|---|---|
| | KEGG | MCx 1 wk | IgG |
| protein metabolism and modification | Celera | Blood 2wk | IgG |
| | Celera | T8 1 wk | 11C7 |
| | Celera | L 1-5 1 wk | 11C7 |
| | Celera | L1-5 2 wk | IgG |
| protein modification | Celera | Blood 2wk | IgG |
| | Celera | T8 1 wk | 11C7 |
| | Celera | L 1-5 1 wk | 11C7 |
| | Celera | L1-5 2 wk | IgG |
| Proteolysis | Celera | T8 1 wk | 11C7 |
| | Celera | L 1-5 1 wk | 11C7 |
| | Celera | L1-5 2 wk | IgG |
| synaptic transmission | Celera | T8 2 wk | 11C7 |
| | Celera | L 1-5 1 wk | IgG |
| | Celera | L1-5 2 wk | 11C7 |
| T-cell mediated immunity | Celera | T8 1 wk | 11C7 |
| | Celera | L 1-5 1 wk | 11C7 |
| | Celera | L1-5 2 wk | IgG |
| toll receptor signalling pathway | Celera public | L 1-5 1 wk | 11C7 |
| | Celera public | L1-5 2 wk | IgG |
| | KEGG | T8 1 wk | 11C7 |
| | KEGG | L 1-5 1 wk | 11C7 |

EXAMPLE 5

AXON GUIDANCE AND GROWTH FACTOR PATHWAYS IDENTIFIED BY GSEA AFFECTED BY ANTI-NOGO A ANTIBODY TREATMENT

TABLE 23. Axon guidance and growth factor pathways identified by GSEA affected by anti-Nogo-A antibody treatment.

| Pathway Name | Pathway Source | Tissue | Enrichment Direction |
|---|---|---|---|
| Axon guidance mediated by slit-robo | Celera public | T8 1 wk | 11C7 |
| | | L 1-5 1 wk | 11C7 |
| | | T1-7 1 wk | 11C7 |
| EGF receptor signalling pathway | Celera public | Motor cx 2 wk | IgG |
| FGF signalling pathway | Celera public | Motor cx 2 wk | IgG |
| NGF signalling pathway | Pathart | Motor Cx 2 wk | IgG |

EXAMPLE 6

PATHWAYS AND GENE GROUPS COORDINATELY AFFECTED BY NOGO A KNOCK OUT IN PURE SV129 AND BL6 MOUSE LINES AND ANTI-NOGO A ANTIBODY TREATMENT IN THE RAT SPINAL CORD INJURY MODEL

Nogo-A (200 kDa, 1163 aa) differs from Nogo-B (55 kDa, 357 aa) by the insertion of a large 787 aa exon (exon 3). A Nogo-A knock-out mouse was generated by homologous recombination as described by Simonen et al. (2003). The chimeric Nogo-A knock-out mice were backcrossed to either Sv129 mice or BL/6 mice for at least 10 generations. The speed congenics strain marker analysis (Markel et al., 1997) was used during backcrossing. Speed congenic breeding, or marker-assisted congenic production, uses microsatellite markers to follow the inheritance of the chromosomal segments of each strain. Optimal breeder mice are selected by the highest level of markers for each strain. The mice used in the present study had a 100% pure C57BL/6 background according to their marker profile, and a 99% pure background for the 129Xl/SvJ strain.

Lumbar spinal cords from three naive, non-injured, wild-type, and knock-out male mice (3 months of age) per strain and genotype were dissected and immediately frozen in liquid nitrogen. For lesion microarray experiment, five female mice (6—7 weeks old) of each genotype and strain underwent a lesion of the spinal cord with the help of fine iridectomy scissors to produce a bilateral lesion of the dorsal and the dorsolateral funiculi and the dorsal horn. Six days after the lesion, a Basso Mouse Scale behavioral analysis for open-field locomotion was performed and four of the five mice per category with the most similar score were selectedt for microarray analysis. One week after the lesion, 1 cm of the spinal cord was dissected with the lesion site in the middle and immediately froze it in liquid nitrogen. For probe preparation, procedures described in the Affymetrix (Santa Clara, CA) GeneChip Analysis manual were followed. Biotinylated cRNA was hybridized onto Affymetrix Mouse Genome 430 2.0 arrays, which represent 45,000 probe sets, in the Affymetrix fluidics station 450, and the chips were then scanned with the Affymetrix Scanner 3000. Each chip was used for hybridization with cRNA isolated from one spinal cord sample from a single animal in a total number of 28 samples. Results were subsequently analyzed using the Affymetrix Microarray Suite 5, followed by the Genespring 7.2 (Silicon Genetics, Redwood City, CA). To identify genes that are differentially expressed in the spinal cords of Sv129 and BL/6 mice of naive and knock-out spinal cords of injured and non-injured animals 1 week after a spinal cord lesion, a statistical filter (ANOVA $p < 0.05$) and fold change thresholds (>1.2/<0.66 or 2/<0.5) were applied following a prefiltering for present calls

Pathways and gene groups commonly affected one week after spinal cord injury in knock-out Sv129 mice and BL/6 mice and in the rat SCI model were identified by comparing the differentially expressed genes identified in two way comparisons between the knock-out and naïve animals and in the rat SCI model, between the control (IgG) —treated and 11C7 anti-Nogo A antibody —treated animals.

113 commonly affected gene groups were identified. They are listed in Table 24.

| TABLE 24 |
|---|
| 113 PATHWAYS AND GENE GROUPS COORDINATELY AFFECTED BY NOGO A KNOCK OUT IN PURE SV129 AND BL6 MOUSE LINES AND ANTI-NOGO A ANTIBODY TREATMENT IN THE RAT SPINAL CORD INJURY MODEL |

| | |
|---|---|
| **A disintegrin and metalloprotease domain** | |
| 1387351_at | a disintegrin and metalloprotease domain 10 |
| 1424798_a_at | a disintegrin and metalloprotease domain 5 |
| 1425170_a_at | a disintegrin and metalloproteinase domain 15 (metargidin) |
| 1367910_at (ADAMTS-1) | a disintegrin and metalloproteinase with thrombospondin motifs 1 |
| 1441841_at | a disintegrin-like and metalloprotease (reprolysin type) with thrombospondin type 1 motif, 16 |
| 1452595_at | a disintegrin-like and metalloprotease (reprolysin type) with thrombospondin type 1 motif, 4 |
| **Actin-related** | |
| 1398588_at | actin related protein 2/3 complex, subunit 1B |
| 1423589_at | actin related protein 2/3 complex, subunit 4 |
| 1419009_at | actin-like 7a |
| **Adenylate cyclase/kinase** | |
| 1418098_at | adenylate cyclase 4 |
| 1395726_at | adenylate kinase 3 |
| 1458812_at | adenylate kinase 3 alpha-like |

(continued)

| Adenylate cyclase/kinase | |
| --- | --- |
| 1421830_at | adenylate kinase 4 |
| **Adrenergic receptors** | |
| 1380719_at | adrenergic receptor, alpha 1b |
| 1422335_at | adrenergic receptor, alpha 2c |
| 1368574_at | adrenergic, alpha 1B, receptor |
| **Amyloid beta (A4) precursor** | |
| 1435857_s_at | amyloid beta (A4) precursor-like protein 1 |
| 1383096_at | amyloid beta (A4) precursor-like protein 2 |
| **Ankyrin** | |
| 1459317_at | ankyrin 2, brain |
| 1384347_at | ankyrin 3 (G) |
| 1446319_at | ankyrin repeat and SOCS box-containing protein 7 |
| **Annexin** | |
| 1419091_a_at | annexin A2 |
| 1367974_at | Annexin III (Lipocortin III) |
| 1387673_a_at | annexin VI |
| **Aolipoprotein** | |
| 1419232_a_at | apolipoprotein A-I |
| 1370669_a_at | apolipoprotein B editing complex 1 |
| 1417561_at | apolipoprotein C-I |
| **Aryl/arylsulfatase B** | |
| 1420669_at | aryl hydrocarbon receptor nuclear translocator 2 |
| 1458281_at | arylsulfatase B |
| 1398533_at | arylsulfatase B |
| 1380442_at | arylsulfatase B |
| **ATP-related** | |
| 1426474_at | ATP synthase mitochondrial F1 complex assembly factor 2 |
| 1371817_at | ATP synthase, H+ transporting, mitochondrial F1 complex, gamma polypeptide 1 |
| 1422908_at | ATPase, (Na+)/K+ transporting, beta 4 polypeptide |
| 1386426_at | ATPase, Ca++ transporting, plasma membrane 1 |

(continued)

| ATP-related | |
|---|---|
| 1416769_s_at | ATPase, H+ transporting, V0 subunit B |
| 1435919_at | ATPase, Na+/K+ transporting, alpha 1 polypeptide |
| 1376208_at | ATP-binding cassette, sub-family A (ABC1), member 1 |
| 1394490_at | ATP-binding cassette, sub-family A (ABC1), member 1 |
| 1440370_at | ATP-binding cassette, sub-family A (ABC1), member 13 |
| 1377189_at | ATP-binding cassette, sub-family B (MDR/TAP), member 4 |
| 1368159_at | ATP-binding cassette, sub-family B (MDR/TAP), member 6 |
| 1398265_at | ATP-binding cassette, sub-family C (CFTR/MRP), member 9 |
| 1367594_at | ATP-binding cassette, sub-family D (ALD), member 2 |
| 1398876_at | ATP-binding cassette, sub-family F (GCN20), member 1 |
| **Bcl-2 -related** | |
| 1371828_at | BCL2/adenovirus E1 B 19 kDa-interacting protein 3, nuclear gene for mitochondrial product |
| 1420363_at | Bcl2-interacting killer-like |
| 1426050_at | Bcl2-like |
| 1373733_at | Bcl-2-related ovarian killer protein |
| **Benzodiazepine receptor** | |
| 1453047_at | benzodiazapine receptor, peripheral-like 1 |
| 1392946_at | benzodiazepin receptor |
| **Biglycan** | |
| 1448323_a_at | biglycan |
| 1372713_at | biglycan |
| **BMPs** | |
| 1398270_at | bone morphogenetic protein 2 |
| 1373092_at | bone morphogenetic protein receptor, type 1A |
| 1422872_at | bone morphogenetic protein receptor, type 1 B |
| **Cadherins** | |
| 1419331_at | cadherin 17 |
| 1387259_at | cadherin 2 |
| 1441690_at | cadherin 8 |
| 1368472_at | cadherin EGF LAG seven-pass G-type receptor 3 |

(continued)

| Voltage-dependent calcium channels | |
|---|---|
| 1393587_a_at | calcium channel, voltage-dependent, beta 1 subunit |
| 1393592_at | calcium channel, voltage-dependent, beta 2 subunit |
| 1451811_at | calcium channel, voltage-dependent, gamma subunit 6 |
| **Calmodulin-related** | |
| 1387772_at | Calmodulin 1 (phosphorylase kinase, delta) |
| 1369937_at | Calmodulin 1 (phosphorylase kinase, delta) |
| 1458560_at | calmodulin binding protein 1 |
| 1422814_at | calmodulin binding protein 1 |
| **Carbonic anhydrases** | |
| 1431288_at | carbonic anhydrase 10 |
| 1421307_at | carbonic anhydrase 13 |
| 1388003_at | carbonic anhydrase 4 |
| **Caspases** | |
| 1387858_at | caspase 1 |
| 1367522_at | caspase 11 |
| 1418748_at | caspase 14 |
| 1389479_at | caspase 3 |
| 1374565_at | caspase-8 |
| **CD-antigens** | |
| 1436346_at | CD109 antigen |
| 1419769_at | CD22 antigen |
| 1450513_at | CD33 antigen |
| 1376304_at | CD36 antigen (collagen type I receptor, thrombospondin receptor)-like 2 |
| 1419206_at | CD37 antigen |
| 1398108_at | CD38 antigen |
| 1369628_at | CD4 antigen |
| 1423760_at | CD44 antigen |
| 1390896_at | CD86 antigen |
| **Cell division cycle** | |
| 1382485_at | CD86 antigen |
| 1387436_at | CDC10 (cell division cycle 10, S.cerevisiae, homolog) |

(continued)

| Cell division cycle | |
|---|---|
| 1431291_at | CDC16 cell division cycle 16 homolog (S. cerevisiae) |
| **Cyclin Dependent Kinases** | |
| 1443087_at | CDC23 (cell division cycle 23, yeast, homolog) |
| 1368322_at | CDK104 mRNA |
| **Cell division cycle homologs or associated with** | |
| 1427967_at | CDK5 regulatory subunit associated protein 2 |
| 1393510_at | cell division cycle 2 homolog A (S. pombe) |
| 1390312_at | cell division cycle 42 homolog (S. cerevisiae) |
| **Centaurin** | |
| 1428069_at | cell division cycle associated 7 |
| 1456337_at | centaurin, delta 1 |
| 1387277_at | Centaurin-alpha2 protein |
| **Ceroid-lipofuscinosis, neuronal** | |
| 1380969_at | ceroid-lipofuscinosis, neuronal 2 |
| 1446374_at | ceroid-lipofuscinosis, neuronal 8 |
| **Chemokine receptors and ligands** | |
| 1422294_at | chemokine (C motif) receptor 1 |
| 1421228_at | chemokine (C-C motif) ligand 7 |
| 1422291_at | chemokine (C-C motif) receptor 8 |
| 1421187_at | chemokine (C-C) receptor 2 |
| 1437668_at | chemokine (C-C) receptor-like 1 |
| 1419698_at | chemokine (C-X-C motif) ligand 11 |
| 1374554_at | chemokine (C-X-C motif) ligand 12 |
| 1422812_at | chemokine (C-X-C motif) receptor 6 |
| 1382775_at | chemokine orphan receptor 1 |
| **Coagulation** | |
| 1423285_at | coagulation factor C homolog (Limulus polyphemus) |
| 1427393_at | coagulation factor IX |
| 1370697_a_at | coagulation factor VIII |
| 1389072_at | coagulation factor VIII |
| **Complement pathway** | |
| 1373386_at | complement component 1, q subcomponent, beta polypeptide |
| 1424041_s_at | complement component 1, s subcomponent |
| 1374627_at | complement component 1, s subcomponent |
| 1390901_at | complement component 2 |
| 1374236_at | complement component 2 |
| 1368000_at | complement component 3 |

(continued)

| **Complement pathway** | |
|---|---|
| 1373266_at | complement component 3 |
| 1425823_at | complement component factor h |
| 1388883_at | complement component factor h |
| **Cysteine** | |
| 1374702 at | cysteine string protein |
| 1416717_at | cysteine-rich secretory protein 2 |
| 1427330_at | cysteinyl-tRNA synthetase |
| **Cytochrome oxidases** | |
| 1421373_at | cytochrome c oxidase subunit IV isoform 2 |
| 1370888_at | cytochrome c oxidase, subunit Va |
| 1449218_at | cytochrome c oxidase, subunit VIIIb |
| 1385572_at | cytochrome c, somatic |
| 1387916_at | cytochrome P450 4F6 |
| 1370706_a_at | cytochrome P450 monooxygenase |
| 1418821_at | cytochrome P450, family 2, subfamily a, polypeptide 12 |
| 1419731_at | cytochrome P450, family 2, subfamily b, polypeptide 19 |
| 1422257_s_at | cytochrome P450, family 2, subfamily b, polypeptide 20 |
| 1419430_at | cytochrome P450, family 26, subfamily a, polypeptide 1 |
| 1377822_at | cytochrome P450, family 27, subfamily a, polypeptide 1 |
| 1374537_at | cytochrome P450-like protein |
| **Decay** | |
| 1427632_x_at | decay accelerating factor 2 |
| 1394570_at | decay-accelarating factor |
| **Diacylglycerol** | |
| 1426738_at | diacylglycerol kinase zeta |
| 1384052_at | diacylglycerol kinase zeta |
| 1419504_at | diacylglycerol O-acyltransferase 2-like 1 |
| **Epididymal protein** | |
| 1438512_at | epididymal protein Av381126 |
| 1373932_at | epididymal secretory protein 1 |
| **Eucaryotic translation elongation factor** | |
| 1387380_at | eukaryotic elongation factor-2 kinase |
| 1418062_at | eukaryotic translation elongation factor 1 alpha 2 |
| 1397520_at | eukaryotic translation initiation factor 4 gamma, 2 |
| 1417563_at | eukaryotic translation initiation factor 4E binding protein 1 |
| 1456613_at | eukaryotic translation initiation factor 4E binding protein 2 |
| **Fatty acid -related** | |
| 1367660_at | fatty acid binding protein 3 |

(continued)

| Fatty acid -related | |
| --- | --- |
| 1382685_at | fatty acid Coenzyme A ligase, long chain 3 |
| 1368453_at | fatty acid desaturase 2 |
| 1388108_at | fatty acid elongase 2 |
| 1423828_at | fatty acid synthase |
| **Fibrillin-1** | |
| 1425896_a_at | fibrillin 1 |
| 1392273_at | fibrillin-1 |
| **FGF-signaling** | |
| 1390390_at | fibroblast growth factor 9 |
| 1374516_at | fibroblast growth factor 9 |
| 1427776_a_at | fibroblast growth factor receptor 4 |
| **FXYD-domain** | |
| 1421374_a_at | FXYD domain-containing ion transport regulator 1 |
| 1382428_at | FXYD domain-containing ion transport regulator 5 |
| 1419200_at | FXYD domain-containing ion transport regulator 7 |
| **G-protein signaling** | |
| 1370178_at | G protein beta-subunit gene |
| 1387342_at | G protein gamma-5 subunit |
| 1388902_at | G protein-coupled receptor 105 |
| 1386049_at | G protein-coupled receptor 51 |
| 1420364_at | G protein-coupled receptor 87 |
| 1375374_at | G protein-coupled receptor kinase 5 |
| 1420538_at | G protein-coupled receptor, family C, group 5, member D |
| 1428053_at | G protein-coupled receptor, family C, group 6, member A |
| 1451250_at | G protein-regulated inducer of neurite outgrowth 1 |
| 1451633_a_at | guanine nucleotide binding protein (G protein), gamma 1 subunit |
| 1451633_a_at | guanine nucleotide binding protein (G protein), gamma 1 subunit |
| 1377739_at | guanine nucleotide binding protein 12 |
| 1450097_s_at | guanine nucleotide binding protein, alpha 12 |
| 1421302_a_at | guanine nucleotide binding protein, alpha 15 |
| 1460212_at | guanine nucleotide binding protein, alpha transducing 1 |
| 1450623_at | guanine nucleotide binding protein, beta 2 |
| 1459520_at | guanine nucleotide binding protein, beta 5 |
| **Gap-junction** | |
| 1375346_at | gap junction membrane channel protein alpha 1 |
| 1455989_at | gap junction membrane channel protein alpha 12 |

(continued)

| Gap-junction | |
|---|---|
| 1379526_at | gap junction membrane channel protein alpha 4 |
| **Glutamatergic signaling** | |
| 1367776_at | glutamate receptor, ionotropic, 2 |
| 1421569_at | glutamate receptor, ionotropic, delta 1 |
| 1427709_at | glutamate receptor, ionotropic, kainate 3 |
| 1385633_at | glutamate receptor, ionotropic, NMDA2B |
| 1431700_at | glutamate receptor, ionotropic, NMDA2B (epsilon 2) |
| 1449245_at | glutamate receptor, ionotropic, NMDA2C (epsilon 3) |
| 1377835_at | glutamate receptor, ionotropic, N-methyl-D-aspartate 3A |
| **Hairy and enhancer of split** | |
| 1423146_at | hairy and enhancer of split 5 (Drosophila) |
| 1386080_at | hairy/enhancer-of-split related with YRPW motif 1 |
| **Heat shock proteins** | |
| 1398916_at | Heat shock 27 kDa protein |
| 1372254_at | heat shock 27kDa protein 1 |
| 1398240_at | Heat shock cognate protein 70 |
| 1424622_at | heat shock factor 1 |
| 1422943_a_at | heat shock protein 1 |
| 1419625_at | heat shock protein 1-like |
| **Hepatocyte growth factors** | |
| 1425379_at | hepatocyte growth factor |
| 1370458_at | hepatoma-derived growth factor, related protein 3 |
| **Hermansky-Pudlak syndrome** | |
| 1385072_at | Hermansky-Pudlak syndrome 1 homolog (human) |
| 1435932_at | Hermansky-Pudlak syndrome 6 |
| **Histones** | |
| 1438009_at | histone 1, H2ae |
| 1390021_at | histone 2b |
| **Hox** | |
| 1420414_at | homeo box A11 |
| 1453501_at | homeo box B1 |
| 1456301_at | homeo box C5 |
| 1368873_at | homeobox A2 |
| **Inositol phosphate** | |
| 1372706_at | inositol 1, 4, 5-triphosphate receptor 3 |
| 1431780_at | inositol hexaphosphate kinase 1 |
| 1369955_at | inositol polyphosphate-5-phosphatase D |

(continued)

| Insulin-like growth factor-associated | |
|---|---|
| 1368123_at | insulin-like growth factor 1 receptor |
| 1423062_at | insulin-like growth factor binding protein 3 |
| 1423756_s_at | insulin-like growth factor binding protein 4 |
| **Integrin-related** | |
| 1455158_at | integrin alpha 3 |
| 1370526_at | integrin alpha E1, epithelial-associated |
| 1426920_x_at | integrin beta 1 (fibronectin receptor beta) |
| **Interferon-related** | |
| 1369031_at | interferon gamma inducing factor binding protein |
| 1370780_at | interferon induced transmembrane protein 3-like |
| 1368073_at | interferon regulatory factor 1 |
| 1383478_at | interferon-gamma inducible gene, Puma-g |
| 1367696_at | interferon-inducible protein 16 |
| 1419569_a_at | interferon-stimulated protein |
| **Interleukins and IL-receptors** | |
| 1375271_at | interleukin 1 receptor accessory protein |
| 1425145_at | interleukin 1 receptor-like 1 |
| 1448731_at | interleukin 10 receptor, alpha |
| 1369315_at | interleukin 12 p35 subunit |
| 1449497_at | interleukin 12b |
| 1370728_at | interleukin 13 receptor, alpha 1 |
| 1434448_at | interleukin 14 |
| 1457471_at | interleukin 17 receptor C |
| 1392531_at | interleukin 18 |
| 1421291_at | interleukin 18 receptor accessory protein |
| 1393414_at | interleukin 2 receptor, gamma chain |
| 1450456_at | interleukin 21 receptor |
| 1421620_at | interleukin 5 receptor, alpha |
| **Karyopterin** | |
| 1374376_at | karyopherin (importin) alpha 2 |
| 1431706_at | karyopherin (importin) beta 3 |
| **Kruppel-associated** | |
| 1368712_at | Kruppel associated box (KRAB) zinc finger 1 |
| 1380203_at | Kruppel-like factor 3 |
| 1441200_at | Kruppel-like factor 3 (basic) |
| 1434025_at | Kruppel-like factor 5 |
| **Lectins** | |
| 1367628_at | lectin, galactose binding, soluble 1 |

(continued)

| Lectins | |
|---|---|
| 1426808_at | lectin, galactose binding, soluble 3 |
| 1368960_at | lectin, galactose binding, soluble 8 |
| 1419951_at | lectin, mannose-binding, 1 |
| **Leu-rich repeat** | |
| 1453126_at | leucine rich repeat and fibronectin type III domain containing 2 |
| 1381374_at | leucine-rich repeat LGI family, member 4 |
| 1453628_s_at | leucine-rich repeat-containing 2 |
| **Lymphocyte antigens** | |
| 1457773_at | lymphocyte antigen 108 |
| 1374793_at | lymphocyte antigen 68 |
| **Macrophage-related** | |
| 1368605_at | macrophage expressed gene 1 |
| 1422062_at | macrophage scavenger receptor 1 |
| **MAGUK** | |
| 1449173_at | membrane protein, palmitoylated 2 (MAGUK p55 subfamily member 2) |
| 1383069_at | membrane protein, palmitoylated 3 (MAGUK p55 subfamily member 3) |
| **MAPs** | |
| 1368411_a_at | microtubule-associated protein 2 |
| 1373268_at | microtubule-associated protein 4 |
| 1421835_at | microtubule-associated protein 7 |
| 1387071_a_at | microtubule-associated protein tau |
| **Mitochondrial ribosoma** | |
| 1369013_a_at | mitochondrial ribosomal protein L17 |
| 1455233_at | mitochondrial ribosomal protein S11 |
| 1452111_at | mitochondrial ribosomal protein S35 |
| 1439210_at | mitochondrial ribosomal protein S9 |
| **Mapk** | |
| 1374405_at | mitogen activated protein kinase 1 |
| 1456565_s_at | mitogen activated protein kinase kinase kinase 12 |
| 1398297_at | mitogen-activated protein kinase 12 |
| 1418060_a_at | mitogen-activated protein kinase 7 |
| 1416437_a_at | mitogen-activated protein kinase 8 interacting protein 3 |
| **Myosin-related** | |
| 1459265_at | myosin head domain containing 1 |
| 1368982_at | myosin IE |
| 1420805_at | myosin light chain 2, precursor lymphocyte-specific |

(continued)

| Myosin-related | |
|---|---|
| 1378580_at | myosin Va |
| 1448826_at | myosin, heavy polypeptide 6, cardiac muscle, alpha |
| 1374494_at | myosin, heavy polypeptide 9 |
| 1427769_x_at | myosin, light polypeptide 3, alkali; ventricular, skeletal, slow |
| **never in mitosis** | |
| 1443999_at | NIMA (never in mitosis gene a)-related expressed kinase 2 |
| 1396428_at | NIMA (never in mitosis gene a)-related expressed kinase 6 |
| 1444753_at | NIMA (never in mitosis gene a)-related expressed kinase 7 |
| 1458157_at | NIMA (never in mitosis gene a)-related expressed kinase 8 |
| **N-myc** | |
| 1450976_at | N-myc downstream regulated 1 |
| 1391438_at | N-myc downstream regulated 4 |
| **Nuclear proteins** | |
| 1373748_at | nuclear factor I/B |
| 1375342_at | nuclear factor I/C |
| 1426032_at | nuclear factor of activated T-cells, cytoplasmic, calcineurin-dependent 2 |
| 1454240_at | nuclear factor, erythroid derived 2, like 3 |
| 1369638_at | nuclear factor, interleukin 3, regulated |
| 1419665_a_at | nuclear protein 1 |
| 1388792_at | nuclear protein 1 |
| 1382194_at | nuclear receptor coactivator 3 |
| 1439710_at | nuclear receptor coactivator 6 interacting protein |
| 1385350_at | nuclear receptor MrgA10 RF-amide G protein-coupled receptor |
| 1451807_at | nuclear receptor subfamily 1, group I, member 2 |
| 1416505_at | nuclear receptor subfamily 4, group A, member 1 |
| **Olfactomedin** | |
| 1425784_a_at | olfactomedin 1 |
| 1393060_at | olfactomedin 3 |
| 1391501_at | olfactomedin related ER localize protein |
| **Opioid receptor** | |
| 1451709_at | opioid receptor, mu |
| 1379625_at | opioid receptor-like |

(continued)

| Oxisterol-binding protein | |
|---|---|
| 1383830_a_at | oxysterol binding protein-like 1A |
| 1425391_a_at | oxysterol binding protein-like 5 |
| 1451831_at | oxysterol binding protein-like 6 |
| **Peroxiredoxins** | |
| 1430979_a_at | peroxiredoxin 2 |
| 1387891_at | peroxiredoxin 4 |
| **Phosphatidylinositol** | |
| 1386089_at | phosphatidylcholine transfer protein |
| 1421023_at | phosphatidylinositol 3-kinase, C2 domain containing, alpha polypeptide |
| 1427305_at | phosphatidylinositol glycan, class A |
| 1435039_a_at | phosphatidylinositol-4-phosphate 5-kinase, type 1 beta |
| 1424954_a_at | phosphatidylinositol-4-phosphate 5-kinase, type 1 gamma |
| **Phospholipases** | |
| 1417785_at | phospholipase A1 member A |
| 1451502_at | phospholipase A2, group X |
| 1372541_at | phospholipase C, beta 4 |
| 1384470_at | phospholipase C, delta 1 |
| 1377049_at | phospholipase D2 |
| 1416013_at | phospholipase D3 |
| **PDGF-associated** | |
| 1450413_at | platelet derived growth factor, B polypeptide |
| 1369642_at | platelet-activating factor acetylhydrolase alpha 2 subunit (PAF-AH alpha 2) |
| 1387807_at | platelet-activating factor acetylhydrolase beta subunit (PAF-AH beta) |
| 1387286_at | platelet-activating factor receptor |
| **Plekstrin homology** | |
| 1426013_s_at | pleckstrin homology domain containing, family A (phosphoinositide binding specific) member 4 |
| 1459324_at | pleckstrin homology domain containing, family C (with FERM domain) member 1 |
| 1423861_at | pleckstrin homology domain containing, family F (with FYVE domain) member 2 |
| 1367727_at | pleckstrin homology, Sec7 and coiled/coil domains 2 |
| **Potassium channels** | |
| 1394939_at | potassium channel interacting protein 4 |
| 1399021_at | potassium channel regulatory factor |
| 1443506_at | potassium channel tetramerisation domain containing 2 |

(continued)

| Potassium channels | |
|---|---|
| 1393220_at | potassium intermediate/small conductance calcium-activated channel, subfamily N, member 2 |
| 1450185_a_at | potassium inwardly-rectifying channel, subfamily J, member 15 |
| 1450503_at | potassium inwardly-rectifying channel, subfamily J, member 2 |
| 1368308_at | potassium large conductance calcium-activated channel, subfamily M, alpha member 1 |
| 1370557_a_at | potassium voltage gated channel, Shaw-related subfamily, member 2 |
| 1370559_at | potassium voltage gated channel, Shaw-related subfamily, member 2 |
| 1375961_at | potassium voltage gated channel, Shaw-related subfamily, member 2 |
| 1382055_at | potassium voltage gated channel, Shaw-related subfamily, member 2 |
| **POU domain transcrition factor** | |
| 1422068_at | POU domain, class 3, transcription factor 1 |
| 1371043_a_at | POU domain, class 3, transcription factor 3 |
| **Procollagen - related** | |
| 1448433_a_at | procollagen C-proteinase enhancer protein |
| 1372897_at | procollagen lysine, 2-oxoglutarate 5-dioxygenase 2 |
| 1423669_at | procollagen, type I, alpha 1 |
| 1450857_a_at | procollagen, type I, alpha 2 |
| 1427883_a_at | procollagen, type 111, alpha 1 |
| 1428571_at | procollagen, type IX, alpha 1 |
| 1384588_at | procollagen, type XI, alpha 1 |
| 1384126_a_at | procollagen, type XXIII, alpha 1 |
| 1429549_at | procollagen, type XXVII, alpha 1 |
| 1380751_at | procollagen-lysine, 2-oxoglutarate 5-dioxygenase (lysine hydroxylase, Ehlers-Danlos syndrome type VI) |
| **Prostaglandin** | |
| 1449310_at | prostaglandin E receptor 2 (subtype EP2) |
| 1377703_at | prostaglandin-endoperoxide synthase 2 |
| **Proteasome subunits** | |
| 1367711_at | Proteasome (prosome, macropain) 26S subunit, ATPase |
| 1393240_at | proteasome (prosome, macropain) 26S subunit, ATPase, 4 |
| 1432726_at | proteasome (prosome, macropain) 26S subunit, non-ATPase, 11 |
| 1431013_at | proteasome (prosome, macropain) 26S subunit, non-ATPase, 11 |

(continued)

| Proteasome subunits | |
|---|---|
| 1444321_at | proteasome (prosome, macropain) 26S subunit, non-ATPase, 9 |
| 1435317_x_at | proteasome (prosome, macropain) subunit, alpha type 6 |
| **Protein-kinase -related** | |
| 1449381_a_at | protein kinase C and casein kinase substrate in neurons 1 |
| 1427562_a_at | protein kinase C, alpha |
| 1427562_a_at | protein kinase C, alpha |
| 1370585_a_at | protein kinase C, beta 1 |
| 1379425_at | protein kinase C, beta 1 |
| 1387226_at | protein kinase C, delta |
| 1455758_at | protein kinase C, gamma |
| 1385162_at | protein kinase C, gamma |
| 1420567_at | protein kinase C, nu |
| 1391653_at | protein kinase inhibitor, alpha |
| 1393280_at | protein kinase inhibitor, alpha |
| 1439718_at | protein kinase inhibitor, gamma |
| 1424119_at | protein kinase, AMP-activated, beta 1 non-catalytic subunit |
| 1440132_s_at | protein kinase, cAMP dependent regulatory, type I beta |
| 1387242_at | Protein kinase, interferon-inducible double stranded RNA dependent |
| 1387072_at | protein kinase, lysine deficient 1 |
| **Protein phosphatase -related** | |
| 1391213_at | protein phospatase 3, regulatory subunit B, alpha isoform, type 1 |
| 1372268_at | protein phosphatase 1, catalytic subunit, beta isoform |
| 1450914_at | protein phosphatase 1, regulatory (inhibitor) subunit 14B |
| 1398790_at | Protein phosphatase 2 (formerly 2A), catalytic subunit, alpha isoform |
| 1431228_s_at | protein phosphatase 2 (formerly 2A), regulatory subunit B", alpha |
| 1425725_s_at | protein phosphatase 2, regulatory subunit B (B56), gamma isoform |
| 1398469_at | protein phosphatase 3 (formerly 2B), catalytic subunit, gamma isoform (calcineurin A gamma) |
| 1388103_at | protein phosphatase 3, catalytic subunit, beta isoform |
| 1430025_at | protein phosphatase 3, catalytic subunit, gamma isoform |
| 1370933_at | protein phosphatase 4, regulatory subunit 1 |
| 1386863_at | Protein phosphatase type 1 alpha, catalytic subunit |
| 1371136_at | Protein phosphatase type 1 B (formely 2C), Mg-dependent, beta isoform |

(continued)

| Protein tyrosine phosphatases | |
|---|---|
| 1379932_at | protein tyrosine phosphatase, non-receptor type 12 |
| 1419054_a_at | protein tyrosine phosphatase, non-receptor type 21 |
| 1375359_at | protein tyrosine phosphatase, receptor type, C |
| 1380190_at | protein tyrosine phosphatase, receptor type, D |
| 1368589_at | Protein tyrosine phosphatase, receptor type, J |
| **Ras and Rab** | |
| 1389803_at | RAB13 |
| 1390707_at | RAB2, member RAS oncogene family |
| 1426800_at | RAB8B, member RAS oncogene family |
| 1424507_at | Ras and Rab interactor 1 |
| 1424507_at | Ras and Rab interactor 1 |
| 1440968_at | Ras association (RalGDS/AF-6) domain family 5 |
| 1449110_at | ras homolog gene family, member B |
| 1370085_at | RAS p21 protein activator 1 |
| 1423619_at | RAS, dexamethasone-induced 1 |
| 1386967_at | ras-like protein |
| **Regulators of G-protein signaling** | |
| 1376665_at | regulator of G-protein signaling 10 |
| 1446199_at | regulator of G-protein signaling 20 |
| 1370918_a_at | regulator of G-protein signaling 7 |
| 1390367_at | regulator of G-protein signalling 19 |
| **Retinol dehydrogenase** | |
| 1377993_at | retinol dehydrogenase 10 (all-trans) |
| 1448723_at | retinol dehydrogenase 7 |
| **Ribosomal proteins** | |
| 1456447_at | ribosomal protein L18 |
| 1441304_at | ribosomal protein L31 |
| 1395248_at | ribosomal protein L31 |
| 1384546_at | ribosomal protein L7 |
| 1415913_at | ribosomal protein S13 |
| 1457726_at | ribosomal protein S15a |
| 1450390_x_at | ribosomal protein S18 |
| 1421935_at | ribosomal protein S20 |
| 1429760_at | ribosomal protein S6 kinase polypeptide 6 |
| 1438243_at | ribosomal protein S6 kinase, polypeptide 4 |
| 1383631_at | ribosomal protein, mitochondrial, L12 |
| 1367686_at | ribosome associated membrane protein 4 |
| 1426123_a_at | ribosome binding protein 1 |

(continued)

| RNA-binding motifs and proteins | |
|---|---|
| 1371583_at | RNA binding motif protein 3 |
| 1369496_at | RNA binding protein HuB |
| 1369971_a_at | RNA binding protein p45AUF1 |
| 1451293_at | RNA, U3 small nucleolar interacting protein 2 |
| 1421265_a at | RNA-binding region (RNP1, RRM) containing 1 |
| **S100 calcium -binding proteins** | |
| 1416762_at | S100 calcium binding protein A10 (calpactin) |
| 1380650_at | S100 calcium binding protein A3 |
| 1424542_at | S100 calcium binding protein A4 |
| **Septins** | |
| 1368984_at | septin 2 |
| 1399099_at | septin 3 |
| 1431973_at | septin 6 |
| **Serine proteinase inhibitors** | |
| 1421564_at | serine (or cysteine) proteinase inhibitor, clade A, member 3C |
| 1448506_at | serine (or cysteine) proteinase inhibitor, clade A, member 6 |
| 1455590_at | serine (or cysteine) proteinase inhibitor, clade B, member 6a |
| 1374018_at | serine (or cysteine) proteinase inhibitor, clade G (C1 inhibitor), member 1, (angioedema, hereditary) |
| 1380496_at | serine (or cysteine) proteinase inhibitor, clade I (neuroserpin), member 1 |
| 1370163_at | serine (or cysteine) proteinase inhibitor, clade I (neuroserpin), member 1 |
| **SH3 domain proteins** | |
| 1395473_at | SH3 domain protein 2A |
| 1432269_a_at | SH3-domain kinase binding protein 1 |
| **Sodium channels** | |
| 1425088_at | sodium channel, nonvoltage-gated, type I, alpha polypeptide |
| 1391714_at | sodium channel, voltage-gated, type 1, alpha polypeptide |
| 1395464_at | sodium channel, voltage-gated, type 2, alpha 1 polypeptide |
| 1442208_at | sodium channel, voltage-gated, type VIII, alpha polypeptide |
| 1420784_at | sodium channel, voltage-gated, type XI, alpha polypeptide |
| **Solute carrier family** | |
| 1379364_at | solute carrier family 1, member 1 |

(continued)

| Solute carrier family | |
|---|---|
| 1369694_at | solute carrier family 1, member 2 |
| 1369693_a_at | solute carrier family 1, member 2 |
| 1368574_at | solute carrier family 1, member 2 |
| 1389075_at | solute carrier family 1, member 3 |
| 1424260_at | solute carrier family 12, member 1 |
| 1419343_at | solute carrier family 15 (oligopeptide transporter), member 1 |
| 1453675_at | solute carrier family 16 (monocarboxylic acid transporters), member 10 |
| 1392830_at | solute carrier family 16, member 1 |
| 1378666_at | solute carrier family 2 (facilitated glucose transporter), member 13 |
| 1449067_at | solute carrier family 2 (facilitated glucose transporter), member 2 |
| 1368215_at | solute carrier family 2, member 5 |
| 1385925_at | solute carrier family 21 (organic anion transporter), member 9 |
| 1392735_at | solute carrier family 21 (organic anion transporter), member 9 |
| 1393141_at | solute carrier family 22 ,member 8 |
| 1373262_at | solute carrier family 22, member 2 |
| 1371606_at | solute carrier family 24 (sodium/potassium/calcium exchanger), member 2 |
| 1376943_at | solute carrier family 24, member 3 |
| 1425841_at | solute carrier family 26, member 7 |
| 1448257_at | solute carrier family 29 (nucleoside transporters), member 2 |
| 1368440_at | solute carrier family 3, member 1 |
| 1397317_at | solute carrier family 3, member 1 |
| 1444027_at | solute carrier family 30 (zinc transporter), member 8 |
| 1444027_at | solute carrier family 30 (zinc transporter), member 8 |
| 1439519_at | solute carrier family 34 (sodium phosphate), member 3 |
| 1378487_at | solute carrier family 35, member B2 |
| 1369473_at | solute carrier family 39 (iron-regulated transporter), member 1 |
| 1416464_at | solute carrier family 4 (anion exchanger), member 1 |
| 1457989_at | solute carrier family 4, sodium bicarbonate transporter-like, member 11 |
| 1428752_at | solute carrier family 5 (sodium/glucose cotransporter), member 10 |
| 1426008_a_at | solute carrier family 7 (cationic amino acid transporter, y+ system), member 2 |

(continued)

| Solute carrier family | |
|---|---|
| 1378245_at | solute carrier family 7 (cationic amino acid transporter, y+system), member 7 |
| 1449301_at | solute carrier family 7, (cationic amino acid transporter, y+ system) member 13 |
| 1387950_at | solute carrier family 7, member 1 |
| 1388645_at | solute carrier family 8 (sodium/calcium exchanger), member 3 |
| 1454053_at | solute carrier family 9 (sodium/hydrogen exchanger), isoform 9 |
| 1449203_at | solute carrier organic anion transporter family, member 1 a5 |
| 1420913_at | solute carrier organic anion transporter family, member 2a1 |
| **Src-associated** | |
| 1386896_at | src associated in mitosis, 68 kDa |
| 1385030_at | src family associated phosphoprotein 2 |
| 1435598_at | src homology 2 domain-containing transforming protein C2 |
| 1393201_at | src-like adaptor |
| **Sterol-regulatory element binding** | |
| 1426690_a_at | sterol regulatory element binding factor 1 |
| 1392655_at | sterol regulatory element binding protein 2 |
| **Sulfatases** | |
| 1385830_at | sulfatase 1 |
| 1430388_a_at | sulfatase 2 |
| **Superoxide dismutase** | |
| 1367641_at | Superoxide dismutase 1, soluble |
| 1372136_at | superoxide dismutase 3 |
| 1417633_at | superoxide dismutase 3, extracellular |
| 1417634_at | superoxide dismutase 3, extracellular |
| **Syntaxin** | |
| 1421673_s_at | syntaxin 1 b-like |
| 1450349_at | syntaxin 1 b-like |
| 1453228_at | syntaxin 11 |
| 1386853_s_at | syntaxin 5a |
| 1454974_at | syntaxin 8 |
| **Tachykinin** | |
| 1419411_at | tachykinin 2 |
| 1392492_at | tachykinin receptor 1 |

(continued)

| Transcription factors | |
|---|---|
| 1427787_at | trans-acting transcription factor 6 |
| 1368842_at | transcription factor 4 |
| 1389092_at | transcription factor 8 |
| 1421996_at | transcription factor AP-2, alpha |
| 1421995_at | transcription factor AP-2, alpha |
| 1429086_at | transcription factor CP2-like 3 |
| 1452643_at | transcription factor CP2-like 3 |
| **Tubulins** | |
| 1452571_at | tubulin alpha, related sequence 1 |
| 1427832_at | tubulin alpha, related sequence 1 |
| 1383637_at | tubulin, beta 5 |
| 1417144_at | tubulin, gamma 1 |
| **Tnf-signaling** | |
| 1460642_at | Tnf receptor associated factor 4 |
| 1448861_at | Tnf receptor-associated factor 5 |
| 1421588_at | tumor necrosis factor (ligand) superfamily, member 14 |
| 1430259_at | tumor necrosis factor receptor superfamily, member 11a |
| 1386259_a_at | tumor necrosis factor receptor superfamily, member 12a |
| 1419307_at | tumor necrosis factor receptor superfamily, member 13c |
| 1376056_at | tumor necrosis factor receptor superfamily, member 1a |
| 1422101_at | tumor necrosis factor receptor superfamily, member 23 |
| 1421481_at | tumor necrosis factor receptor superfamily, member 9 |
| **Tumor proteins** | |
| 1458668_at | tumor protein D52 |
| 1387131_at | tumor protein p53 |
| **Ubiquitin conjugating enzyme** | |
| 1370250_at | Ubiquitin conjugating enzyme E21 |
| 1444523_s_at | ubiquitin-conjugating enzyme E2 variant 1 |
| 1383770_at | ubiquitin-conjugating enzyme E2 variant 2 |
| 1416475_at | ubiquitin-conjugating enzyme E2D 2 |
| 1436457_at | ubiquitin-conjugating enzyme E21 |
| 1417172_at | ubiquitin-conjugating enzyme E2L 6 |
| 1430962_at | ubiquitin-conjugating enzyme E2S |
| **UDP-Gal:betaGlcNAc beta 1,3-galactosyltransferase, polypeptide 1** | |
| 1450530_at | UDP-Gal:betaGlcNAc beta 1,3-galactosyltransferase, polypeptide 1 |
| 1379445_at | UDP-Gal:betaGlcNAc beta 1,4-galactosyltransferase, polypeptide 1 |

(continued)

| Ubiquitin conjugating enzyme | |
|---|---|
| 1451815_at | UDP-GlcNAc:betaGal beta-1 ,3-N-acetylglucosaminyltransferase 4 |
| **v-maf** | |
| 1368388_at | v-maf musculoaponeurotic fibrosarcoma (avian) oncogene homolog (c-maf) |
| 1387407_at (avian) | v-maf musculoaponeurotic fibrosarcoma oncogene family, protein B |
| 1448916_at (avian) | v-maf musculoaponeurotic fibrosarcoma oncogene family, protein G |
| **Williams-Beuren syndrome** | |
| 1445746_at | Williams-Beuren syndrome chromosome region 1 homolog (human) |
| 1369653_at | Williams-Beuren syndrome chromosome region 14 homolog (human) |
| **Wingless-related MMTV integration sites** | |
| 1450772_at | wingless-related MMTV integration site 11 |
| 1449425_at | wingless-related MMTV integration site 2 |
| 1387130_at | wingless-type MMTV integration site family, member 2B |
| **Zinc finger proteins** | |
| 1449535_at | zinc and ring finger 4 |
| 1439698_at | zinc finger protein (C2H2 type) 276 |
| 1435131_at | zinc finger protein 13 |
| 1445649_x_at | zinc finger protein 142 |
| 1418360_at | zinc finger protein 179 |
| 1387105_at | zinc finger protein 22 (KOX 15) |
| 1447228_at | zinc finger protein 289 |
| 1450152_at | zinc finger protein 316 |
| 1425976_x_at | zinc finger protein 353 |
| 1369959_at | zinc finger protein 36, C3H type-like 1 |
| 1367716_at | zinc finger protein 36, C3H type-like 1 |
| 1451696_at | zinc finger protein 64 |
| 1370705_at | zinc finger protein HIT-4 |
| 1425666_at | zinc finger protein of the cerebellum 5 |
| 1449512_a_at | zinc finger protein X-linked |
| 1428046_a_at | zinc finger protein X-linked |
| 1458450_at | zinc finger RNA binding protein |
| 1441639_at | zinc finger, CCHC domain containing 8 |
| 1424551_at | zinc finger, FYVE domain containing 27 |
| 1422750_a_at | zinc finger, MYND domain containing 10 |
| 1419791_at | zinc fingers and homeoboxes 3 |

CONSOLIDATION OF THE DATA

The above data were further confirmed by 2D-gels and/or isotope-coded affinity tag (ICAT).
A list of the genes differentially regulated after inhibition or downregulation of Nogo-A considered to be the most relevant ones is provided in Table 25.

TABLE 25. List of the most relevant genes

| Description | Symbol |
|---|---|
| **Adhesion** | |
| cadherin 11 | Cdh11 |
| cadherin 2 | Cdh2 |
| cadherin 8 | Cdh8 |
| cadherin 22 | Cdh22 |
| Eph receptor A3 | epnra3 |
| Eph receptor A4 | epnra4 |
| Ephrin A3 | Epna3 |
| Ephrin B2 | epnb2 |
| Eph B2 receptor | ephb2 |
| sema domain, immunoglobulin domain (Ig), transmembrane domain (TM) and short cytoplasmic domain, (semaphorin) 4A | Sema4a |
| sema domain, immunoglobulin domain (Ig), transmembrane domain (TM) and short cytoplasmic domain, (semaphorin) 4D | Sema4d |
| sema domain, immunoglobulin domain (Ig), transmembrane domain (TM) and short cytoplasmic domain, (semaphorin) 4F | Sema4f |
| sema domain, transmembrane domain (TM), and cytoplasmic domain, (semaphorin) 6A | Sema6a |
| sema domain, transmembrane domain (TM), and cytoplasmic domain, (semaphorin) 6B | Sema6b |
| semaF cytoplasmic domain associated protein 3 | Sema3 |
| plexin B2 | Plxn2 |
| **Cytoskeleton** | |
| capping protein (actin filament), gelsolin-like | Capg |
| casein kinase 1, delta | Csnk1d |
| centractin | |
| gelsolin | Gsn |
| microtubule-associated protein tau | Mapt |
| neurofilament 68 | NF68 |
| **Olfactomedin-family** | |
| myocilin, TIGR | TIGR |
| olfactomedin 1 | Olfm1 |
| olfactomedin 3 | Olfm 3 |
| **Interferon-mediated signaling** | |
| Interferon gamma | Ifng |
| **Signaling** | |
| Rho-GDP-dissociation ihibitor 1 | |

(continued)

| Cytoskeleton | |
|---|---|
| Dihydropyrimidinase related protein 2 (CRMP2) | CRMP2 |
| Dihydropyrimidinase related protein 1 (CRMP1) | CRMP1 |
| Dihydropyrimidinase related protein 5 (CRMP5) | CRMP5 |
| Alzheimer related | |
| Synuclein | |
| Amyloid beta (A4) PP-binding A1 | |
| Amyloid beta (A4) precursor-like protein 1 and 2 | |
| Others | |
| prostaglandin E synthase | Ptges |
| benzodiazepine receptor | Bzrp |
| biglycan | Bgn |

EQUIVALENTS

The present invention is not to be limited in terms of the particular embodiments described in this application, which are intended as single illustrations of individual aspects of the invention. Many modifications and variations of this invention can be made without departing from its spirit and scope, as will be apparent to those skilled in the art. Functionally equivalent methods and apparatuses within the scope of the invention, in addition to those enumerated herein, will be apparent to those skilled in the art from the foregoing descriptions. Such modifications and variations are intended to fall within the scope of the appended claims. The present invention is to be limited only by the terms of the appended claims along with the full scope of equivalents to which such claims are entitled.

**Claims**

1. A method for predicting the response of a subject to a medicament comprising an anti-Nogo-A antibody, wherein the expression of at least one gene of Table 25 is assessed before and after administration of said medicament comprising an anti-Nogo-A antibody and wherein said expression of said at least one gene of Table 25 after administration of said medicament comprising an anti-Nogo-A antibody is compared to the expression of said gene prior to said administration of the medicament comprising an anti-Nogo-A antibody.

2. The method of claim 1 wherein a dysregulation of said expression of at least one gene of Table 25 after administration of the medicament comprising an anti-Nogo-A antibody as compared to the expression of said gene prior said administration of the medicament comprising an anti-Nogo-A antibody is indicative of a positive response (responder) to said administration of the medicament comprising an anti-Nogo-A antibody.

3. The method of claim 1 wherein the lack of a dysregulation of said expression of at least one gene of Table 25 after administration of the medicament comprising an anti-Nogo-A antibody as compared to the expression of said gene prior said administration of the medicament comprising an anti-Nogo-A antibody is indicative of a lack of response (non-responder) to said administration of the medicament comprising an anti-Nogo-A antibody.

4. The method of claims 2 or 3 wherein said dysregulation of said expression of at least one gene of Table 25 after administration of the medicament comprising an anti-Nogo-A antibody is a change in expression that is larger or equal to 1.2 fold and statistically significant ($p < 0.05$, Student's t-test) as compared to the expression of said gene prior said administration of the medicament comprising an anti-Nogo-A antibody.

5. The method of claims 1-4 wherein the expression of at least one gene of each of the groups of adhesion genes, cytoskeleton genes and signalling genes is assessed, wherein said group of adhesion genes consists of cadherin 11, cadherin 2, cadherin 8, cadherin 22, Eph receptor A3, Eph receptor A4, Ephrin A3, Ephrin B2, Eph receptor B2, semaphorin 4A, semaphorin 4D, semaphorin 4F, semaphorin 6A, semaphorin 6B, semaF cytoplasmic domain associated protein 3 and plexin B2,

wherein said group of cytoskeleton genes consist of capping protein (actin filament) gelsolin-like, casein kinase 1 delta, centractin, gelsolin, microtubule-associated protein tau and neurofilament 68, and

wherein said group of signalling genes consists of Rho-GDP-dissociation inhibitor 1, dihydropyrimidinase related protein 2, dihydropyrimidinase related protein 1, dihydropyrimidinase related protein 5.

6. The method of claims 1-5 wherein the expression of all the genes of Table 25 is assessed.

7. The method of claim 1 wherein a dysregulation of said expression of at least one gene of Table 25 after administration of the medicament comprising an anti-Nogo-A antibody as compared to the expression of said gene prior said administration of the medicament comprising an anti-Nogo-A antibody is indicative of indicates central nervous system regeneration.

8. The method of claims 1-7 which is performed *in vitro.*

9. The use of an anti-Nogo-A antibody in the manufacture of a medicament for the treatment of central nervous system injury in a patient population, wherein the patient population is selected by the method of claims 1-8.

10. The use of claim 9, wherein the anti-Nogo-A antibody is a fully human monoclonal antibody (IgG4/κ) that binds to the epitope of human Nogo-A fragment from amino acid 342-357.

11. A method for treating a central nervous system injury in a subject, comprising the steps of:

(a) administering an anti-Nogo-A antibody to a subject with a central nervous system injury;
(b) determining the gene expression pattern of the subject according to the method of claims 1-8; and
(c) either:

(i) continuing with the anti-Nogo-A antibody therapy if the gene expression of biomarkers indicates central nervous system regeneration, or
(ii) stopping or reducing the anti-Nogo-A antibody therapy if the gene expression of biomarkers does not indicate central nervous system regeneration.

12. A method for diagnosing central nervous system regeneration in a subject, comprising the steps of:

(a) administering an anti-Nogo-A antibody to the subject;
(b) determining the gene expression pattern of the subject according to the method of claims 1-8; and
(c) determining whether the gene expression of biomarkers indicates central nervous system regeneration.

13. A kit for performing the methods of claims 1-12 comprising at least two probes, each probe being capable of specifically detecting the expression of one gene of Table 25, wherein said at least two probes do not detect the expression of the same gene.

# FIG. 1

## Enrichment of immunity and defence–related transcripts in the direction of 11C7 after one week of treatment identified by GSEA in T8

# FIG. 2

# Enrichment of cytokine and chemokine mediated signalling pathway in the direction of 11C7 after one week of treatment identified by GSEA in T8

# FIG. 3

## Enrichment of Jak-stat cascade–related transcripts in the direction of 11C7 after one week of treatment identified by GSEA in T8

# FIG. 4

## Enrichment of oxidative phosphorylation–related transcripts in the direction of 11C7 after two weeks of treatment identified by GSEA in T8

# FIG. 5

## Enrichment of synaptic transmission–related transcripts in the direction of 11C7 after two weeks of treatment identified by GSEA in T8

# FIG. 6

## Enrichment of ECM-mediated signalling– related transcripts in the direction of IgG after one week of treatment identified by GSEA in T1-7

extracellular matrix protein-mediated signaling probesets

all other probesets

# FIG. 7

## Enrichment of lipid metabolism–related transcripts in the direction of 11C7 after one week of treatment identified by GSEA in T1-7

# FIG. 8

## Enrichment of growth factor homeostasis–related transcripts in the direction of IgG after one week of treatment identified by GSEA in T1-7

# FIG. 9

## Enrichment of immunity and defence–related transcripts in the direction of 11C7 after one week of treatment identified by GSEA in L1-5

# FIG. 10

## Enrichment of signal transduction–related transcripts in the direction of 11C7 after one week of treatment identified by GSEA in L1-5

# FIG. 11

## Enrichment of cell communication–related transcripts in the direction of 11C7 after one week of treatment identified by GSEA in L1-5

# FIG. 12

## Enrichment of immunity and defence–related transcripts in the direction of IgG after two weeks of treatment identified by GSEA in L1-5

# FIG. 13

## Enrichment of cell communication–related transcripts in the direction of IgG after two weeks of treatment identified by GSEA in L1-5

# FIG. 14

# Enrichment of synaptic transmission–related transcripts in the direction of 11C7 after two weeks of treatment identified by GSEA in L1-5

# FIG. 15

## Enrichment of Huntington's disease–related transcripts in the direction of IgG after two weeks of treatment identified by GSEA in motor-somatosensory cortex

# FIG. 16

## Enrichment of EGF receptor mediated signalling–related transcripts in the direction of IgG after two weeks of treatment identified by GSEA in motor-somatosensory cortex

# FIG. 17

## Enrichment of FGF receptor mediated signalling–related transcripts in the direction of IgG after two weeks of treatment identified by GSEA in motor-somatosensory cortex

# FIG. 18

## Enrichment of NGF receptor mediated signalling–related transcripts in the direction of IgG after two weeks of treatment identified by GSEA in motor-somatosensory cortex

# FIG. 19

## Enrichment of receptor mediated endocytosis–related transcripts in the direction of 11C7 after one week of treatment identified by GSEA in blood

## FIG. 20

## Enrichment of interferon mediated immunity–related transcripts in the direction of 11C7 after one week of treatment identified by GSEA in blood

# FIG. 21

## Enrichment of neuroactive ligand-receptor interaction–related transcripts in the direction of IgG after one week of treatment identified by GSEA in blood

# FIG. 22

## Enrichment of macrophage mediated immunity–related transcripts in the direction of 11C7 after one week of treatment identified by GSEA in blood

# FIG. 23

## Enrichment of Il1b signaling–related transcripts in the direction of IgG after one week of treatment identified by GSEA in blood

# FIG. 24

## Enrichment of B cell activation–related transcripts in the direction of 11C7 after one week of treatment identified by GSEA in blood

# FIG. 25

# Enrichment of immunity and defense–related transcripts in the direction of IgG after two weeks of treatment identified by GSEA in blood

# FIG. 26

# Upregulation of Cxcr4 and Cxcl12 (slit-robo pathway) after one week of 11C7 treatment in spinal cord

| Y-axis: | Tissue atlas 2, Default Interpretation |
| Colored by: | N FCX |
| Gene List: | Axon guidance mediated by Slit-Robo (3), 3 genes select... |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0031235 A
- WO 200005364A1 A
- WO 9005191 A
- US 4965188 A
- WO 9001069 A
- US 5130238 A
- EP 0329822 A
- US 5169766 A
- WO 8906700 A
- WO 9511995 A
- US 5202231 A
- US 5445934 A
- US 5525464 A
- US 5695940 A
- US 5744305 A
- US 5795716 A
- US 5800992 A
- US 4843155 A
- US 4683232 A, Mullis
- US 5854033 A
- EP 0534858 A1
- US 4376110 A
- US 4946778 A
- US 5932448 A
- US 5693762 A
- US 5693761 A
- US 5585089 A
- US 5530101 A
- US 5569825 A
- US 5625126 A
- US 5633425 A
- US 5789650 A
- US 5661016 A
- US 5770429 A
- WO 9324834 A
- US 5792664 A
- US 5062935 A
- WO 9014148 A
- US 5777325 A
- US 5742049 A
- US 5654545 A
- US 5641959 A
- US 5760393 A
- US 4694167 A
- US 4686366 A
- US 4295046 A
- US 5045694 A
- US 5719060 A
- US 5894063 A
- US 6020208 A
- US 6027942 A
- US 6124137 A
- US 20030003465 A
- US 5410068 A
- US 5612474 A
- US 5198531 A
- US 786988 A
- US 08787639 A
- WO 9820166 A
- US 08787639 B

**Non-patent literature cited in the description**

- **BAREYRE FM ; SCHWAB ME.** *Trends Neurosci.,* 2003, vol. 26, 555-563
- **MOOTHA VK et al.** *Nat. Genet.,* 2003, vol. 34, 267-273
- **SUBRAMANIAN et al.** *Proc. Natl. Acad. Sci. U.SA,* 2005, vol. 102 (43), 15545-50
- **LIEBERAM I et al.** *Neuron,* 2005, vol. 47, 667-679
- **BREGMAN BS et al.** *Nature,* 1995, vol. 378, 498-501
- **BROSAMLE C et al.** *J Neurosci.,* 2000, vol. 20, 8061-8068
- **BAREYRE FM et al.** *J. Neurosci.,* 2002, vol. 22, 7097-7110
- **CHEN et al.** *Nature,* 2000, vol. 403, 434-439
- **FIEDLER M et al.** *Protein Eng.,* 2002, vol. 15, 931-941
- **MERKLER D et al.** *J. Neurosci.,* 2001, vol. 21, 3665-3673
- **OERTLE T et al.** *J Neurosci.,* 2003, vol. 23, 5393-5406
- **PAPADOPOULOS CM et al.** *Ann. Neurol.,* 2002
- **VON MEYENBURG J et al.** *Exp.Neurol.,* 1998, vol. 154, 583-594
- **WIESSNER C et al.** Pharmacology of Cerebral Ischemia. 2003, 343-353
- **WIESSNER C et al.** *J. Cereb. Blood Flow & Metab.,* 2003, vol. 23, 154-165

- **LIEBSCHER et al.** *Ann. Neurol.,* 2005, vol. 58, 706-719
- Current Protocols in Molecular Biology. 1997, vol. I-III
- **SAMBROOK et al.** Molecular Cloning: A Laboratory Manual. Cold Spring Harbor Laboratory Press, 1989
- DNA Cloning: A Practical Approach. 1985, vol. I, II
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **PERBAL.** A Practical Guide to Molecular Cloning; the series, Methods in Enzymol. Academic Press, Inc, 1984
- Gene Transfer Vectors for Mammalian Cells. Cold Spring Harbor Laboratory, 1987
- Methods in Enzymology. vol. 154, 155
- **BARANY et al.** *Proc. Natl. Acad. Sci. USA,* 1991, vol. 88, 189-193
- **LANDEGREN et al.** *Science,* 1988, vol. 241, 1077-1080
- **WALKER et al.** *Proc. Natl. Acad. Sci., USA,* 1992, vol. 89, 392-396
- Molecular Cloning A Laboratory Manual. Cold Spring Harbor Laboratory Press, 1989
- DNA Cloning. 1985, vol. I, II
- Nucleic Acid Hybridization. 1984
- **STOREY JD ; TIBSHIRANI R.** *Proc Natl Acad Sci USA,* 2003, vol. 100, 9440-9445
- **FISHER LD ; VANBELLE G.** Biostatistics: A Methodology for the Health Sciences. Wiley-Interscience, 1993
- Genetic Algorithms and Their Uses in Chemistry. **JUDSON R.** Reviews in Computational Chemistry. VCH Publishers, 1997, vol. 10, 1-73
- **PRESS et al.** Numerical Recipes in C: The Art of Scientific Computing. Cambridge University Press, 1992
- **RICH E ; KNIGHT K.** Artificial Intelligence. McGraw-Hill, 1991
- **PERBAL.** A Practical Guide to Molecular Cloning; the series, Meth. Enzymol. Academic Press, Inc, 1984
- **WU ; GROSSMAN.** Meth. Enzymol. vol. 154, 155
- **STRACHAN ; READ.** Human Molecular Genetics. John Wiley and Sons, Inc, 1999
- **SAMBROOK J. et al.** Molecular Cloning: A Labratoty Manual. Cold Spring Harbor Press, 2000
- **JOHNSTON M.** *Curr. Biol.,* 1998, vol. 8, R171-174
- **IYER VR et al.** *Science,* 1999, vol. 283, 83-87
- **ELIAS P.** New human genome 'chip' is a revolution in the offing. *Los Angeles Daily News,* 03 October 2003
- Curr. Prot. Mol. Biol. John Wiley & Sons, 1987
- **GUATELLI et al.** *Proc, Natl. Acad. Sci, USA,* 1990, vol. 87, 1874-1878
- **KWOH et al.** *Proc. Natl. Acad. Sci. USA,* 1989, vol. 86, 1173-1177
- **LIZARDI et al.** *Biol. Technolog,* 1988, vol. 6, 1197
- **LUTHRA et al.** *Am. J. Pathol.,* 1998, vol. 153, 63-68
- **KUIMELIS et al.** *Nucl. Acids Symp. Ser.,* 1997, vol. 37, 255-256
- **MULLAH et al.** *Nucl. Acids Res.,* 1998, vol. 26 (4), 1026-1031
- **HEID et al.** *Genome Res.,* 1996, vol. 6 (6), 986-994
- **GIBSON et al.** *Genome Res.,* 1996, vol. 6, 995-1001
- **LAKOWICZ et al.** *J. Biol. Chem.,* 1983, vol. 258, 4794-4801
- **PRASHAR et al.** *Proc. Natl. Acad. Sci., USA,* 1996, vol. 93 (2), 659-663
- **VELCULESCU.** *Science,* 1995, vol. 270, 484-487
- **BAILEY NTJ.** Statistical Methods In Biology. Cambridge University Press, 1995
- **HARLOW ; LANE.** Antibodies: A Laboratory Manual. Cold Spring Harbor, 1988
- **HUSE et al.** *Science,* 1989, vol. 246, 1275-1281
- **KOHLER ; MILSTEIN.** *Nature,* 1975, vol. 256, 495-497
- **KOSBOR et al.** *Immunol. Today,* 1983, vol. 4, 72
- **COLE et al.** *Proc. Natl. Acad. Sci., USA,* 1983, vol. 80, 2026-2030
- **COLE et al.** Monoclonal Antibodies and Cancer Therapy. Alan R. Liss, Inc, 1985, 77-96
- **MORRISON et al.** *Proc Natl Acad. Sci. USA,* 1984, vol. 81, 6851-6855
- **NEUBERGER et al.** *Nature,* 1984, vol. 312, 604-608
- **TAKEDA et al.** *Nature,* 1985, vol. 314, 452-454
- **BIRD.** *Science,* 1988, vol. 242, 423-426
- **HUSTON et al.** *Proc. Natl Acad. Sci. USA,* 1988, vol. 85, 5879-5883
- **WARD et al.** *Nature,* 1989, vol. 334, 544-546
- **HAMES et al.** Gel Electrophoresis of Proteins: A Practical Approach. IRL Press, 1990
- **SHEVCHENKO et al.** *Proc Natl. Acad. Sci. USA,* 1996, vol. 93, 14440-14445
- **SAGLIOCCO et al.** *Yeast,* 1996, vol. 12, 1519-1533
- **LANDER.** *Science,* 1996, vol. 274, 536-539

- **VALASKOVIC et al.** *Science,* 1996, vol. 273, 1199-1202
- **LI et al.** *J Am. Chem. Soc.,* 1996, vol. 118, 1662-1663
- **VORM et al.** *Anal. Chem.,* 1994, vol. 61, 3281
- **VALASKOVIC et al.** *Anal. Chem.,* 1995, vol. 67, 3802
- **FENN et al.** *J. Phys. Chem.,* 1984, vol. 88, 4451-4459
- **SMITH et al.** *Anal. Chem.,* 1990, vol. 62, 882-89
- **ARDREY.** *Spectroscopy,* 1992, vol. 4, 10-18
- **JUHASZ et al.** *Analysis, Anal. Chem.,* 1996, vol. 68, 941-946
- Bordeaux Mass Spectrometry Conference Report. 1988, 354-362
- Bordeaux Mass Spectrometry Conference Report. 1988, 416-417
- **KARAS ; HILLENKAMP.** *Anal. Chem.,* 1988, vol. 60, 2299-2301
- **KARAS et al.** *Biomed Environ Mass Spectrum,* 1989, vol. 18, 841-843
- **HUTCHENS ; YIP.** *Rapid Common. Mass Spectrom.,* 1993, vol. 7, 576-580
- **RINK.** *Tetrahedron Lett.,* 1976, vol. 28, 3787
- **LEZNOFF.** *Ace Chem. Res.,* 1978, vol. 11, 327
- **BODANSKY et al.** Peptide Synthesis. Academic Press, 1976
- **OERTLE T et al.** *J. Neurosci.,* 2003, vol. 23, 5393-5406
- **JURYNEC MJ et al.** *Mol. Cell. Neurosci.,* 2003, vol. 23, 69-80
- **VEYRAC A et al.** *Eur. J Neurosci.,* 2005, vol. 21, 2635-2648
- **ARAKAWA Y et al.** *J. Cell. Biol.,* 2003, vol. 161, 381-391
- **PUJOL F et al.** *J. Cell Sci.,* 2005, vol. 118, 1071-1080
- **XIANG Y et al.** *Nat. Neurosci.,* 2002, vol. 5, 843-848
- **KARNEZIS T et al.** *Nat. Neurosci.,* 2004, vol. 7, 736-744
- **FONTOURA P et al.** *J Immunol.,* 2004, vol. 173, 6981-6992
- **HE Z ; KOPRIVICA V.** *Annu. Rev. Neurosci.,* 2004, vol. 27, 341-368
- **KOPRIVICA V et al.** *Science,* 2005, vol. 310, 106-110